# EUROPEAN PATENT SPECIFICATION

(11) **EP 1 026 949 B1**
(45) Date of publication and mention of the grant of the patent: **15.09.2010**
(21) Application number: 98949384.6
(22) Date of filing: 18.09.1998
(51) Int. Cl.: C07K 14/29

(54) **OUTER MEMBRANE PROTEINS OF EHRLICHIA CHAFFEENSIS**
PROTEINE DER ÄUSSEREN MEMBRAN VON EHRLICHIA CHAFFEENSIS
PROTEINES DE MEMBRANE EXTERNE DE EHRLICHIA CHAFFEENIS

(30) Priority: 19.09.1997 US 59353 P
(43) Date of publication of application: 16.08.2000
(60) Divisional application: 10007543.1
(73) Proprietor: The Ohio State University Research Foundation, Columbus, OH 43212 (US)
(72) Inventor: RIKIHISA, Yasuko, Worthington, OH 43085 (US); OHASHI, Noris, Columbus, OH 43212 (US)
(74) Representative: Kremer, Simon Mark
(86) International application number: PCT/US1998/019600
(87) International publication number: WO 1999/013720

(56) References cited:
- US-A- 5 401 656
- US-A- 5 413 931
- US-A- 5 789 176
- US-A- 5 869 335
- P. BROUQUI ET AL.: "Serologic diagnosis of human monocytic ehrlichiosis by Immunoblot analysis" CLINICAL AND DIAGNOSTIC LABORATORY IMMUNOLOGY, vol. 1, no. 6, November 1994 (1994-11), pages 645-649, XP008041540
- N. OHASHI ET AL.: "Immunoprotective 28-kDa outer membrane protein of Ehrlichia chaffeensis is a member of multi-sized protein antigen family" ABSTRACTS OF THE 97TH GENERAL MEETING OF THE AMERICAN SOCIETY FOR MICROBIOLOGY, 4 May 1997 (1997-05-04), page 221, XP002313864
- XUEJIE YU ET AL.: "Detection of Ehrlichia chaffeensis in human tissue by using a species-specific monoclonal antibody" JOURNAL OF CLINICAL MICROBIOLOGY, vol. 31, 1993, pages 3284-3288, XP008041569
- S.-M. CHEN ET AL.: "Identification of the antigenic constituents of Ehrlichia chaffeensis" AMERICAN JOURNAL OF TROPICAL MEDICINE AND HYGIENE, vol. 50, no. 1, 1994, pages 52-58, XP008041590
- XUE-JIE YU ET AL.: "Cloning and sequencing of the gene for a 120-kDa immunodominant protein of Ehrlichia chaffeensis" GENE, vol. 184, no. 1, 1 September 1997 (1997-09-01), pages 149-154, XP004093317
- G. ROMAN REDDY ET AL.: "Sequence heterogeneity of the Major antigenic protein 1 genes from Cowdria ruminantium isolates from different geographical areas" CLINICAL AND DIAGNOSTIC LABORATORY IMMUNOLOGY, vol. 3, no. 4, July 1996 (1996-07), pages 417-422, XP002313866
- XUE-JIE YU ET AL.: "Sequence and characterization of an Ehrlichia chaffeensis gene encoding 314 amino acids highly homologous to the NAD A enzyme" FEMS MICROBIOLOGY LETTERS, vol. 154, no. 1, 1 September 1997 (1997-09-01), pages 53-58, XP002313867
- NORIO OHASHI ET AL.: "Immunodominant Major Outer Membrane proteins of Ehrlichia chaffeensis are encoded by a polymorphic multigene family" INFECTION AND IMMUNITY, vol. 66, no. 1, January 1998 (1998-01), pages 132-139, XP002313868
- G. ROMAN REDDY ET AL.: "Molecular characterization of a 28 kDa surface antigen gene family of the tribe Ehrlichiae" BIOCHEMICAL AND BIOPHYSICAL RESEARCH COMMUNICATIONS, vol. 247, June 1998 (1998-06), pages 636-643, XP002313869
- NORIO OHASHI ET AL.: "Cloning and characterization of multigenes encoding the immunodominant 30-Kilodalton Major outer membrane proteins of Ehrlichia canis and application of the recombinant protein for serodiagnosis" JOURNAL OF CLINICAL MICROBIOLOGY, vol. 36, no. 9, September 1998 (1998-09), pages 2671-2680, XP002313870
- DATABASE MEDLINE ON DIALOG, US NATIONAL LIBRARY OF MEDICINE, (Bethesda, MD, USA), BROUQUI P. et al., "Serologic Diagnosis of Human Monocytic Ehrlichiosis by Immunoblot Analysis", XP002916761; & CLINICAL DIAGNOSTIC LABORATORY IMMUNOLOGY, November 1994, Vol. 1, No. 6, pages 645-649.
- DATABASE MEDLINE ON DIALOG, US NATIONAL LIBRARY OF MEDICINE, (Bethesda, MD, USA), BROUQUI P. et al., "Antigenic Characterization of Ehrlichiae: Protein Immunoblotting of Ehrlichia Canis, Ehrlichia Sennetsu and Ehrlichia Risticii"; XP002916762; & JOURNAL OF CLINICAL MICROBIOLOGY, May 1992, Vol. 30, No. 5, pages 1062-1066.
- DATABASE MEDLINE ON DIALOG, US NATIONAL LIBRARY OF MEDICINE, (Bethesda, MD, USA), CHEN S.M. et al., "Identification of the Antigenic Constituents of Ehrlichia Chaffeensis", XP002916763; & AMERICAN JOURNAL OF TROPICAL MEDICINE AND HYGIENE, January 1994, Vol. 50, No. 1, pages 52-58.
- DATABASE MEDLINE ON DIALOG, US NATIONAL LIBRARY OF MEDICINE, (Bethesda, MD, USA), CHEN SHENG-MIN et al., "Analysis and Ultrastructural Localization of Ehrlichia Chaffeensis Proteins with Monoclonal Antibobies", XP002916764; & THE AMERICAN JOURNAL OF TROPICAL MEDICINE AND HYGIENE, April 1996, Vol. 54, No. 4, pages 405-412.
- DATABASE MEDLINE ON DIALOG, US NATIONAL LIBRARY OF MEDICINE, (Bethesda, MD, USA), CHEN S.M. et al., "Western Immunoblotting Analysis of the Antibody Responses of Patients with Human Monocytotropic Ehrlichiosis to Different Strains of Ehrlichia Chaffeensis and Ehrlichia Canis", XP002916765; & CLINICAL DIAGNOSTIC AND LABORATORY IMMUNOLOGY, November 1997, Vol. 4, No. 6, pages 731-735.
- DATABASE MEDLINE ON DIALOG, US NATIONAL LIBRARY OF MEDICINE, (Bethesda, MD, USA), DAWSON J.E. et al., "The Interface between Research and the Diagnosis of an Emerging Tick-Borne Disease, Human Ehrlichiosis Due to Ehrlichia Chaffeensis"; & ARCHIVES OF INTERNAL MEDICINE, 22 January 1996, Vol. 156, No. 2, pages 137-end.
- DATABASE MEDLINE ON DIALOG, US NATIONAL LIBRARY OF MEDICINE, (Bethesda, MD, USA), KELLY P.J. et al., "Serological Evidence for Antigenic Relationships between Ehrlichia Canis and Cowdria Ruminantiu", XP002916767; & RESEARCH IN VETERINARY SCIENCE, March 1994, Vol. 56, No. 2, pages 170-174.
- DATABASE MEDLINE ON DIALOG, US NATIONAL LIBRARY OF MEDICINE, (Bethesda, MD, USA), RIKIHISA Y. et al., "Enzyme Linked Immunosorbent Assay and Western Immunoblot Analyses of Ehrlichia- Canis and Canine Granulocytic Ehrlichia Infection", XP002916768; & JOURNAL OF CLINICAL MICROBIOLOGY, January 1992, Vol. 30, No. 1, pages 143-148.
- DATABASE MEDLINE ON DIALOG, US NATIONAL LIBRARY OF MEDICINE, (Bethesda, MD, USA), YU X.J. et al., "Sequence and Characterization of an Ehrlichia Chaffeensis Gene Encoding 314 Amino Acids Highly Homologous to the NAD A Enzyme", XP002916769; & FEMS MICROBIOLOGY LETTERS, 01 September 1997, Vol. 154, No. 1, pages 53-58.
- YU ET AL.: J. CLIN. MICROBIOL., 1999, pages 1137-1143,
- YU ET AL.: GENE, 2000, pages 24859-24868,
- OHASHI ET AL.: INFECTION AND IMMUNITY, 2001, pages 2083-2091,

## Description

This work was supported by grant ROI A140934 from National Institutes of Health. The government has certain rights in this invention.

### BACKGROUND OF THE INVENTION

The ehrlichiae are obligate intracellular bacteria that infect circulating leucocytes. *Ehrlichia chaffeensis* infects the monocytes and macrophages in humans and causes human monocytic ehrlichiosis. The clinical manifestations of ehrlichiosis in humans are nonspecific and similar to Rocky Mountain spotted fever. The clinical manifestations include fever, chills, headache myalgia or vomiting and weight loss. Most patients have a history of tick exposure.

*Ehrlichia canis* infects and causes ehrlichiosis in animals belonging to the family Canidae. Canine ehrlichiosis consists of an acute and a chronic phase. The acute phase is characterized by fever, serous nasal and ocular discharges, anorexia, depression, and loss of weight. The chronic phase is characterized by severe pancytopenia, epistaxis, hematuria, blood in feces in addition to more severe clinical signs of the acute disease. If treated early during the course of the disease, dogs respond well to doxycycline. However, chronically infected dogs do not respond well to the antibiotic. Therefore, early diagnosis is very important for treating canine ehrlichiosis.

The primary diagnostic test for diagnosing canine ehrlichiosis and human ehrlichiosis is the indirect fluorescent antibody (IFA) test. This test uses the etiologic agent *Ehrlichia canis* to diagnose canine ehrlichiosis. The IFA test uses *Ehrlichia chaffeensis* as antigen for diagnosing human ehrlichiosis. The IFA test has, however, serious limitations. The IFA test is subject to false positives because the antigens are made of whole infected cells which comprise many nonspecific proteins which will cross-react with sera from some patients. The IFA test is also subject to false negatives because IFA antigens are unstable and may become inactivated during storage. In addition the IFA test requires a special equipment to perform the test. For example, the IFA test requires a tissue culture system for growing the bacterium that are used to prepare the antigen slides, a fluorescent microscope, and trained persons to evaluate the serum reactivity to the bacterial antigen on the slide.

Tools which permit simpler, more rapid, and objective serodiagnosis of canine ehrlichiosis or human ehrlichiosis are desirable.

XUEJIE YU ET AL. ("Detection of Ehrlichia chaffeensis in human tissue by using a species-specific monoclonal antibody" JOURNAL OF CLINICAL MICROBIOLOGY, vol. 31 , 1993, pages 3284-3288) discuss the use of monoclonal antibody (Mab1A9) to detect E. chaffeensis in human tissue. This antibody was reported to react with proteins having masses 29 and 27 kDa.

N. OHASHI ET AL. ("Immunoprotective 28-kDa outer membrane protein of Ehrlichia chaffeensis is a member of multi-sized protein antigen family" ABSTRACTS OF THE 97TH GENERAL MEETING OF THE AMERICAN SOCIETY FOR MICROBIOLOGY, 4 May 1997, page 221) report the identification of five major outer membrane proteins from E. chaffeensis. A recombinant 28k kDa protein was reported to be immunoprotective. However detailed characterisation of the proteins was not disclosed.

NORIO OHASHI ET AL. "Immunodominant Major Outer Membrane proteins of Ehrlichia chaffeensis are encoded by a polymorphic multigene family" INFECTION AND IMMUNITY, vol. 66, no. 1, January 1998, pages 132-139, was published after the priority date of the present application. This paper describes several immunodominant major proteins in the 23 to 30 kDa range from Ehrlichia chaffeensis and Ehrlichia canis.

G. ROMAN REDDY ET AL. "Molecular characterization of a 28 kDa surface antigen gene family of the tribe Ehrlichiae" BIOCHEMICAL AND BIOPHYSICAL RESEARCH COMMUNICATIONS, vol. 247, June 1998, pages 636-643, was published after the priority date of the present application. This paper purported to analyze the structure the 28 kDa antigen genes from Ehrlichia chaffeensis, Ehrlichia canis and Cowdria ruminanium.

### SUMMARY OF THE INVENTION

The present invention relates to improved diagnostic tools for veterinary and human use which are used for serodiagnosing ehrlichiosis in mammals, particularly in members of the Canidae family and in humans.

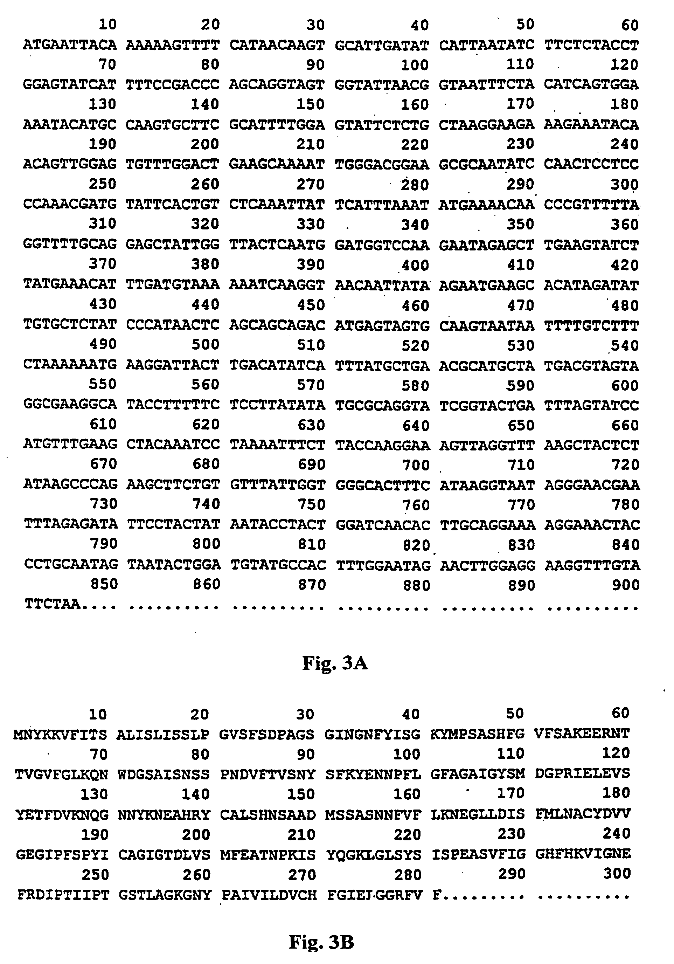

The present disclosure provides polynucleotides or nucleic acids which encode the outer membrane proteins of *E. chaffeensis.* The OMP-1 polynucleotide encodes an OMP-1 protein of *E*. *chaffeensis* having a molecular weight of about 27.7 kDa and an amino acid sequence which is shown in FIG.3B.

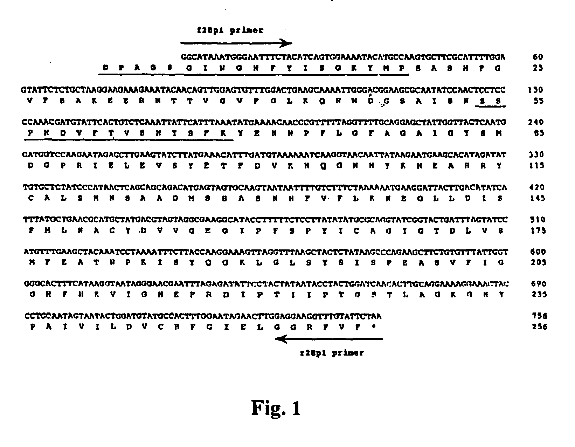

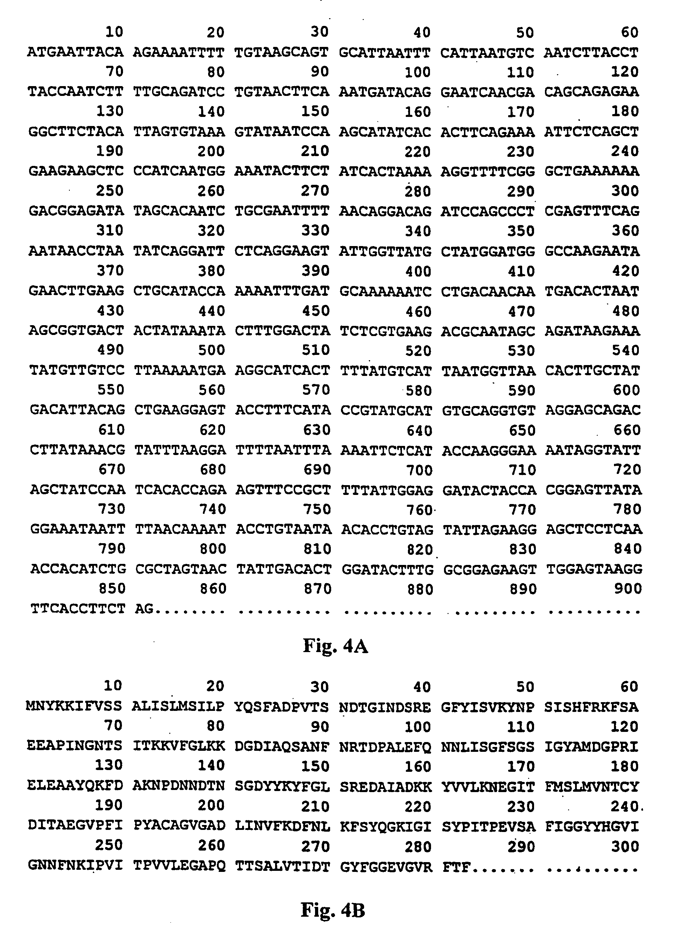

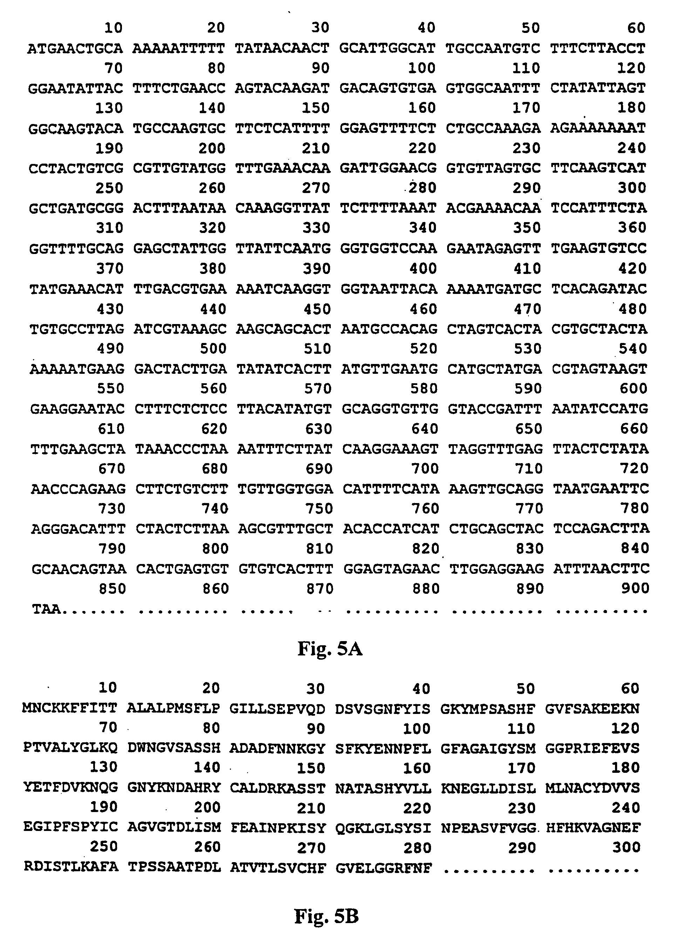

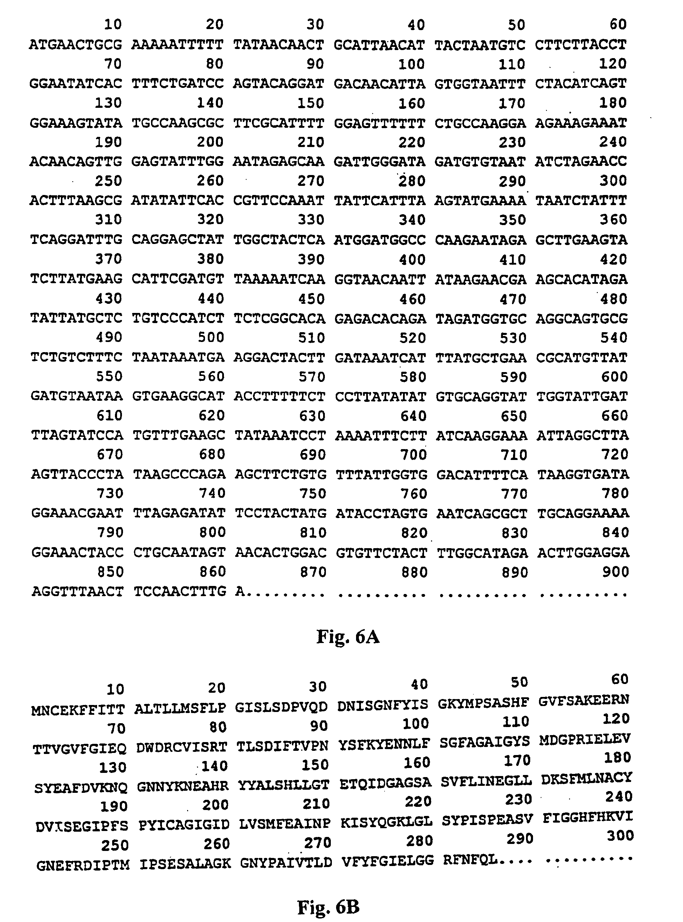

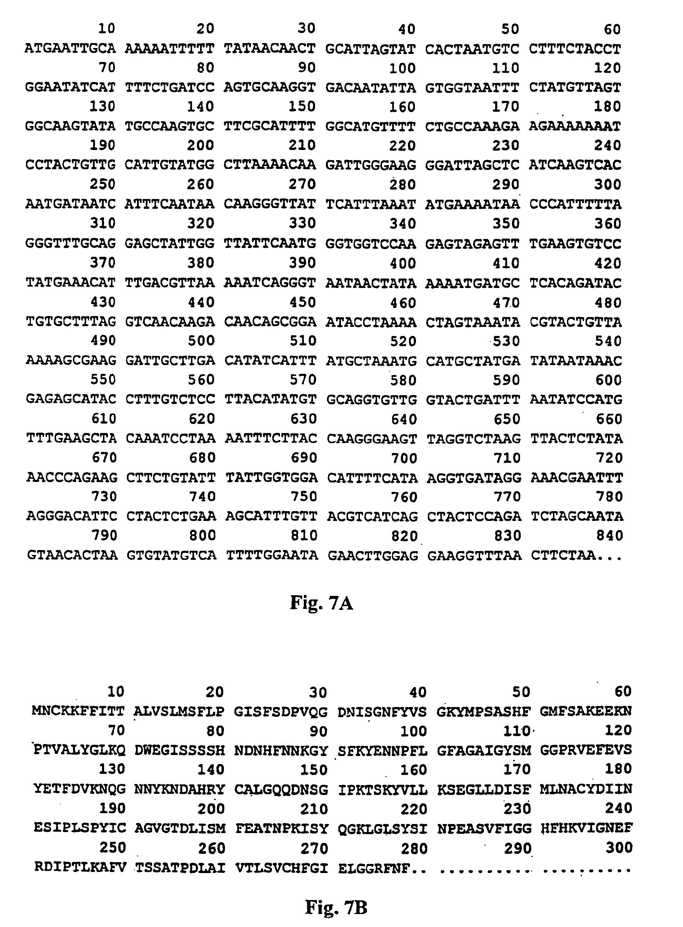

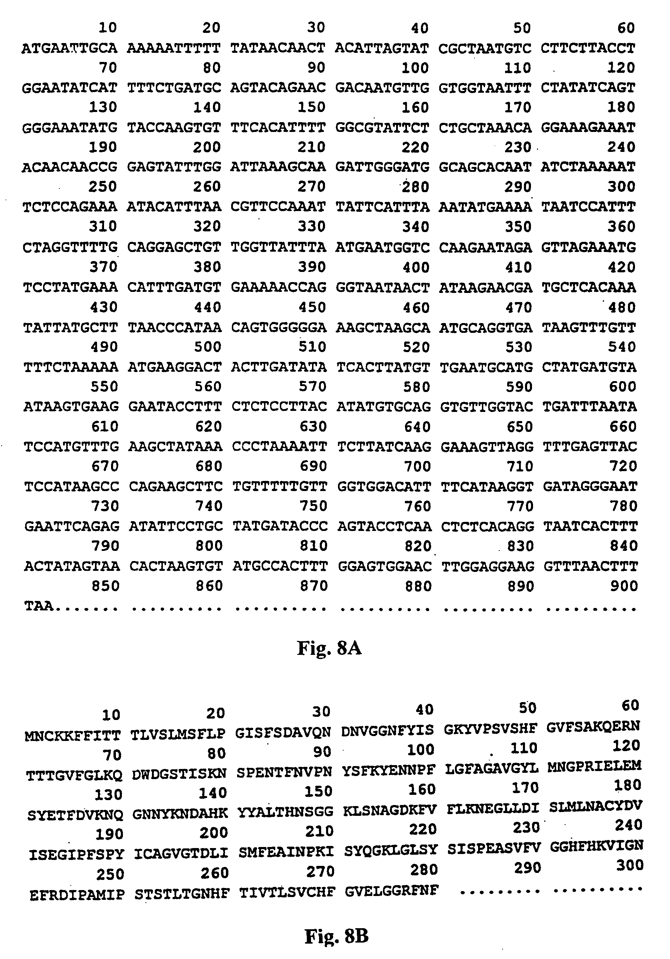

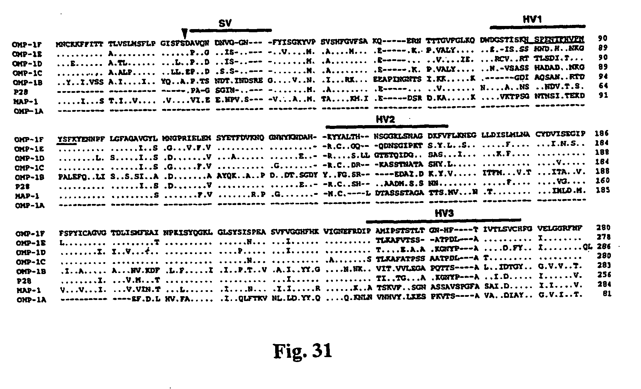

The present invention also provides the following polynucleotides or nucleic acids which encode the outer membrane proteins of *E*. *chaffeensis* selected from the group:
(a) an isolated outer membrane protein of *E*. *chaffeensis* or an immunogenic fragment thereof, wherein the outer membrane protein comprises an amino acid sequence which is at least 85% homologous to a sequence selected from the group consisting of
   the amino acid sequence in Fig. 1 corresponding to P28,
   the amino acid sequence in Fig. 4B corresponding to OMP-1B,
   the amino acid sequence in Fig. 5B corresponding to OMP-1C,
   the amino acid sequence in Fig. 6B corresponding to OMP-1D,
   the amino acid sequence in Fig. 7B corresponding to OMP-1E,
   the amino acid sequence in Fig. 8B corresponding to OMP-1F,
   wherein each outer membrane protein or immunogenic fragment thereof is useful for producing an antibody that is immunospecific for the corresponding outer membrane protein;
(b) an isolated rP28 immunogenic fragment of P28 protein, said fragment comprising a sequence identical to the amino acid sequence extending from amino acid 6 through amino acid 256 in Fig. 1, wherein the rP28 immunogenic fragment binds to antibodies in serum from a human infected with *E*. *chaffeensis;* and
(c) an isolated OMP-1A protein fragment comprising the OMP-1A amino acid sequence in Fig. 31.

The present invention also provides the following isolated proteins of *E*. *chaffeensis*:
(a) an isolated outer membrane protein of *E*. *chaffeensis* or an immunogenic fragment thereof, wherein the outer membrane protein comprises an amino acid sequence which is at least 85% homologous to a sequence selected from the group consisting of
   the amino acid sequence in Fig. 1 corresponding to P28,
   the amino acid sequence in Fig. 4B corresponding to OMP-1B,
   the amino acid sequence in Fig. 5B corresponding to OMP-1C,
   the amino acid sequence in Fig. 6B corresponding to OMP-1D,
   the amino acid sequence in Fig. 7B corresponding to OMP-1E,
   the amino acid sequence in Fig. 8B corresponding to OMP-1F,
   wherein each outer membrane protein or immunogenic fragment thereof is useful for producing an antibody that is immunospecific for the corresponding outer membrane protein;
(b) an isolated rP28 immunogenic fragment of P28 protein, said fragment comprising a sequence identical to the amino acid sequence extending from amino acid 6 through amino acid 256 in Fig. 1, wherein the rP28 immunogenic fragment binds to antibodies in serum from a human infected with *E*. *chaffeensis*; and
(c) an isolated OMP-1A protein fragment comprising the OMP-1A amino acid sequence in Fig. 31.

The outer membrane proteins from *E. chaffeensis,* particularly a recombinant form of OMP-1, are immunogenic and, thus are useful for preparing antibodies. Such antibodies are useful for immunolabeling isolates of *E. chaffeensis* and for detecting the presence of *E chaffeensis* in body fluids, tissues, and particularly in monocytes and macrophages. The isolated outer membrane proteins, particularly OMP-1, are also useful for detecting antibodies to *E. chaffeensis* in the blood of patients with clinical signs of ehrlichiosis. The isolated outer membrane protein, particularly OMP-1, are also useful immunogens for raising antibodies that are capable of reducing the level of infection in an immunized mammal that has been infected with *E*. *chaffeensis.* The isolated membrane proteins are also useful in a vaccine for protecting against infection with *E. chaffeensis.*

The present disclosure also relates to isolated polynucleotides which encode 30 kDa outer membrane proteins from *Ehrlichia canis*. The proteins are designated P30 and P30a. The proteins, particularly P30, are immunogenic and are, thus, useful for preparing antibodies that are useful for immunolabeling isolates of *E cans*. The P30 protein is also useful for diagnosing canine ehrlichiosis in mammals, particularly in members of the family Canidae, most particularly in dogs and for diagnosing infections with *E. chaffeensis* in humans. The P30 protein is also a useful immunogen for raising antibodies that reduce the level of infection in an immunized mammal that has been infected with *E*. *canis*. The P30 protein is also useful in a vaccine for protecting animals against infection with *E. canis*.

The present specification also describes the following isolated proteins of *E. chaffeensis* OMP-1 (also known in its mature form as p28), OMP-1A, OMP-1B, OMP-1C, OMP-1D, OMP-1E, OMP-1F, OMP-1R, OMP-1S, OMP-1T, OMP -1U, OMP-1V, OMP-1W, OMP-1X, and OMP-1Z, referred to hereinafter collectively as the "OMP family". The present specification also describes the following isolated proteins of *E*. *canis* P30, P30-a, P30-1, P30-2, P30-3, P30-4, P30-5, P30-6, P30-7, P30-8, P30-9, and P30-10, referred to hereinafter as the P30 family.

The present disclosure also relates to an assay for diagnosing ehrlichiosis in humans using a recombinant outer membrane protein of *E. chaffensis*, particularly OMP-1. The present disclosure also relates to an assay for diagnosing ehrlichiosis in humans and members of the family Canidae using a recombinant outer membrane protein of *E*. *canis,* particularly P30.

### Brief Description of the Figures

FIG. 1. shows the DNA sequence and the amino acid sequence encoded by the *E*. *chaffeensis (p28)* gene cloned in pCRII*p28*. The N-terminal amino acid sequence of native *omp-1* protein (P28) determined chemically is underlined. Five amino acid residues at the N terminus of P28 which were not included in the *p28* gene, are indicated by boldface. Arrows indicate annealing positions of the primer pair designed for PCR
FIG. 2. shows the restriction map of 6.3-kb genomic DNA including the *omp-1* gene copies in *E*. *chaffeensis*. The four DNA fragments were cloned from the genomic DNA (pPS2.6. pPS3.6, pEC2.6, and pEC3.6). A recombinant plasmid pPS2.6 has an overlapping sequence with that of pEC3.6. The closed boxes at the bottom show PCR-amplified fragments from the genomic DNA for confirmation of the overlapping area. Open boxes at the top indicate open reading frames (ORF) of *omp-1* gene copies with direction by arrows. Open boxes at the bottom show DNA fragments subcloned for DNA sequencing.
FIG. 3B shows one embodiment of the OMP-1 protein; FIG. 3A shows one embodiment of the OMP-1 polynucleotide.
FIG. 4B shows one embodiment of the OMP-1B protein, FIG. 4A shows one embodiment of the OMP-1B polynucleotide
FIG. 5A shows one embodiment of the OMP-1C polynucleotide; FIG 5B shows one embodiment of the OMP-1C protein.
FIG. 6B shows one embodiment of the OMP-1D protein; FIG. 6A shows one embodiment of the OMP-1D polynucleotide.
FIG. 7B shows one embodiment of the OMP-1E protein; FIG 7A shows one embodiment of the OMP-1E polynucleotide.
FIG. 8B shows one embodiment of the OMP-1F protein; FIG 8A shows one embodiment of the OMP-I F polynucleotide.
FIG. 9B shows one embodiment of the OMP-1A protein, FIG 9A shows one embodiment of the OMP-1A polynucleotide.

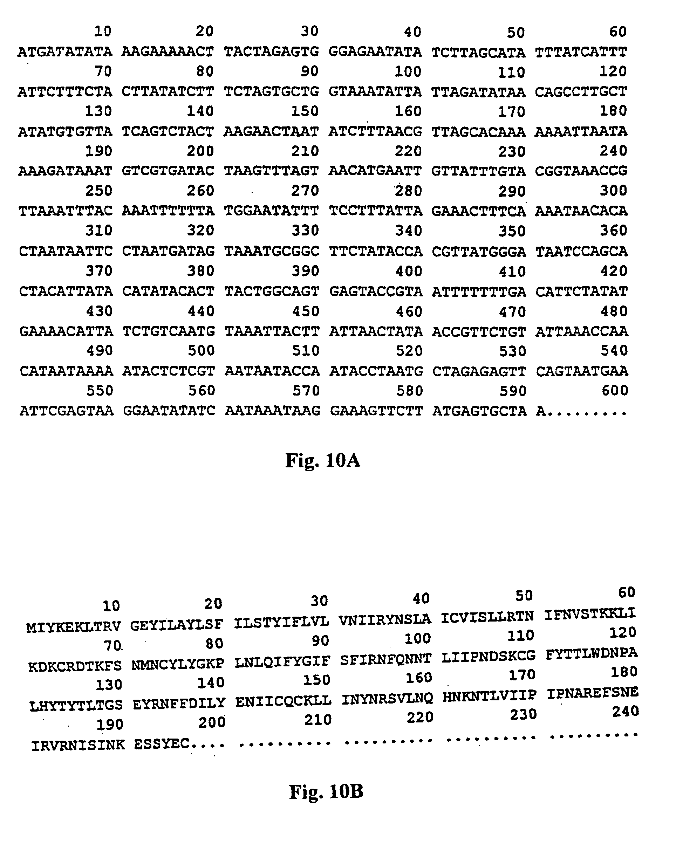

FIG. 10 B shows one embodiment of a portion of the OMP-1R protein. FIG 10A shows one embodiment of an OMP-1R polynucleotide encoding such polypeptide.

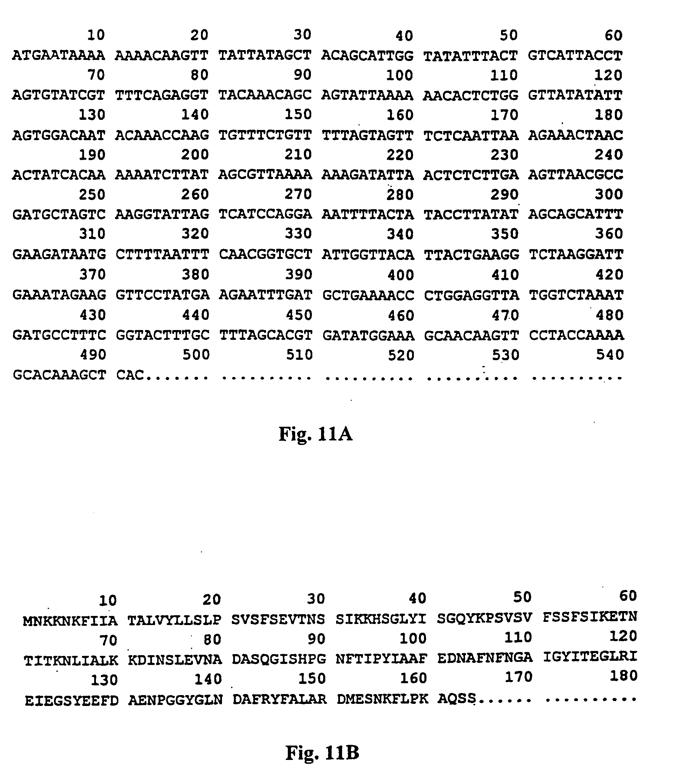

FIG. 11 B shows one embodiment of a portion of the OMP-1S protein. FIG 11A shows one embodiment of the OMP-1S polynucleotide encoding such polypeptide.

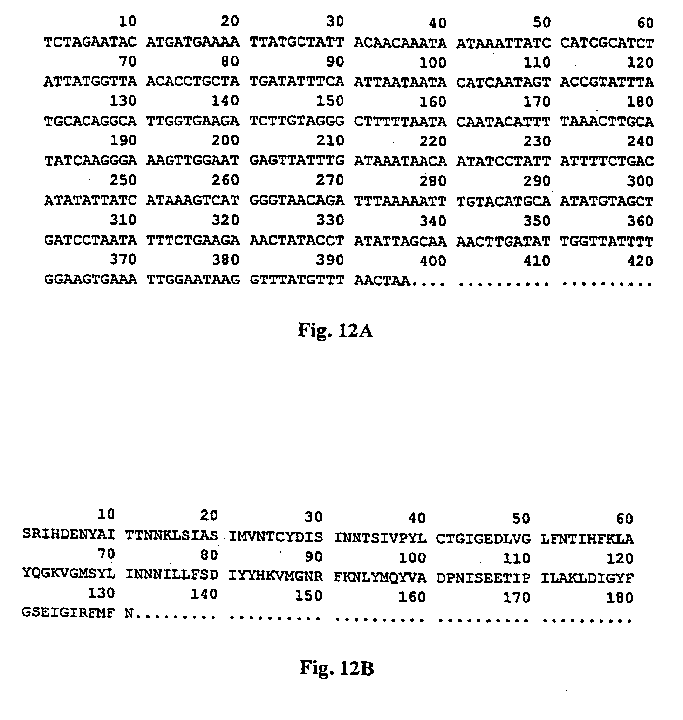

FIG. 12 B shows one embodiment of a portion of the OMP-1T protein, FIG 12A shows one embodiment of the OMP-1T polynucleotide encoding such polypeptide.

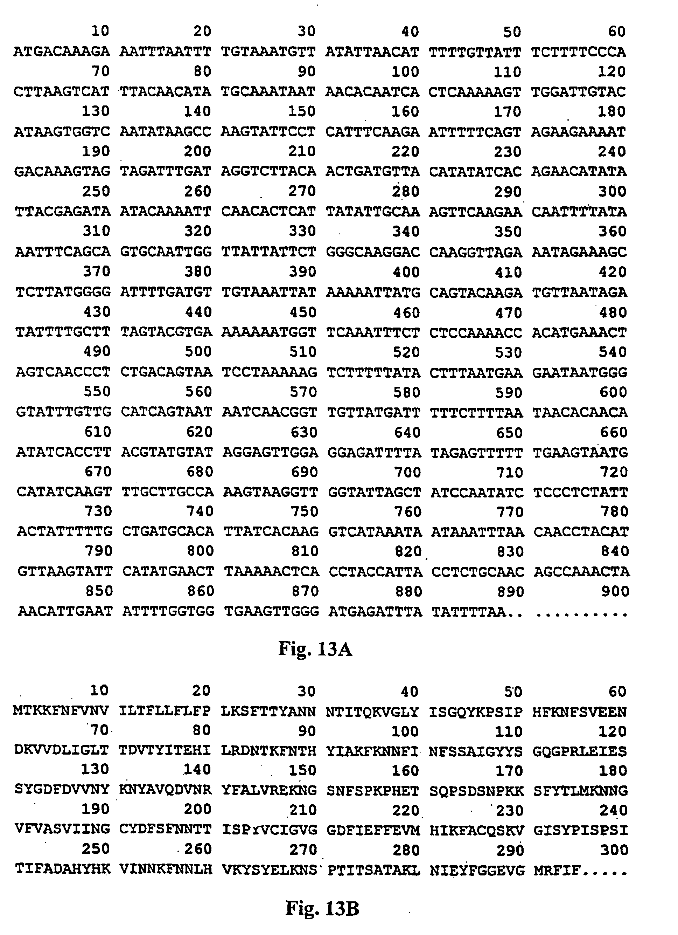

FIG. 13 B shows one embodiment of the OMP-1U protein, FIG 13A shows one embodiment of the OMP-1U polynucleotide.

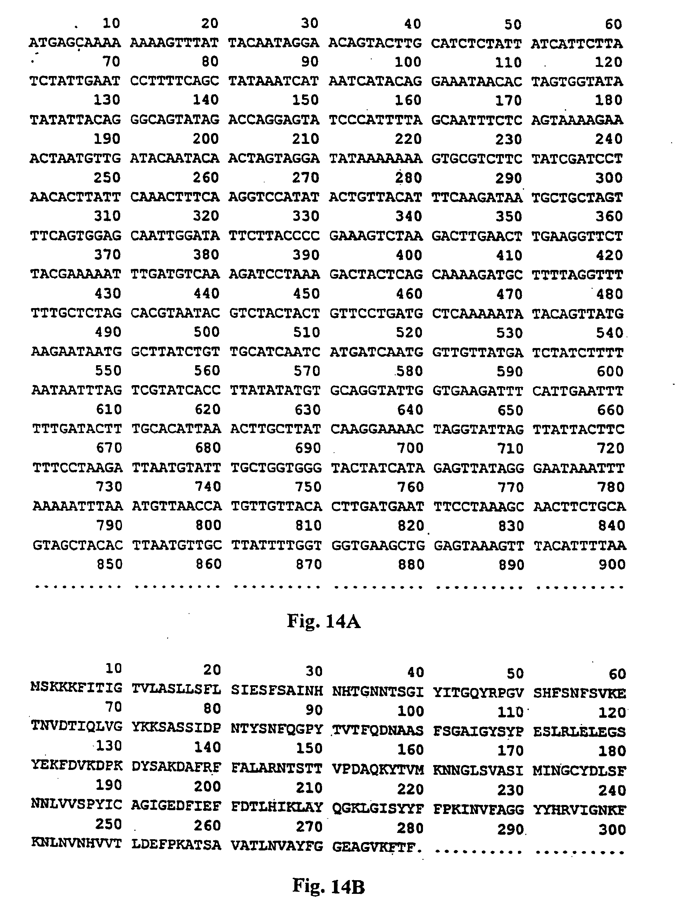

FIG. 14 B shows one embodiment of the OMP-1V protein, FIG 14A shows one embodiment of the OMP-1V polynucleotide.

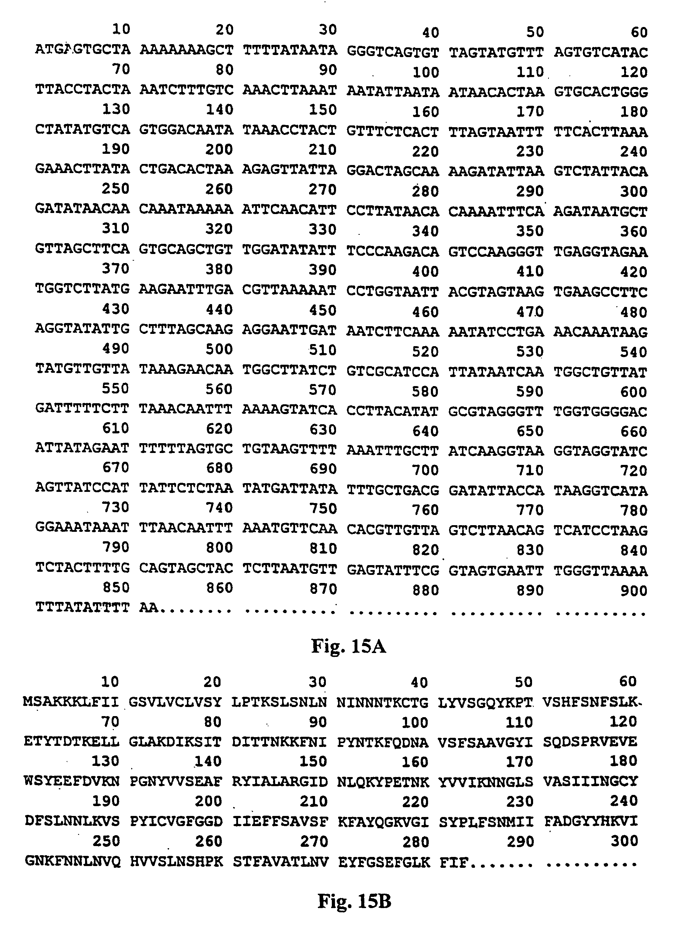

FIG. 15 B shows one embodiment of the OMP-1W protein, FIG 15A shows one embodiment of the OMP-1W polynucleotide.

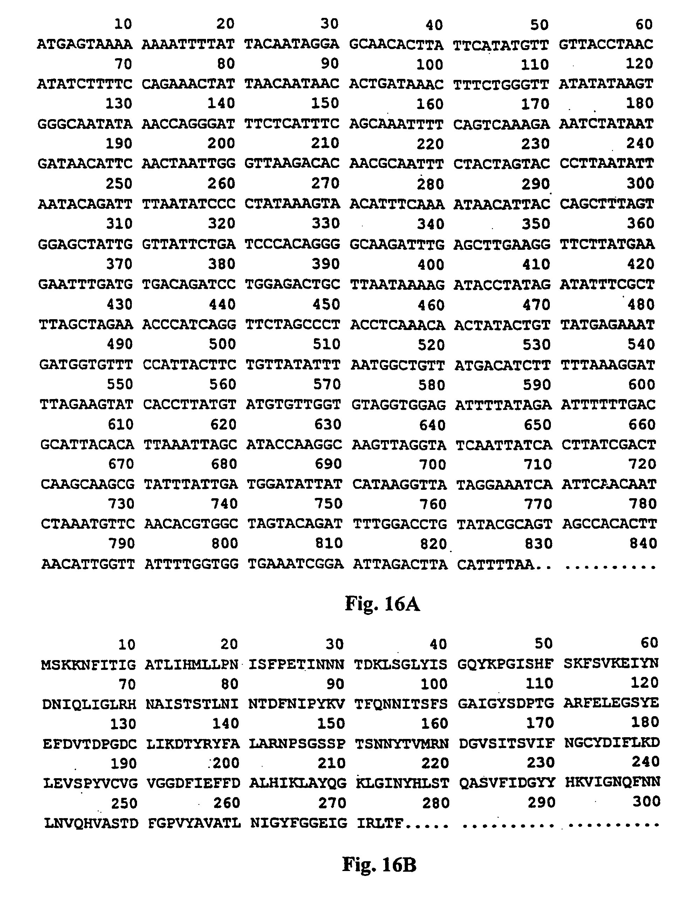

FIG. 16 B shows one embodiment of the OMP-1X protein, FIG 16A shows one embodiment of the OMP-1X polynucleotide.

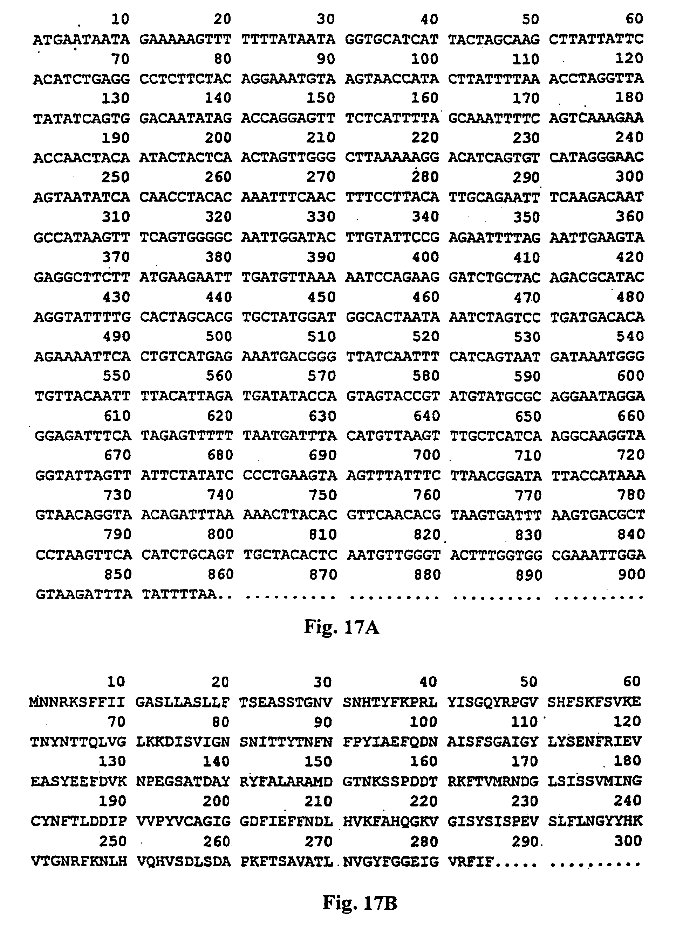

FIG. 17 B shows one embodiment of the OMP-1Y protein, FIG 17A shows one embodiment of the OMP-1Y polynucleotide.

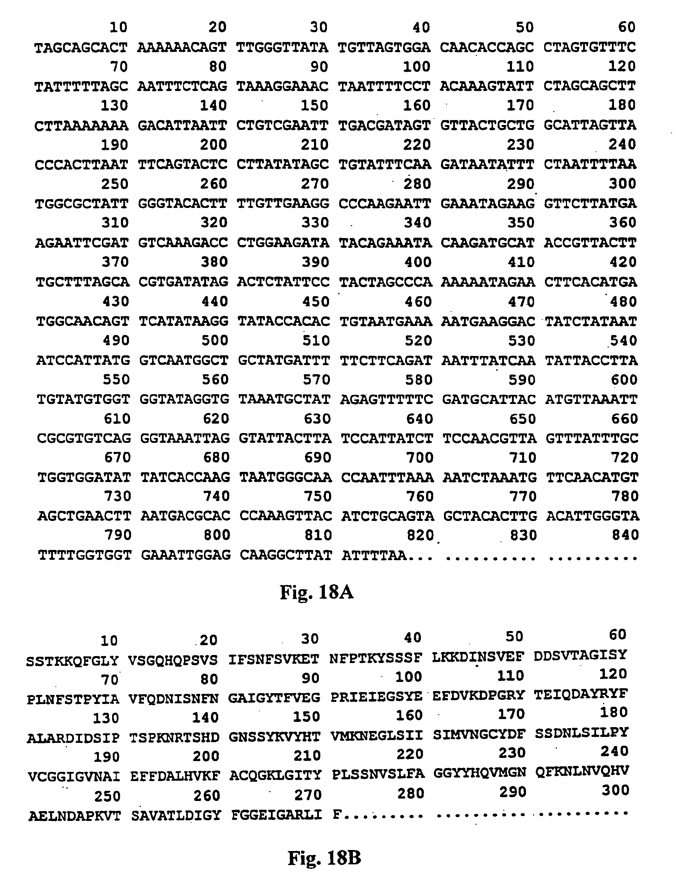

FIG. 18 B shows one embodiment of the OMP-1Z protein. FIG 18A shows one embodiment of the OMP-1Z polynucleotide.

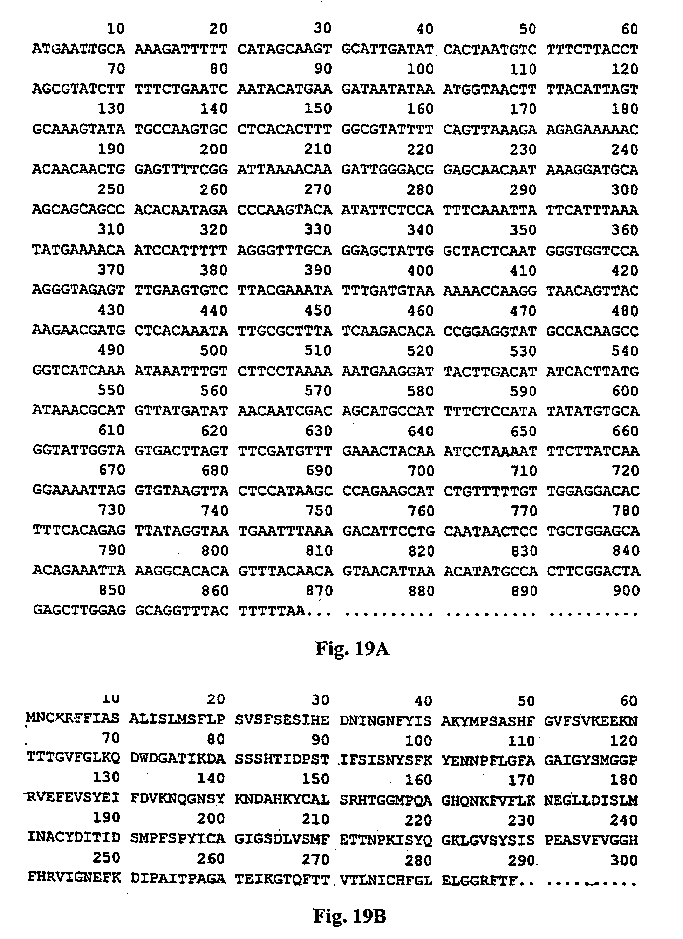

FIG. 19 B shows one embodiment of the P30 protein, FIG 19A shows one embodiment of the P30 polynucleotide.

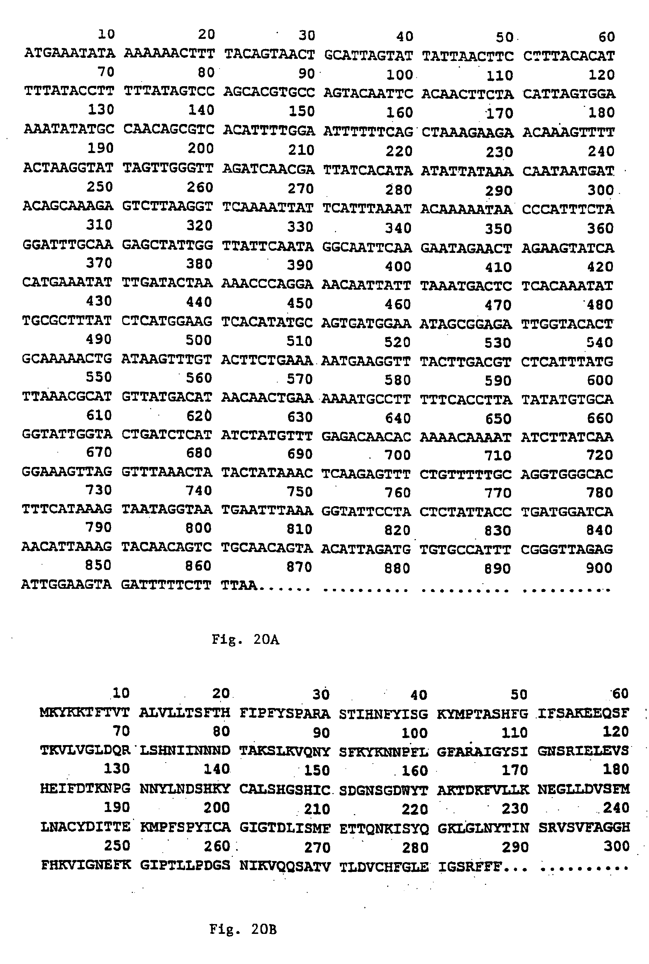

FIG. 20 B shows one embodiment of the P30a protein, FIG 20A shows one embodiment of the p30A polynucleotide.

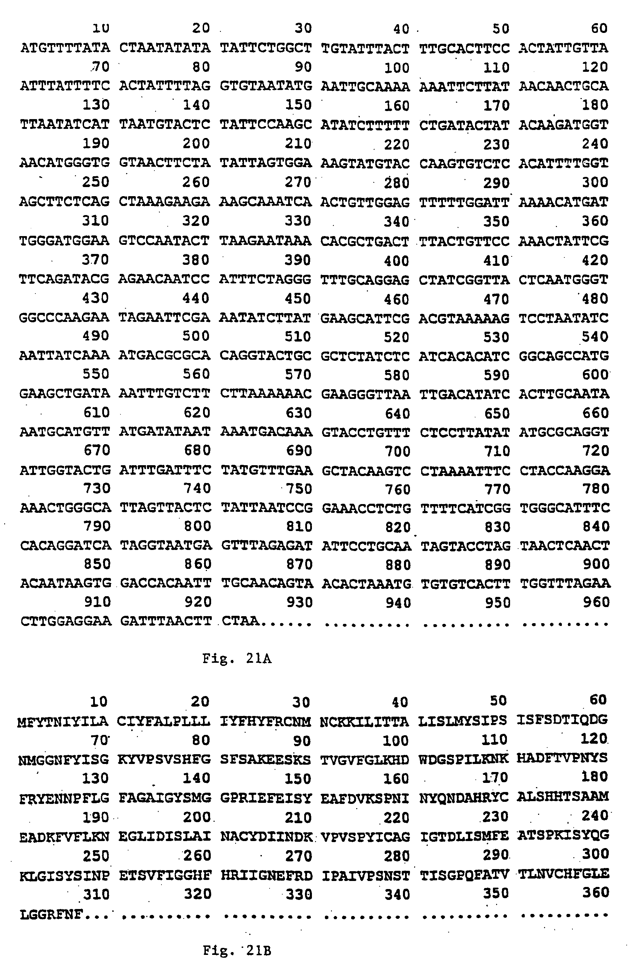

FIG. 21 B shows one embodiment of the P30-1 protein, FIG 21A shows one embodiment of the p30-1 polynucleotide.

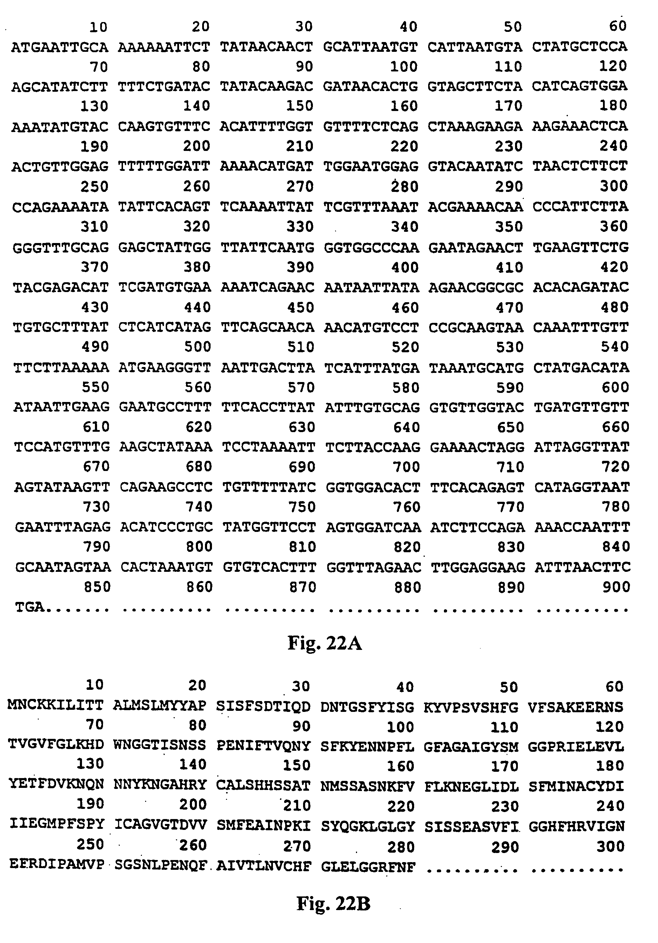

FIG. 22 B shows one embodiment of the P30-2 protein, FIG 22 A shows one embodiment of the p30-2 polynucleotide.

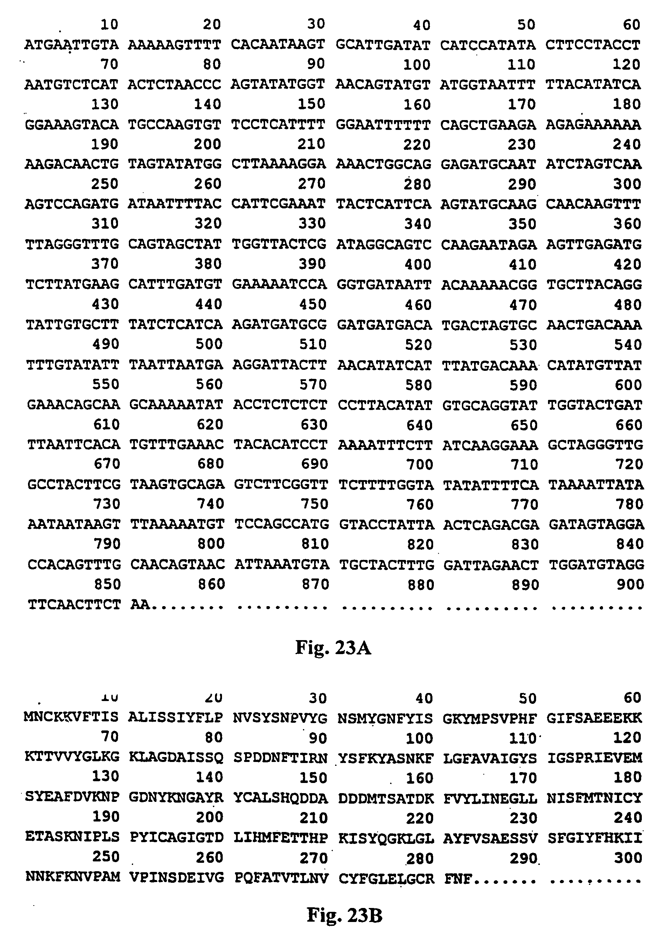

FIG. 23 B shows one embodiment of the P30-3 protein, FIG 23 A shows one embodiment of the p30-3 polynucleotide.

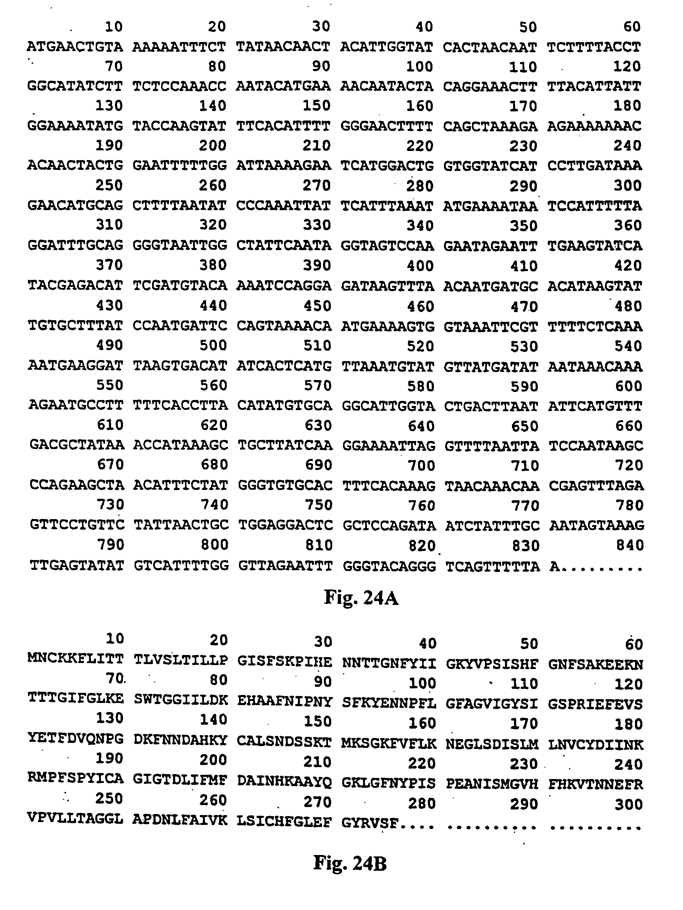

FIG. 24 B shows one embodiment of the P30-4 protein, FIG 22 A shows one embodiment of the p30-4 polynucleotide.

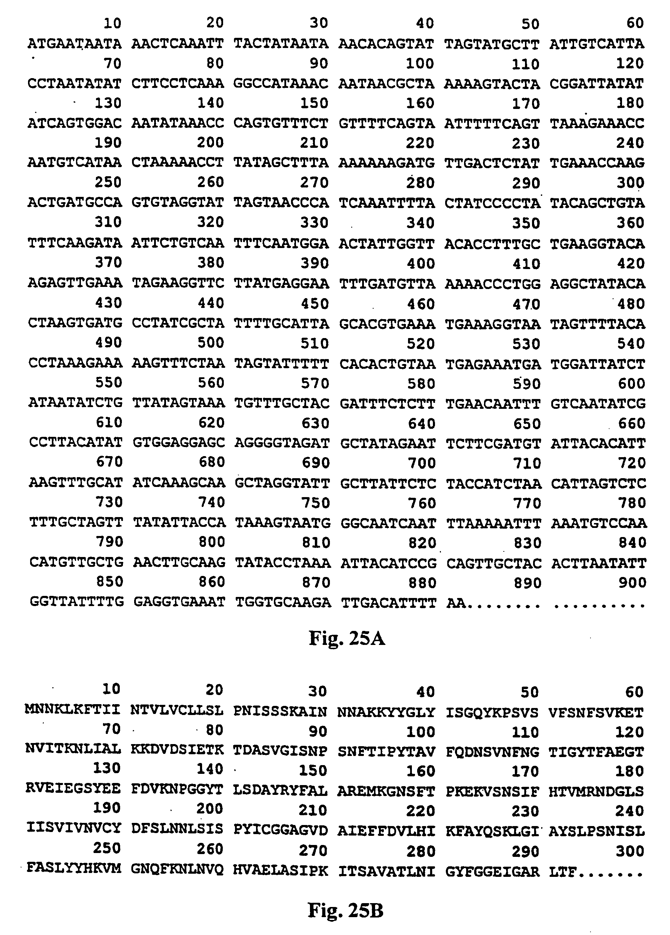

FIG. 25 B shows one embodiment of the P30-5 protein, FIG 22 A shows one embodiment of the p30-5 polynucleotide.

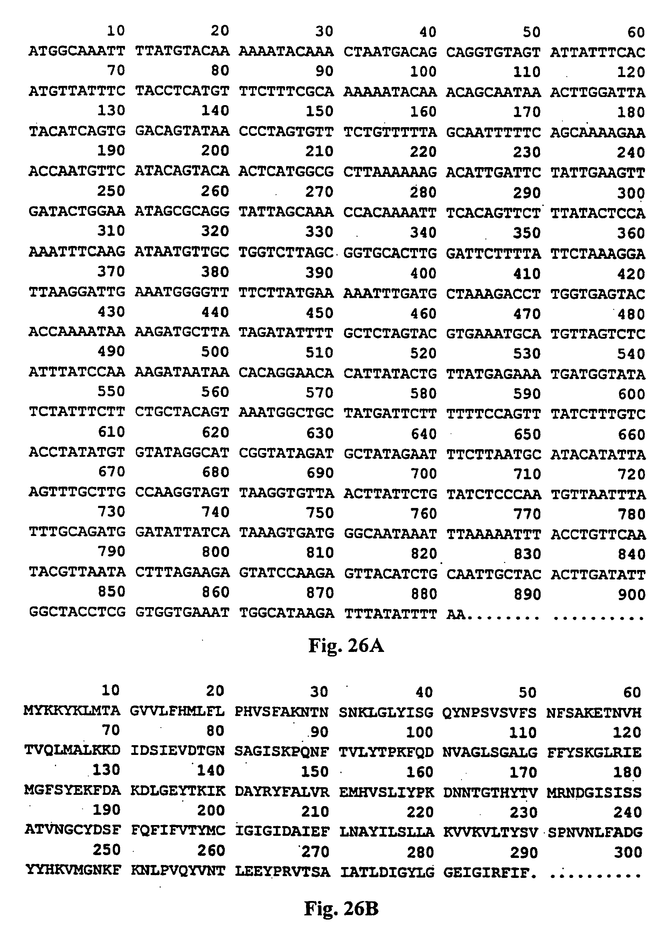

FIG. 26 B shows one embodiment of the P30-6 protein, FIG 26 A shows one embodiment of the p30-6 polynucleotide.

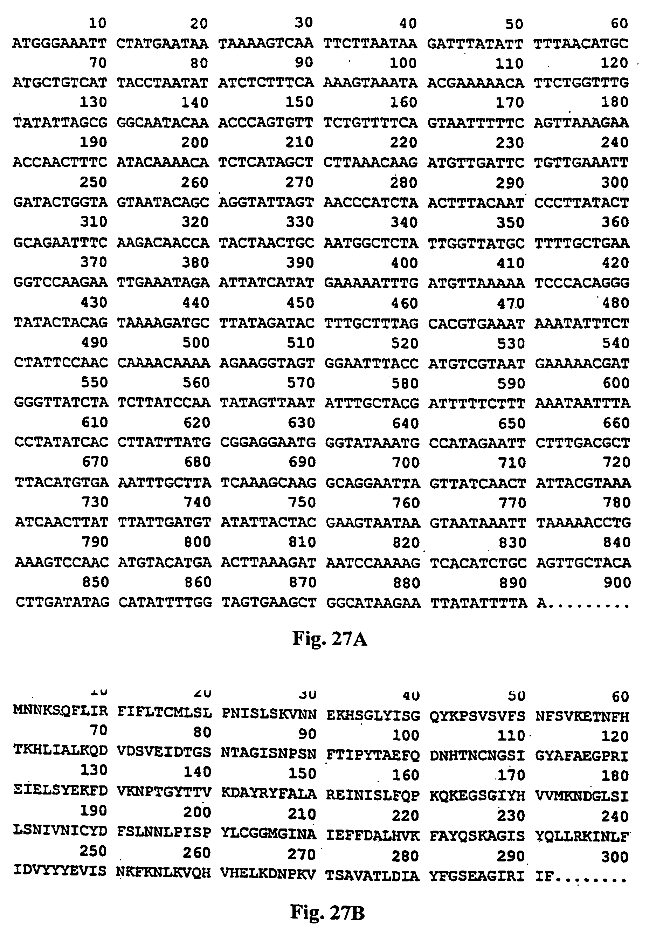

FIG. 27 B shows one embodiment of the P30-7 protein, FIG 27 A shows one embodiment of the p30-7 polynucleotide.

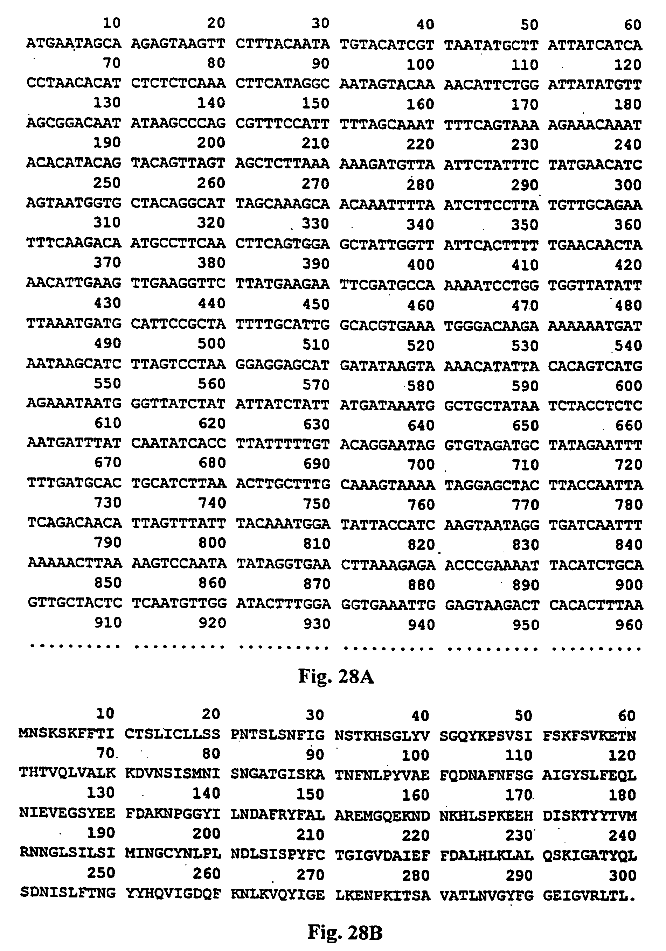

FIG. 28 B shows one embodiment of the P30-8 protein, FIG 28 A shows one embodiment of the p30-8 polynucleotide.

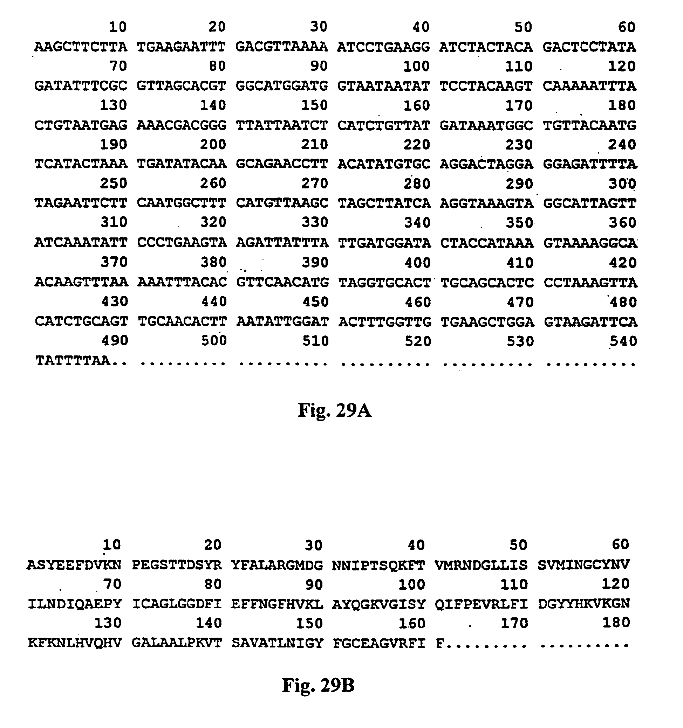

FIG. 29 B shows one embodiment of a portion of the P30-9 protein. FIG 29 A shows one embodiment of the p30-9 polynucleotide.

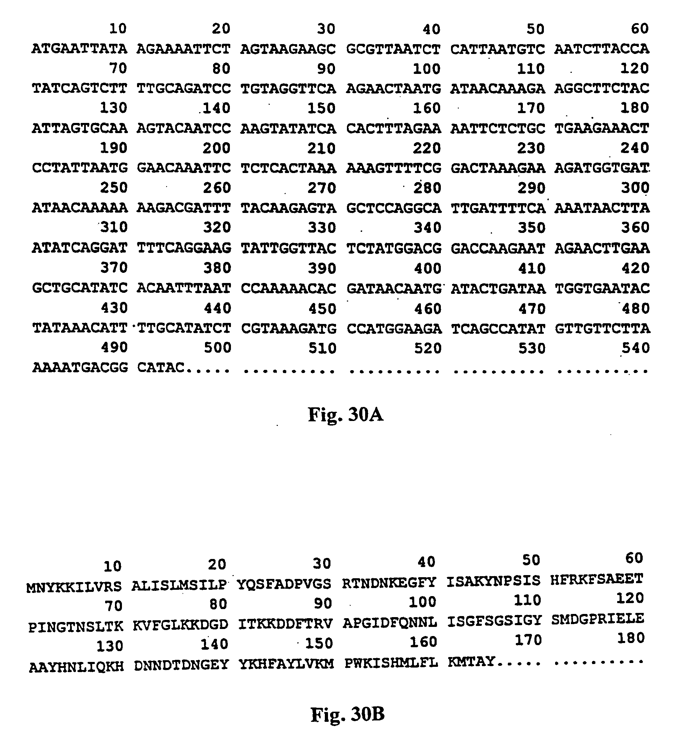

FIG. 30 B shows one embodiment of a portion of the P30-10 protein, FIG 30 A shows one embodiment of the p30-10 polynucleotide encoding such protein.
FIG. 31 depicts the amino acid sequences alignment of seven *E*. *chaffeensis* OMP-1s and *Cowdria ruminantium* MAP-1. Aligned positions of identical amino acids with OMP-1F are shown with dots. The sequence of *C. ruminantium* MAP-1 is from the report of Van Vliet et al (1994) Molecular cloning, sequence analysis, and expression of the gene encoding the immunodominant 32-kilodalton protein of *Cowdria ruminantium.* Infect. Immun. 62:1451-1456. Gaps indicated by dashes were introduced for optimal alignment of all proteins. Bars indicates semivariable region (SV) and three hypervariable regions (HV1, HV2, and HV3).

### DETAILED DESCRIPTION OF THE INVENTION

### Isolated Polynucleotides Encoding OMP-1,OMP-1A, OMP-1B, OMP-1C, OMP-1D, OMP-I1F and the OMP from E. canis

In one aspect, the present invention, provides isolated polynucleotides that encode the outer membrane proteins, mature OMP-1 (or p28), OMP-1B, OMP-1C, OMP-1D, OMP- 1E, OMP-1F, and OMP-1A fragment as described in the claims from *E chaffeensis*.

The polynucleotide is single stranded or double stranded. The polynucleotide may be a DNA or RNA molecule, preferably a DNA molecule, and comprises a sequence which codes for the respective outer membrane protein. Preferably, the polynucleotide encodes at least the mature form of outer membrane protein. The polynucleotide optionally further comprises a leader sequence and encode an outer membrane preprotein that is processed in the cell to form the mature protein. The polynucleotide of the present invention may also be fused in frame to a marker sequence which allows for purification of the corresponding outer membrane protein.

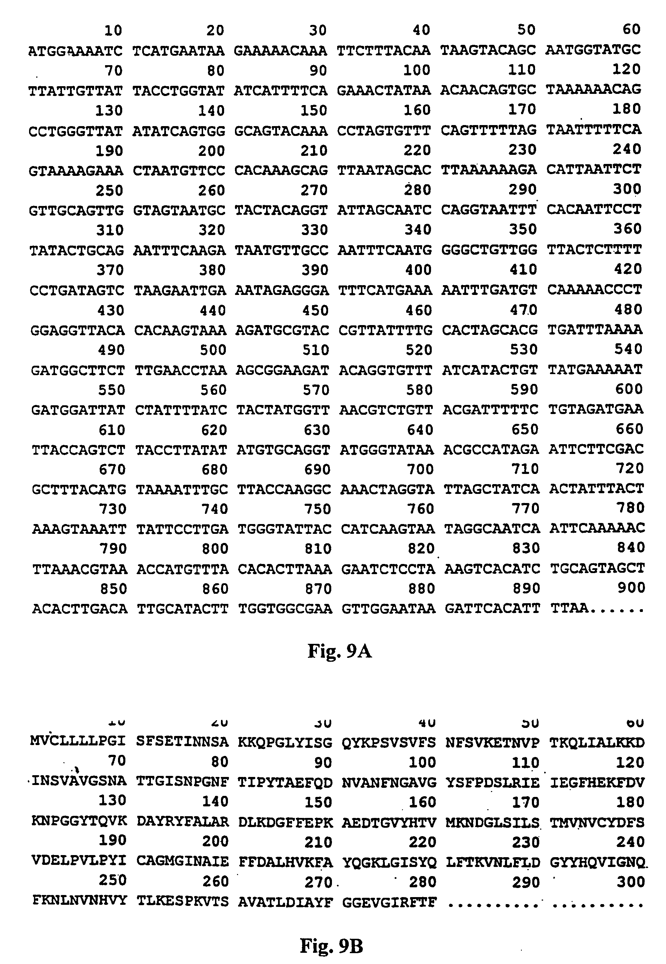

The OMP-1 polynucleotide encodes an OMP-1 protein of *E. chaffeensis* having a molecular weight of about 27.7 kDa and an amino acid sequence shown in FIG. 3B SEQ ID NO: 2; Figure 3B shows one embodiment of the OMP-1 protein, Figure 3A shows one embodiment of the OMP-1 polynucleotide. The OMP-1B polynucleotide encodes an OMP-1B protein of *E*. *chaffeensis* having a molecular weight of about 28.2 kDa and an amino acid sequence which is at least 85% homologous to the amino acid sequence shown in FIG. 4B SEQ ID NO: 4: Figure 4B shows one embodiment of the OMP-1B protein, Figure 4A shows one embodiment of the OMP-1B polynucleotide. The OMP-1C polynucleotide encodes an OMP-1C protein of *E. chaffeensis* having a molecular weight of about 27.6 kDa and an amino acid sequence which is at least 85% homologous to the amino acid sequence shown in FIG. 5B SEQ ID NO: 6; Figure 5B shows one embodiment of the OMP-1C protein, Figure 5A shows one embodiment of the OMP-1C polynucleotide. The OMP-1D polynucleotide encodes an OMP-1D protein of *E. chaffeensis* having a molecular weight of about 28.7 kDa and an amino acid sequence which is at least 85% homologous to the amino acid sequence shown in FIG. 6B SEQ ID NO: 8; Figure 6B shows one embodiment of the OMP-1D protein, Figure 6A shows one embodiment of the OMP-1D polynucleotide. The OMP-1E polynucleotide encodes an OMP-1E protein of *E. chaffeensis* having a molecular weight of about 27.8 kDa and an amino acid sequence which is at least 85% homologous to the amino acid sequence shown in FIG. 7B SEQ ID NO: 10: Figure 7B shows one embodiment of the OMP-1E protein, Figure 7A shows one embodiment of the OMP-1E polynucleotide. The OMP-1F polynucleotide encodes an OMP-1F protein of *E. chaffeensis* having a molecular weight of about 27.9 kDa and an amino acid sequence which is at least 85% homologous to the amino acid sequence shown in FIG. 8B, SEQ ID NO: 12; Figure 8B shows one embodiment of the OMP1F protein, Figure 8A shows one embodiment of the OMP-1F polynucleotide. The OMP-1A polynucleotide encodes an OMP-1A protein of *E. chaffeensis* having a molecular weight of about 29.6 kDa and an amino acid sequence which is at shown in FIG. 9B, SEQ ID NO: 14.

The polynucleotides encode an *E. chaffeensis* outer membrane protein having a sequence that is at least 83%, preferably at least 90%, more preferably at least 95% homologous to or similar to the amino acid sequences shown in Figures 3B through 8B, and thus embrace polynucleotides encoding outer membrane proteins from different strains of *E. chaffeensis*. The polynucleotides encode an outer membrane protein whose conserved regions collectively are at least 90%, preferably at 95%, more preferably at least 97% homologous to the conserved regions of the amino acid sequences of the present invention. The outer membrane proteins of *E chaffeensis* and *E canis* have six conserved regions, which are separated by one semivariable region and three hypervariable regions. The conserved regions of the outer membrane proteins OMP-1, OMP-1A, OMP-1B, OMP1-C, OMP-1D, OMP1-F are depicted in Fig. 31. Preferably, the amino acid sequence of the outer membrane proteins of E. chaffeensis are at least 30% divergent from the amino acid sequence of MAP-1. Such sequences include allelic, strain variants and other amino acid sequence variants (e.g., including "muteins" or "mutant proteins"), whether naturally-occurring or biosynthetically produced. As used herein. "amino acid sequence homology" is understood to mean amino acid sequence similarity, and homologous sequences share identical or similar amino acids, where similar amino acids are conserved amino acids as defined by Dayoff et al., Atlas of Protein Sequence and Structure; vol. 5, Supp. 3, pp. 345-362 (M. O. Dayoff, ed.. Nat'l BioMed. Research Fdn., Washington D.C. 1978.) Thus, a candidate sequence sharing 85% amino acid sequence homology with a reference sequence requires that, following alignment of the candidate sequence with the reference sequence, 85% of the amino acids in the candidate sequence are identical to the corresponding amino acid in the reference sequence, or constitute a conservative amino acid change thereto. "Amino acid sequence identity" is understood to require identical amino acids between two aligned sequences. Thus, a candidate sequence sharing 85% amino acid identity with a reference sequence requires that, following alignment of the candidate sequence with the reference sequence, 85% of the amino acids in the candidate sequence are identical to the corresponding amino acid in the reference sequence.

As used herein, all homologies and identities are calculated using the amino acid sequences shown in the cited Figure or SEQ ID NO as the reference sequence. Thus, to determine whether an amino acid sequence is 85% homologous to OMP-1, one uses the amino acid sequence shown in Fig. 3B, SEQ ID NO: 2 as a reference.

Also as used herein, sequences are aligned for homology and identity calculations using the method of the software basic local alignment search tool in the BLAST network service (the National Center for Biotechnology Information, Bethesda, MD) which employs the method of Altschul, S. F., Gish, W., Miller, W., Myers, E. W. & Lipman. D. J. (1990) J. Mol. Biol. 215, 403-410. Identities are calculated by the Align program (DNAstar, Inc.) In all cases, internal gaps and amino acid insertions in the candidate sequence as aligned are ignored when making the homology/identity calculation.

In another aspect, the present invention provides a nucleotide sequence encoding a polypeptide which comprises a fragment of the OMP1 protein, hereinafter referred to as "rP28". The rP28 polypeptide weighs approximately 31 kDa and comprises all but of the first 5 amino acids of mature OMP-1 protein, The rP28 polypeptide comprises the amino acid sequence extending from amino acid 6 through amino acid 256 of the amino acid sequence shown in Fig.1. The present invention also embraces polypeptides where one or more of the amino acids in the sequence extending from amino acid 1 or 6 through amino acid 251 Fig. 1 are replaced by conservative amino acid residues. The present invention also relates to derivatives of rP28 that have an amino acid sequence identity of at least 85%, more preferably at least 90%, and most preferably of at least 95% with the amino acid sequence extending from amino acid I or 6 through amino acid 251 of the protein and which derivative binds to antibodies in sera from humans infected with *E. chaffeensis.*

The polynucleotides are useful for producing the outer membrane proteins of *E. chaffeensis* and *E*. *canis.* For example, an RNA molecule encoding the outer membrane protein OMP-1 is used in a cell-free translation systems to prepare OMP-1. Alternatively, a DNA molecule encoding the outer membrane protein is introduced into an expression vector and used to transform cells. Suitable expression vectors include for example chromosomal, nonchromosomal and synthetic DNA sequences, e.g., derivatives of SV40, bacterial plasmids, phage DNAs: yeast plasmids, vectors derived from combinations of plasmids and phage DNAs, viral DNA such as vaccinia, adenovirus, fowl pox virus, and pseudorabies. The DNA sequence is introduced into the expression vector by conventional procedures.

Accordingly, the present invention also relates to recombinant constructs comprising one or more of the polynucleotide sequences. Suitable constructs include, for example, vectors, such as a plasmid, phagemid, or viral vector, into which a sequence that encodes the outer membrane protein has been inserted. In the expression vector, the DNA sequence which encodes the outer membrane protein is operatively linked to an expression control sequence, i.e., a promoter, which directs mRNA synthesis. Representative examples of such promoters, include the LTR or SV40 promoter, the *E .coli* lac or trp, the phage lambda PL promoter and other promoters known to control expression of genes in prokaryotic or eukaryotic cells or in viruses. The promoter may also be the natural promoter of the outer membrane protein coding sequence. The expression vector also contains a ribosome binding site for translation initiation and a transcription terminator. Preferably, the recombinant expression vectors also include an origin of replication and a selectable marker, such as for example, the ampicillin resistance gene of *E. coli* to permit selection of transformed cells, i.e. cells that are expressing the heterologous DNA sequences. The polynucleotide sequence encoding the outer membrane protein is incorporated into the vector in frame with translation initiation and termination sequences. Optionally, the sequence encodes a fusion outer membrane protein which includes an N-terminal or C-terminal peptide or tag that stabilizes or simplifies purification of the expressed recombinant product. Representative examples of such tags include sequences which encode a series of histidine residues, the Herpes simplex glycoprotein D, or glutathione S-transferase.

Polynucleotides which encode portions of the outer membrane proteins of *E. chaffeensis* and *E*. *canis* are useful as probes for isolating and identifying *E. chaffeensis* genes and *E canis* genes, particularly full-length genes from new strains or isolates of *E. chaffeensis* and *E. canis.*

### The Outer Membrane Proteins of E. chaffeensis and E canis

In addition to the outer membrane proteins mature OMP-1 (P28), OMP-1B, OMP-1C, OMP-1D, OMP-1 E, and OMP-1F, from *E. chaffeensis*, the present inventions embraces non-naturally occurring allelelic forms or derivatives of the outer membrane proteins, where one or more of the amino acids have been replaced by conservative amino acid residues, typically by using direct synthesis or recombinant techniques.

### Preparing the Outer Membrane Proteins

The outer membrane proteins of the present invention are synthetically produced by conventional peptide synthesizers. The outer membrane proteins are also produced using cell-free translation systems and RNA molecules derived from DNA constructs that encode the outer membrane protein. Alternatively, the outer membrane protein is made by transfecting host cells with expression vectors that comprise a DNA sequence which encodes the outer membrane protein and then inducing expression of the outer membrane protein in the host cells.

The outer membrane protein is expressed in suitable host cells, preferably bacteria, under the control of suitable promoters. Host cells are transformed with the expression vectors of this invention and cultured in conventional nutrient media. Such media optionally contains additional compounds, such as for example compounds that induce promoters, such as for example isopropyl-β-D-thiogalactoside which induces the Lac promoter, or compounds, such as for example, ampicillin, which allows for selection of transformants.

Following transformation of the suitable host strain and growth of the host strain to an appropriate cell density, the cells are typically harvested by centrifugation, disrupted by physical or chemical means, and the resulting crude extract retained for further purification of the outer membrane protein. Such purification usually involves salting-out of the protein fraction, and one or more chromatography steps, including aqueous ion exchange chromatography, size exclusion chromatography steps, and high performance liquid chromatography (HPLC).

### Preparation of Antibodies

The isolated outer membrane proteins, particularly the recombinant forms of the outer membrane proteins, are used as immunogens to produce antibodies immunospecific for the corresponding protein. The term "immunospecific" means the antibodies have substantially greater affinity for the protein used as an immunogen than for other proteins. Such antibodies are generated using conventional techniques by administering the respective outer membrane protein or a portion thereof, i.e., the recombinant polypeptide, to an animal, preferably a nonhuman, collecting blood from the immunized animals and isolating the serum and/or the IgG fraction from the blood. Monoclonal antibodies are prepared by injecting animals with the immunogens, extracting antibody-producing B cells from the animal, fusing the B cells with a myeloma cells to produce hybridomas, obtaining the monoclonal antibodies from the hybridomas.

Antibodies to the outer membrane proteins of *E. chaffeensis* are useful research tools for identifying cells, particularly monocytes, infected with *E. chaffeensis* and for purifying the corresponding outer membrane protein of *E. chaffeensis* from partially purified preparations by affinity chromatography. Such antibodies are also useful for identifying bacterial colonies, particularly colonies of genetically-engineered bacteria, that are expressing the major outer membrane protein.

### Diagnostic Method

The present invention also provides a method for detecting antibodies to the *E*. *chaffeensis* in a sample of a bodily fluid from a patient. The method comprises providing an isolated outer membrane protein of *E. chaffeensis*, particularly a recombinant form of the isolated protein, contacting the outer membrane protein or polypeptide with a sample taken from the patient; and assaying for the formation of a complex between the outer membrane protein or polypeptide and antibodies in the sample. For ease of detection, it is preferred that the isolated protein or polypeptide be attached to a substrate such as a column, plastic dish, matrix, or membrane, preferably nitrocellulose. The sample may be a tissue or a biological fluid, including urine, whole blood, or exudate, preferably serum. The sample may be untreated, subjected to precipitation, fractionation, separation, or purification before combining with the isolated protein or peptide. Interactions between antibodies in the sample and the isolated protein or peptide are detected by radiometric, colorimetric, or fluorometric means, size-separation, or precipitation. Preferably, detection of the antibody-outer membrane protein complex is by addition of a secondary antibody that is coupled to a detectable tag, such as for example, an enzyme, fluorophore, or chromophare. Formation of the complex is indicative of the presence of anti-E chaffeensis or anti-E canis antibodies, either IgM or IgG, in the patient. Thus, the method is used to determine whether a patient is infected with *E. chaffeensis*.

Preferably, the method employs an enzyme-linked immunosorbent assay (ELISA) or a Western immunoblot procedure . Such methods are relatively simple to perform and do not require special equipment as long as membrane strips are coated with a high quality antigen. Accordingly, it is more advantageous to use a recombinant form of the outer membrane protein of *E chaffeensis* since such proteins, typically, are more pure and consistent in quality than a purified form of such protein.

### Immunogenic Composition

The present invention also relates to immunogenic compositions comprising one or more of the isolated outer membrane proteins of *E. chaffeensis* and a pharmaceutically acceptable adjuvant which, preferably, enhances the immunogenic activity of the outer membrane protein in the host animal.

### Preparation of a Polynucleotide which Encodes OMP-1(P28)

### A. Isolation of the Outer Membrane Proteins

*E. chaffeensis* Arkansas strain and *E. canis* Oklahoma strain were cultivated in the DH82 dog macrophage cell line and purified by Percoll density gradient centrifugation. Purified ehrlichiae (100 µg) were suspended with 10 mM sodium phosphate buffer, pH 7.4, containing 0.1% Sodium *N*-lauroyl sarcosine (Sarkosyl) [Sigma, St. Louis, MO], 50 µg/ml each Dnase I (Sigma) and Rnase A (Sigma), and 2.5 mM MgCl₂, After incubation at 37° for 30 min, the sample was separated by centrifugation at 10,000 x g for 1 h into the soluble supernatant and the insoluble precipitate. The insoluble pellet was resuspended 2 to 3 times with 0.1% Sarkosyl and centrifuged. The final pellet was analyzed by sodium dodecyl sulfate (SDS)-polyacrylamide gel electrophoresis (PAGE) and by electron microscopy.

Transmission electron microscopy revealed that the purified ehrlichial fraction consists of a mixture of electron dense and light forms of *E. chaffeensis* with slight disintegration of inner membrane . Ehrlichiae were not surrounded with the host inclusion membrane. Various sizes of membrane vesicles (< 1 µm) without significant ribosomes or nuclear materials were observed in the Sarkosyl-insoluble fraction from the organism. Succinic dehydrogenase (inner membrane marker enzyme of gram negative bacteria) activities were at less than the detection limit (1 n moles / min / mg of protein) in the Sarkosyl-insoluble fraction compared to approximately 10 n moles / min / mg of protein in the Percoll-puriried organisms, suggesting that the insoluble fraction primarily consisted of the outer membrane of *E. chaffeensis*.

Analysis of the Sarkosyl-soluble, and insoluble fraction of *E. chaffeensis* by SDS-PAGE suggested that proteins of 30-kDa range in the insoluble fraction represent the major outer membrane proteins of this organism. Analysis of the Sarkosyl-soluble, and insoluble fraction of *E. cams* by SDS-PAGE suggested that proteins of 30-kDa range in the insoluble fraction represent the major outer membrane proteins of this organism also. *E*. *canis* was antigenically cross reactive with *E chaffeensis.* These findings indicate that the 30-kDa range proteins represent the major outer membrane proteins of these two *Ehrlichia* spp.

To improve resolution of the outer membrane proteins, proteins in the Sarkosyl-insoluble pellet prepared from 400 µg of purified *E. chaffeensis* were separated by a reversed-discontinuous (Rd) SDS-PAGE (2.5-cm-long 17% gel on top of 11-cm-long 12% gel). At least five proteins of 30-kDa range in *E*. *chaffeensis* (P23, P25, P27, P28, and P29) were resolved from the Sarkosyl-insoluble proteins.

### B. Cloning and sequencing of the p28 gene

The portion of the membrane containing bound proteins was excised and analyzed with an Applied Biosystems protein sequencer (Model 470). The N-terminal amino acid sequence of P28 was determined as D P A G S G I N G N F Y S G K Y M P, SEQ ID NO 57. Based on 6th to 12th amino acids of this sequence, a forward primer. FECH1, having the sequence: 5'-CGGGATCCGAATTCGG(A/T/G/C)AT(A/T/C)AA(T/C)GG(A/T/GIC)AA(T/C)TT(T/C)TA-3'. SEQ ID NO 49 was designed. Amino acids at the I to 5 positions of the N terminus of P28 were not included in this primer design. For insertion into an expression vector, a 14-bp sequence (underlined) was added at the 5' end of primer to create an *Eco*RI and a *Bam*HI site. The reverse primer, RECH2, which includes a *Not*I site at the 5' end for ligation into an expression vector had the sequence : 5'-AGCGGCCGCTTA(A/G)AA(T/C)A(C/G) (A/G)AA (C/T)CT T(C/G)C TCC-3'. SEQ ID NO 50.

Genomic DNA of *E. chaffeensis* was isolated from purified organisms. PCR amplification with FECH 1 and RECH2 primers was performed using a Perkin-Elmer Cetus DNA Thermal Cycler (model 480). A 0.8-kb amplified product was cloned in the pCRII vector of a TA closing kit. as described by the manufacturer (Invitrogen Co., San Diego, CA). The clone obtained was designated pCRII*p28*. Both strands of the inserted DNA were sequenced by a dideoxy-termination method with an Applied Biosystems 373A DNA sequencer.

The 0.8-kb DNA fragment, cloned in pCRII*p28*, had an open reading frame (ORF) of 756 bp encoding a 251-amino acid recombinant protein (including both PCR primer regions) with a molecular mass of 27,685 Da. The nucleotide sequence of the open reading frame, and the amino acid sequence of the polypeptide of the OMP-1 protein, are shown in Figs 3A and 3B, respectively.

A DNA fragment comprising the *p30* gene was prepared in a similar manner, i.e., by PCR amplification of genomic DNA of *E*. *canis* using primers FECH1 and RECHI.

### Preparation of Polynucleotides which encode OMP 1A, OMP1B, OMP1-C, OMP-1D, OMP-1F, and OMP1-E

A. Southern blot analysis. Genomic DNA extracted from the purified *E. chaffeensis* (200 ng each) was digested with restriction endonucleases, electrophoresed, and transferred to Hybond-N⁺ nylon membrane (Amersham, Arlington Heights, IL), by a standard method. The 0.8-kb *p28* gene fragment from the clone pCRII*p28* was labeled with [α-³²P]dATP by the random primer method using a kit (Boehringer Mannheim, Indianapolis, IN) and the labeled fragment was used as a DNA probe. Hybridization was performed at 60°C in rapid hybridization buffer (Amersham) for 20 h. The nylon sheet was washed in 0.1 x SSC (1 x x SSC containing 0.15M sodium chloride and 0.015M sodium citrate) with 1% SDS at 55°C and the hybridized probes were exposed to Hyperfilm (Amersham) at -80°C.

Genomic Southern blot analysis with several restriction enzymes resulted in one or more DNA fragment(s) of *E. chaffeensis* which hybridized to ³²P-labeled *p28* gene probe. The restriction enzymes used did not cut within the *p28* gene portion of the pCRII*p28* insert. *Xba* 1, *BgI* II, and *Kpn* I produced two bands, Spe I generated three bands, and *Eco*R V and *Pst* I produced multiple bands with different densities. *Eco*R I generated a broad band of 2.5 to 4kb. These *p28* homologous genes are designated as *omp-I* (outer membrane protein-1) family.

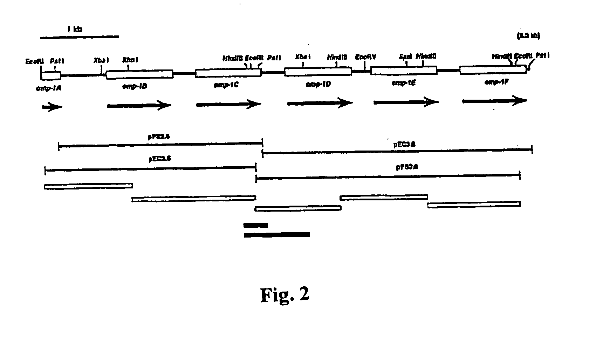

B. Cloning and sequencing of genomic copies of *E. chaffeensis p28* gene. The *EcoR* I and *Pst* I fragments of DNA, detected by genomic Southern blot analysis as described above, were inserted into pBluescript II KS (+) vectors, and the recombinant plasmids were introduced into *E. coli* DH5α. Using the colony hybridization method with the ³²P-labeled *p28* gene probe, four positive clones were isolated from the transformant. The positive clones were designated pEC2.6, pEC3.6, pPS2.6, and pPS3.6. These contained the ehrlichial DNA fragments of 2.6-kb (*Eco*R I), 3.6 kb (*Eco*R 1), 2.6 kb (*Pst* I), and 3.6 kb (*Pst* 1), respectively. The inserts of the clones pEC3.6 and pPS2.6 overlapped as shown in Fig. 2. The overlapping area was further confirmed by PCR of *E. chaffeensis* genomic DNA with two pairs of primer sets interposing the junctions of the four clones. The 1.1- to 1.6-kb DNA fragments of *Hin*dIII-*Hin*dIII, *Hin*dIII-*Eco*RI, or *Xho*I-*Eco*RI in the pEC2.6 and pEC3.6 were subcloned for sequencing. DNA sequencing was performed with suitable synthetic primers by dideoxy-termination method as described above.

Four DNA fragments from 2.6 to 3.6 kb were cloned from the *Eco*RI-digested and the *Pst*I-digested genomic DNA of *E. chaffeensis* by colony hybridization with radiolabeled *p28* gene probe. The inserted DNA of the two recombinant clones, pEC3.6 and PPS2.6, were overlapped. Sequencing revealed one 5'-truncated ORF of 243 bp (designated *omp-1A*) and five complete ORF of 836-861 bp (designated *omp-1B* to *omp-IF*), which are tandemly-arrayed and are homologous to the *p28* gene (but are not identical), in the ehrlichial genomic DNA of 6,292 bp. The intergenic spaces were 581 bp between *omp-IA* and *omp-IB* and 260-308 bp among others. Putative promoter regions and ribosome-binding sites were identified in the noncoding regions.

### Sequence analysis and GenBank accession number.

Nucleotide sequences were analyzed with the DNASIS program (Hitachi Software Engineering Co., Ltd., Yokohama, Japan). A homology search was carried out with databases of the GenBank, Swiss Plot, PDB and PIR by using the software basic local alignment search tool in the BLAST network service (the National Center for Biotechnology Information, Bethesda, MD). Phylogenetic analysis was performed by using the PHYLIP software package (version 3.5). An evolutional distance matrix, generated by using the Kimura formula in the PROTDIST, was used for construction of a phylogenetic tree by using the unweighted pair-group method analysis (UPGMA) (Felsenstein, J. 1989. PHYLIP-phylogeny inference package (version 3.3). Cladistics 5:164-166). The data were also examined using parsimony analysis (PROTPARS in PHYLIP). A bootstrap analysis was carried out to investigate the stability of randomly generated trees by using SEQBOOT and CONSENSE in the same package. The nucleotide sequence of the *p28* gene and its gene copies has been assigned GenBank accession numbers U72291 and AF021338, respectively.

### Proteins of the E. chaffeensis omp-I Family.

Five complete *omp*-*I* gene copies (*omp-1B* to *omp*-*I*F) encode 279 to 287-amino acid proteins with molecular masses of 30.320 - 31.508 Da. *Omp-1A* encodes an 82-amino acid partial protein (9.243 Da) which lacks the N-terminal region. The 25-amino acid sequence at the N-terminus of OMP-1B to OMP-1F (encoded in *omp-1B* to *omp-1F*) is predicted to be a signal peptide because three carboxyl-terminal amino acids of the signal peptides (Ser-X-Ala in OMP-1B, Leu-X-Ser for OMP-C, and Ser-X-Ser for OMP-1D and OMP-1F) are included in the preferred amino acid sequence of signal peptidase at the processing sites proposed by Oliver. The calculated molecular masses of the mature OMP-1B to OMP-1F from the predicted amino acid sequences are 28,181 Da for OMP-1B, 27,581 Da for OMP-1C, 28.747 Da for OMP-1D, 27,776 Da for OMP-1E, and 27.933 Da for OMP-1F. The estimated isoelectric points are 4.76-5.76 in the mature OMP-1B to OMP-1F. An amino acid sequence in *omp-1F* gene (the 80th to 94th amino acids) was identical to the N-terminal amino acid sequences of *E. chaffeensis* native P23 protein as determined chemically, which indicates that P23 is derived from the *omp-1F* gene. Amino acid sequences identical to the N-terminal sequences of P25, P27, and P29 were not found in those from *omp*-1 gene copies cloned in this study.

Alignment of predicted amino acid sequences of the *E. chaffeensis* OMP-1 family and *Cowdria rummantium,* revealed substitutions or deletions of one or several contiguous amino acid residues throughout the molecules. The significant differences in sequences among the aligned proteins are seen in the regions indicated SV (semivariable region) and HV (hypervariable region) 1 to 3 in Fig 31. Computer analysis for hydropathy revealed that protein molecules predicted from all *omp-1* gene copies contain alternative hydrophilic and hydrophobic motifs which are characteristic of transmembrane proteins. The HV I and HV2 were found to locate in the hydrophilic regions.

The amino acid sequences of 5 mature proteins without signal peptides (OMP-1C to OMP-1F and P28) were similar to one another (71-83%) but the sequence of OMP-1B was dissimilar to those of the 5 proteins (45-48%). The amino acid sequences of the 5 proteins showed an intermediate degree of similarity with that of *C. ruminantium* MAP-1 (59-63%), but the similarity between that of the OMP-1B and the *C. ruminantium* MAP-1 was low (45%). These relations are shown in a phylogenetic tree which was obtained based on the amino acid sequence alignment by UPGMA method in the PHYLIP software package. Three proteins (P28, OMP-1D, and OMP-1F) and two proteins (OMP-1C and OMP-1E) formed two separate clusters. The OMP-1B was located distantly from these two clusters. The *C. ruminantium* MAP-1 was positioned between the OMP-1B and other members in the OMP-1 family.

### Preparation of a Recombinant form of OMP-1 and P30

The 0.8-kb p28 gene was excised from the clone pCRII*p28* by *Eco*RI-*Not*I double-digestion, ligated into *Eco*RI-*Not*I sites of a pET 29a expression vector, and amplified in *Escherichia coli* BL21 (DE3)pLysS (Novagen, Inc., Madison, WI). The clone (designated pET29*p28*) produced a fusion protein with a 35-amino acid sequence carried from the vector at the N terminus. The amino acid sequence of the OMP-1 portion of the fusion protein is depicted in Fig. 1, amino acid 6 through 256.

An expression vector comprising the *p30* gene was used to prepare the recombinant form of P30.

### Preparation of anti-rP28 (anti-OMP1) antibody

The (r) P28 antigen was prepared by excising the gel band corresponding to the rP28 in SDS-PAGE. mincing the band in phosphate-buffered saline (PBS), pH 7.4, and mixing with an equal volume of Freund's incomplete adjuvant (Sigma). The rP28 mixture (1 mg of protein each time) was subcutaneously injected into a rabbit every 2 weeks four times. A serum sample was collected from the rabbit to provide the anti-rP28 antibody

The anti-rP28 antibody was examined by western immunoblots analysis. The results indicated that the rabbit anti-rP28 antibody recognized not only rP28 (31 kDa) and P28, but also P29 and P25 of *E. chaffeensis* and P30 of *E. canis* These results indicate that P28 shares antigenic epitopes with P25 and P29 in *E*. *chaffeensis* and P30 of *E. cants*.

The following examples are for purposes of illustration only and are not intended to limit the scope of the claims which are appended hereto.

### Example 1. Assaying for the presence of anti-OMP-1 antibody in a Patient

Convalescent-phase serum from a patient with clinical signs of human ehrlichiosis was used. Western blot analyses using the rP28 protein as antigen was performed with 1:1,000 dilutions of this serum. Alkaline phosphatase-conjugated affinity-purified anti-human immunoglobulin G (Kirkegaard & Perry Laboratories, Inc., Gaithersburg, MD) was used at a 1:1,000 or 1:2,000 dilution as secondary antibodies. Results indicated that serum from a patient with clinical signs of human ehrlichiosis reacted strongly to rP28 (31 kDa).

### Example 2 Assaying for the presence of anti-OMP-1 antibody in a Patient

Convalescent-phase serum from a patient with clinical signs of human ehrlichiosis was reacted with the rP30 protein of *E. canis* as described in Example 1. The serum reacted strongly to rP30. These results indicate the rP30 is useful for diagnosing an infection with *E. chaffeensis* in human patients.

### Example 3. Identifying E. chaffeensis-infected cells using anti-rP 28 antibody

*E. chaffeensis*-infected DH82 cells were sonicated and centrifuged at 400 x g for 10 min. The supernatant was then centrifuged at 10,000 x g for 10 min to obtain ehrlichia-enriched pellet. The pellet was resuspended and incubated with rabbit anti-rP28 antibody or normal rabbit serum (1:100 dilution) at 37°C for 1h in PBS containing 1% bovine serum albumin (BSA-PBS). After washing, the ehrlichiae was incubated with gold-conjugated protein G (20 nm), Sigma) at 1:30 dilution for I h at room temperature in BSA-PBS. After washing again, the specimen was fixed with 1.25% formaldehyde, 2.5% glutaraldehyde, and 0.03% trinitrophenol in 0.1 M cacodylate buffer (pH 7.4) for 24h and postfixed in 1% osmium-1.5% potassium ferricyanide for 1 h (34). The section was then embedded in PolyBed 812 (Polysciences, Warraington, Pa). The specimen was ultrathin sectioned at 60 nm, stained with uranyl acetate and lead citrate, and observed with a Philips 300 transmission electron microscope at 60 kV.

Transmission immunoelectron microscopy with colloidal gold-conjugated protein G and rabbit anti-rP28 antibody revealed gold particles bound to *E. chaffeensis* surface. The distribution of the particles was random, close to the surface, and appeared as if almost embedded in the membrane, suggesting that the antigenic epitope protrudes very little from the lipid bilayer. Nonetheless, the antigenic epitope was surface-exposed, and thus, could be recognized by rabbit anti-rP28 antibody. No gold particles were observed on host cytoplasmic membrane or *E. chaffeensis* incubated with normal rabbit serum.

### Example 4. Immunization of mice and E. chaffeensis challenge.

The rP28 band in SDS- PAGE was excised, minced, and mixed with an equal volume of Freund's incomplete or complete adjuvant. Nine BALB.c male mice (6 weeks old) were divided into two groups. Five mice were intraperitoneally immunized a total of four times at 10-day intervals: twice with a mixture of the minced gel with the rP28 (30 to 40 µg of protein per mouse each time) and incomplete adjuvant, and twice with a mixture of the recombinant protein (the same amount as before) and complete adjuvant. Four mice were intraperitoneally injected with a mixture of the minced gel without protein and the respective adjuvants. For ehrlichia-challenge, approximately 1 x 10⁷ DH82 cells heavily-infected with *E*. *chaffeensis* were disrupted by sonication in serum-free DMEM (GIBCO-BRL) and centrifuged at 200 x g for 5 min. The supernatant was diluted to a final volume of 5 ml, and 0.3 ml was inoculated intraperitoneally into each mouse 10 days after the last immunization. Before challenge, all 5-immunized mice had a titer of 1:160 against *E. chaffeensis* antigen by IFA and all 4-nonimmunized mice were negative.

At day 5 post-challenge, approximately 1 ml of blood was collected in an EDTA tube from each mouse and protection was assessed by PCR detection of *E. chaffeensis* 16S rDNA in the buffy coat of the collected blood. *E. chaffeensis* could not be reisolated in cell culture at day 10 postinfection. Day 5 post challenge is the optimum time at which establishment of ehrlichial infection can be examined by PCR without the influence of residual DNA from the ehrlichiae used as the challenge before the spontaneous clearance of organisms take place. The *E. chaffeensis*-specific DNA fragment was observed in all nonimmunized mice but not in any immunized mice, indicating that immunization of rP28 apparently protects mice from ehrlichial infection and indicating that the P28 is a potential protective antigen.

### Reference Example 5 Assaying for the presence of anti-P30 antibody in Dogs

The rP30 protein was used as an antigen in a Western immunoblot analysis and dot blot analysis to detect the presence of antibody to *E. canis* in serum from E-canis infected dogs. The results of the Western immunoblot analysis indicated that reactivity of the sera with rP30 was stronger than the reactivity that was observed when purified *E*. *canis* was used as antigen. The results of the dot blot assay indicated that rP30 is a useful and sensitive tool for serodiagnosis of canine ehrlichiosis.

## Claims

1. A protein selected from the group:
(a) an isolated outer membrane protein of *E*. *chaffeensis* or an immunogenic fragment thereof,
wherein the outer membrane protein comprises an amino acid sequence which is at least 85% homologous to a sequence selected from the group consisting of
the amino acid sequence in Fig. I corresponding to P28,
the amino acid sequence in Fig. 4B corresponding to OMP-1B,
the amino acid sequence in Fig. 5B corresponding to OMP-1C,
the amino acid sequence in Fig. 6B corresponding to OMP-1D,
the amino acid sequence in Fig. 7B corresponding to OMP-1E,
the amino acid sequence in Fig. 8B corresponding to OMP-1F,
wherein each outer membrane protein or immunogenic fragment thereof is useful for producing an antibody that is immunospecific for the corresponding outer membrane protein;
(b) an isolated rP28 immunogenic fragment of P28 protein, said fragment comprising a sequence identical to the amino acid sequence extending from amino acid 6 through amino acid 256 in Fig. I, wherein the rP28 immunogenic fragment binds to antibodies in serum from a human infected with *E*. *chaffeensis;* and
(c) an isolated OMP-1A protein fragment comprising the OMP-1A amino acid sequence in Fig. 31.

2. A protein of claim 1(a) wherein said protein is an OMP-1B protein which comprises the amino acid sequence shown in Fig. 4B.

3. A protein of claim 1(a) wherein said protein is an immunogenic fragment of outer membrane protein OMP-1B which outer membrane protein comprises the amino acid sequence shown in Fig. 4B, wherein said immunogenic fragment is useful for producing an antibody that is immunospecific for the OMP-1B.

4. A protein of claim 1(a) wherein said protein is a mature OMP-1B protein and comprises the sequence extending from amino acid 26 through amino acid 283 in Fig. 4B.

5. A protein of claim 1(a) wherein said protein is an OMP-1C protein which comprises the amino acid sequence shown in Fig. 5B.

6. A protein of claim 1(a) wherein said protein is an immunogenic fragment of outer membrane protein OMP-1C which outer membrane protein comprises the amino acid sequence shown in Fig. 5B, wherein said immunogenic fragment is useful for producing an antibody that is immunespecific for the OMP-1C.

7. A protein of claim 1(a) wherein said protein is a mature OMP-1C protein and comprises the sequence extending from amino acid 26 through amino acid 280 in Fig. 5B.

8. A protein of claim 1(a) wherein said protein is an OMP-1D protein which comprises the amino acid sequence shown in Fig. 6B.

9. A protein of claim 1(a) wherein said protein is an immunogenic fragment of outer membrane protein OMP-1D which outer membrane protein comprises the amino acid sequence shown in Fig. 6B, wherein said immunogenic fragment is useful for producing an antibody that is immunospecific for the OMP-1D.

10. A protein of claim 1(a) wherein said protein is a mature OMP-1D protein and comprises the sequence extending from amino acid 26 through amino acid 286 in Fig. 6B.

11. A protein of claim 1(a) wherein said protein is an OMP-1E protein which comprises the amino acid sequence shown in Fig. 7B.

12. A protein of claim 1(a) wherein said protein is an immunogenic fragment of outer membrane protein OMP-1E which outer membrane protein comprises the amino acid sequence shown in Fig. 7B, wherein said immunogenic fragment is useful for producing an antibody that is immunospecific for the OMP-1E.

13. A protein of claim 1(a) wherein said protein is a mature OMP-1E protein and comprises the sequence extending from amino acid 26 through amino acid 278 in Fig. 7B.

14. A protein of claim 1(a) wherein said protein is an OMP-1F protein which comprises the amino acid sequence shown in Fig. 8B.

15. A protein of claim 1(a) wherein said protein is an immunogenic fragment of outer membrane protein OMP-1F which outer membrane protein comprises the amino acid sequence shown in Fig. 8B, wherein said immunogenic fragment is useful for producing an antibody that is immunospecific for the OMP-1F.

16. A protein of claim 1(a) wherein said protein is a mature OMP-1F protein and comprises the sequence extending from amino acid 26 through amino acid 280 in Fig. 8B.

17. A protein of claim 1(a) wherein said protein is a P28 protein which comprises the amino acid sequence shown in Fig. 1.

18. A protein of claim 1(a) wherein said protein is an immunogenic fragment of outer membrane protein P28 which outer membrane protein comprises the amino acid sequence shown in Fig. 1, wherein said immunogenic fragment is useful for producing an antibody that is immunospecific for the P28.

19. A protein of claim 1(b) wherein said protein is an rP28 immunogenic fragment of P28 protein, which fragment comprises the amino acid sequence extending from amino acid 6 through amino acid 256 in Fig. 1, wherein the rP28 immunogenic fragment binds to antibodies in serum from a human infected with *E. chaffeensis.*

20. A fusion protein comprising the immunogenic fragment of claim 1(b) and an N-terminal or C-terminal peptide or tag.

21. An isolated polynucleotide selected from the group:
(a) an isolated polynucleotide encoding an outer membrane protein of *E. chaffeensis* or an immunogenic fragment thereof of claim 1(a);
(b) an isolated polynucleotide encoding an rP28 immunogenic fragment of claim 1(b); and
(c) an isolated polynucleotide encoding an OMP-1A protein fragment of claim 1(c).

22. An isolated polynucleotide of claim 21(a) wherein the polynucleotide encodes an OMP-1B protein, immunogenic fragment thereof, or mature OMP-1B of anyone of claims 2, 3, or 4.

23. An isolated polynucleotide of claim 21(a) wherein the polynucleotide encodes an OMP- 1C protein, immunogenic fragment thereof, or mature OMP-1C of anyone of claims 5, 6 or 7.

24. An isolated polynucleotide of claim 21(a) wherein the polynucleotide encodes an OMP-1D protein, immunogenic fragment thereof, or mature OMP-1D of anyone of claims 8, 9 or 10.

25. An isolated polynucleotide of claim 21(a) wherein the polynucleotide encodes an OMP-1E protein, immunogenic fragment thereof, or mature OMP-1E of any one of claims 11, 12 or 13.

26. An isolated polynucleotide of claim 21(a) wherein the polynucleotide encodes an OMP-1F protein, immunogenic fragment thereof, or mature OMP-1F of any one of claims 14, 15, or 16.

27. An isolated polynucleotide of claim 21(a) or claim 21(b) wherein the polynucleotide encodes a P28 protein, an immunogenic fragment thereof or an rP28 immunogenic fragment of anyone of claims 17, 18 or 19.

28. A method for detecting antibodies to *E*. *chaffeensis* in a sample comprising the steps of:
(a) providing an outer membrane protein of claim 1(a) or an rP28 immunogenic fragment of claim 1(b);
(b) contacting the sample with the outer membrane protein or the rP28 immunogenic fragment; and
(c) assaying for the formation of a complex between antibodies in the sample and the outer membrane protein or the rP28 immunogenic fragment, wherein formation of said complex is indicative of the presence of *E*. *chaffeensis* antibodies.

29. An antibody immunospecific for at least one outer membrane protein of *E*. *chaffeensis,* wherein said antibody is produced by an immunogen selected from one or more of the following: an OMP-1B protein of claim 2; an OMP-1C protein of claim 5; an OMP-1D protein of claim 8; an OMP-1E protein of claim 11; an OMP-1F protein of claim 14 or a P28 protein of claim 17.

30. The antibody of claim 29 , wherein said antibody is monoclonal.

31. An immunogenic composition comprising a protein selected from:
(a) at least one outer membrane protein of *E*. *chaffeensis* of claim 1(a);
(b) an rP28 immunogenic fragment of claim 1(b);
and further comprising a pharmaceutically acceptable adjuvant, wherein said composition is capable of protecting a mammal against infection with *E*. *chaffeensis.*

32. A vaccine comprising an immunogenic composition according to claim 31.

## Patentansprüche

1. Protein, ausgewählt aus der folgenden Gruppe:
(a) einem isolierten Außenmembranprotein von E. chaffeensis oder einem immunogenen Fragment davon, worin das Außenmembranprotein eine Aminosäuresequenz umfasst, die zu einer aus der aus
der Aminosäuresequenz in Fig. 1, die P28 entspricht,
der Aminosäuresequenz in Fig. 4B, die OMP-1B entspricht,
der Aminosäuresequenz in Fig. 5B, die OMP-1C entspricht,
der Aminosäuresequenz in Fig. 6B, die OMP-1D entspricht,
der Aminosäuresequenz in Fig. 7B, die OMP-1E entspricht,
der Aminosäuresequenz in Fig. 8B, die OMP-1F entspricht,
bestehenden Gruppe ausgewählten Sequenz zu mindestens 85 % homolog ist, worin jedes Außenmembranprotein oder immunogene Fragment davon zur Herstellung eines Antikörpers zweckdienlich ist, der für das entsprechende Außenmembranprotein immunspezifisch ist;
(b) einem isolierten immunogenen rP28-Fragment des Proteins P28, wobei das Fragment eine Sequenz umfasst, die mit der Aminosäuresequenz, die sich in Fig. 1 von Aminosäure 6 bis Aminosäure 256 erstreckt, identisch ist, worin das immunogene rP28-Fragment an Antikörper in Serum von einem mit E. chaffeensis infizierten Menschen bindet; und
(c) einem isolierten OMP-1A-Proteinfragment, das die OMP-1A-Aminosäuresequenz in Fig. 31 umfasst.

2. Protein nach Anspruch 1(a), worin das Protein ein OMP-1B-Protein ist, das die in Fig. 4B gezeigte Aminosäuresequenz umfasst.

3. Protein nach Anspruch 1(a), worin das Protein ein immunogenes Fragment des Außenmembranproteins OMP-1B ist, wobei dieses Außenmembranprotein die in Fig. 4B gezeigte Aminosäuresequenz umfasst, worin das immunogene Fragment zur Herstellung eines Antikörpers zweckdienlich ist, der für das OMP-1B immunspezifisch ist.

4. Protein nach Anspruch 1(a), worin das Protein ein reifes OMP-1B-Protein ist und die Sequenz umfasst, die sich in Fig. 4B von Aminosäure 26 bis Aminosäure 283 erstreckt.

5. Protein nach Anspruch 1(a), worin das Protein ein OMP-1C-Protein ist, das die in Fig. 5B gezeigte Aminosäuresequenz umfasst.

6. Protein nach Anspruch 1(a), worin das Protein ein immunogenes Fragment des Außenmembranproteins OMP-1C ist, wobei dieses Außenmembranprotein die in Fig. 5B gezeigte Aminosäuresequenz umfasst, worin das immunogene Fragment zur Herstellung eines Antikörpers zweckdienlich ist, der für das OMP-1C immunspezifisch ist.

7. Protein nach Anspruch 1(a), worin das Protein ein reifes OMP-1C-Protein ist und die Sequenz umfasst, die sich in Fig. 5B von Aminosäure 26 bis Aminosäure 280 erstreckt.

8. Protein nach Anspruch 1(a), worin das Protein ein OMP-1D-Protein ist, das die in Fig. 6B gezeigte Aminosäuresequenz umfasst.

9. Protein nach Anspruch 1(a), worin das Protein ein immunogenes Fragment des Außenmembranproteins OMP-1D ist, wobei dieses Außenmembranprotein die in Fig. 6B gezeigte Aminosäuresequenz umfasst, worin das immunogene Fragment zur Herstellung eines Antikörpers zweckdienlich ist, der für das OMP-1D immunspezifisch ist.

10. Protein nach Anspruch 1(a), worin das Protein ein reifes OMP-1D-Protein ist und die Sequenz umfasst, die sich in Fig. 6B von Aminosäure 26 bis Aminosäure 286 erstreckt.

11. Protein nach Anspruch 1 (a), worin das Protein ein OMP-1 E-Protein ist, das die in Fig. 7B gezeigte Aminosäuresequenz umfasst.

12. Protein nach Anspruch 1(a), worin das Protein ein immunogenes Fragment des Außenmembranproteins OMP-1 E ist, wobei dieses Außenmembranprotein die in Fig. 7B gezeigte Aminosäuresequenz umfasst, worin das immunogene Fragment zur Herstellung eines Antikörpers zweckdienlich ist, der für das OMP-1 E immunspezifisch ist.

13. Protein nach Anspruch 1(a), worin das Protein ein reifes OMP-1 E-Protein ist und die Sequenz umfasst, die sich in Fig. 7B von Aminosäure 26 bis Aminosäure 278 erstreckt.

14. Protein nach Anspruch 1(a), worin das Protein ein OMP-1 F-Protein ist, das die in Fig. 8B gezeigte Aminosäuresequenz umfasst.

15. Protein nach Anspruch 1(a), worin das Protein ein immunogenes Fragment des Außenmembranproteins OMP-1 F ist, wobei dieses Außenmembranprotein die in Fig. 8B gezeigte Aminosäuresequenz umfasst, worin das immunogene Fragment zur Herstellung eines Antikörpers zweckdienlich ist, der für das OMP-1 F immunspezifisch ist.

16. Protein nach Anspruch 1(a), worin das Protein ein reifes OMP-1 F-Protein ist und die Sequenz umfasst, die sich in Fig. 8B von Aminosäure 26 bis Aminosäure 280 erstreckt.

17. Protein nach Anspruch 1(a), worin das Protein ein P28-Protein ist, das die in Fig. 1 gezeigte Aminosäuresequenz umfasst.

18. Protein nach Anspruch 1(a), worin das Protein ein immunogenes Fragment des Außenmembranproteins P28 ist, wobei dieses Außenmembranprotein die in Fig. 1 gezeigte Aminosäuresequenz umfasst, worin das immunogene Fragment zur Herstellung eines Antikörpers zweckdienlich ist, der für das P28 immunspezifisch ist.

19. Protein nach Anspruch 1 (b), worin das Protein ein immunogenes rP28-Fragment des Proteins P28 ist, wobei dieses Fragment die Aminosäuresequenz umfasst, die sich in Fig. 1 von Aminosäure 6 bis Aminosäure 256 erstreckt, worin das immunogene rP28-Fragment an Antikörper in Serum von einem mit E. chaffeensis infizierten Menschen bindet.

20. Fusionsprotein, umfassend das immunogene Fragment nach Anspruch 1(b) und ein Peptid oder eine Markierung am N-Terminus oder am C-Terminus.

21. Isoliertes Polynucleotid, ausgewählt aus der folgenden Gruppe:
(a) einem isolierten Polynucleotid, das für ein Außenmembranprotein von E. chaffeensis oder immunogenes Fragment davon nach Anspruch 1(a) kodiert;
(b) einem isolierten Polynucleotid, das für ein immunogenes rP28-Fragment nach Anspruch 1 (b) kodiert; und
(c) einem isolierten Polynucleotid, das für ein OMP-1A-Proteinfragment nach Anspruch 1 (c) kodiert.

22. Isoliertes Polynucleotid nach Anspruch 21 (a), worin das Polynucleotid für ein OMP-1B-Protein, ein immunogenes Fragment davon oder reifes OMP-1B nach einem der Ansprüche 2, 3 oder 4 kodiert.

23. Isoliertes Polynucleotid nach Anspruch 21 (a), worin das Polynucleotid für ein OMP-1C-Protein, ein immunogenes Fragment davon oder reifes OMP-1C nach einem der Ansprüche 5, 6 oder 7 kodiert.

24. Isoliertes Polynucleotid nach Anspruch 21(a), worin das Polynucleotid für ein OMP-1D-Protein, ein immunogenes Fragment davon oder reifes OMP-1D nach einem der Ansprüche 8, 9 oder 10 kodiert.

25. Isoliertes Polynucleotid nach Anspruch 21 (a), worin das Polynucleotid für ein OMP-1 E-Protein, ein immunogenes Fragment davon oder reifes OMP-1 E nach einem der Ansprüche 11, 12 oder 13 kodiert.

26. Isoliertes Polynucleotid nach Anspruch 21 (a), worin das Polynucleotid für ein OMP-1 F-Protein, ein immunogenes Fragment davon oder reifes OMP-1 F nach einem der Ansprüche 14, 15 oder 16 kodiert.

27. Isoliertes Polynucleotid nach Anspruch 21 (a) oder Anspruch 21 (b), worin das Polynucleotid für ein P28-Protein, ein immunogenes Fragment davon oder ein immunogenes rP28-Fragment nach einem der Ansprüche 17, 18 oder 19 kodiert.

28. Verfahren zum Detektieren von Antikörpern zu E. chaffeensis in einer Probe, umfassend die folgenden Schritte:
(a) das Bereitstellen eines Außenmembranproteins nach Anspruch 1(a) oder eines immunogenen rP28-Fragments nach Anspruch 1 (b);
(b) das Kontaktieren der Probe mit dem Außenmembranprotein oder dem immunogenen rP28-Fragment; und
(c) das Testen auf die Bildung eines Komplexes zwischen Antikörpern in der Probe und dem Außenmembranprotein oder dem immunogenen rP28-Fragment, worin die Bildung des Komplexes auf die Gegenwart von E.-chaffeensis-Antikörpern hindeutet.

29. Für mindestens ein Außenmembranprotein von E. chaffeensis immunspezifischer Antikörper, worin der Antikörper von einem Immunogen produziert wird, das aus einem oder mehreren der folgenden ausgewählt ist: einem OMP-1B-Protein nach Anspruch 2; einem OMP-1C-Protein nach Anspruch 5; einem OMP-1D-Protein nach Anspruch 8; einem OMP-1 E-Protein nach Anspruch 11; einem OMP-1 F-Protein nach Anspruch 14 oder einem P28-Protein nach Anspruch 17.

30. Antikörper nach Anspruch 29, worin der Antikörper monoklonal ist.

31. Immunogene Zusammensetzung, umfassend ein aus den folgenden ausgewähltes Protein:
(a) mindestens ein Außenmembranprotein von E. chaffeensis nach Anspruch 1(a);
(b) ein immunogenes rP28-Fragment nach Anspruch 1 (b);
und weiters umfassend ein pharmazeutisch annehmbares Adjuvans, worin die Zusammensetzung in der Lage ist, ein Säugetier gegen eine Infektion mit E. chaffeensis zu schützen.

32. Impfstoff, umfassend eine immunogene Zusammensetzung nach Anspruch 31.

## Revendications

1. Protéine sélectionnée dans le groupe:
(a) une protéine isolée à membrane extérieure de *E. chaffensis* ou un fragment immunogène de celle-ci, où la protéine à membrane extérieure comprend une séquence d'acides aminés qui a au moins 85% d'homologie avec une séquence sélectionnée dans le groupe consistant en la séquence d'acides aminés sur la Fig. I correspondant à P28,
la séquence d'acides aminés sur la Fig. 4B correspondant à OMP-1B,
la séquence d'acides aminés sur la Fig. 5B correspondant à OMP-1C,
la séquence d'acides aminés sur la Fig. 6B correspondant à OMP-1D,
la séquence d'acides aminés sur la Fig. 7B correspondant à OMP-1E,
la séquence d'acides aminés sur la Fig. 8B correspondant à OMP-1F,
où chaque protéine à membrane extérieure ou fragment immunogène de celle-ci est utile pour produire un anticorps qui est immunospécifique à la protéine à membrane extérieure correspondante;
(b) un fragment immunogène de rP28 isolé de la protéine P28, ledit fragment comprenant une séquence identique à la séquence d'acides aminés s'étendant de l'acide aminé 6 jusqu'à l'acide aminé 256 sur la Fig. 1, où le fragment immunogène rP28 se lie à des anticorps dans du sérum d'un humain infecté avec *E*. *chaffensis;* et
(c) un fragment de protéine OMP-1A isolé comprenant la séquence d'acides aminés OMP-1A sur la Fig. 31.

2. Protéine selon la revendication 1(a), où ladite protéine est une protéine OMP-1B qui comprend la séquence d'acides aminés représentée sur la Fig. 4B.

3. Protéine selon la revendication 1(a), où ladite protéine est un fragment immunogène de la protéine à membrane extérieure OMP-1B, ladite protéine à membrane extérieure comprend la séquence d'acides aminés représentée sur la Fig. 4B, où ledit fragment immunogène est utile pour produire un anticorps qui est immunospécifique à la OMP-1B.

4. Protéine selon la revendication 1(a), où ladite protéine est une protéine OMP-1B mature et comprend la séquence s'étendant de l'acide aminé 26 jusqu'à l'acide aminé 283 sur la Fig. 4B.

5. Protéine selon la revendication 1(a), où ladite protéine est une protéine OMP-1C qui comprend la séquence des acides aminés représentée sur la Fig. 5B.

6. Protéine selon la revendication 1(a), où ladite protéine est un fragment immunogène de la protéine à membrane extérieure OMP-1C, ladite protéine à membrane extérieure comprend la séquence d'acides aminés représentée sur la Figure 5B, où ledit fragment immunogène est utile pour produire un anticorps qui est immunospécifique à la OMP-1C.

7. Protéine selon la revendication 1(a), où ladite protéine est une protéine OMP-1C mature et comprend la séquence s'étendant de l'acide aminé 26 jusqu'à l'acide aminé 280 sur la Fig. 5B.

8. Protéine selon la revendication 1(a), où ladite protéine est une protéine OMP-1D qui comprend la séquence d'acides aminés représentée sur la Fig. 6B.

9. Protéine selon la revendication 1(a), où ladite protéine est un fragment immunogène de la protéine à membrane extérieure OMP-1D, ladite protéine à membrane extérieure comprend la séquence d'acides aminés représentée sur la Fig. 6B, où ledit fragment immunogène est utile pour produire un anticorps qui est immunospécifique à la OMP-1D.

10. Protéine selon la revendication la, où ladite protéine est une protéine OMP-1D mature et comprend la séquence s'étendant de l'acide aminé 26 jusqu'à l'acide aminé 286 sur la Fig. 6B.

11. Protéine selon la revendication 1a, où ladite protéine est une protéine OMP-1E qui comprend la séquence d'acides aminés représentée sur la Fig. 7B.

12. Protéine selon la revendication 1(a), où ladite protéine est un fragment immunogène de la protéine à membrane extérieure OMP-1E, ladite protéine à membrane extérieure comprend la séquence d'acides aminés représentée sur la Fig. 7B, où ledit fragment immunogène est utile pour produire un anticorps qui est immunospécifique à la OMP-1E.

13. Protéine selon la revendication 1(a), où ladite protéine est une protéine OMP-1E mature et comprend la séquence s'étendant de l'acide aminé 26 jusqu'à l'acide aminé 278 sur la Fig. 7B.

14. Protéine selon la revendication 1(a), où ladite protéine est une protéine OMP-1F qui comprend la séquence d'acides aminés représentée sur la Fig. 8B.

15. Protéine selon la revendication 1(a), où ladite protéine est un fragment immunogène de la protéine OMP-1F à membrane extérieure, ladite protéine à membrane extérieure comprend la séquence d'acides aminés représentée sur la Fig. 8B, où ledit fragment immunogène est utile pour produire un anticorps qui est immunospécifique à la OMP-1F.

16. Protéine selon la revendication 1(a), où ladite protéine est une protéine OMP-1F mature et comprend la séquence s'étendant de l'acide aminé 26 jusqu'à l'acide aminé 280 sur la Fig. 8B.

17. Protéine selon la revendication 1(a), où ladite protéine est une protéine P28 qui comprend la séquence d'acides aminés représentée sur la Fig. 1.

18. Protéine selon la revendication 1(a), où ladite protéine est un fragment immunogène de la protéine P28 à membrane extérieure, ladite protéine à membrane extérieure comprend la séquence d'acides aminés représentée sur la Fig. 1, où ledit fragment immunogène est utile pour produire un anticorps qui est immunospécifique à P28.

19. Protéine selon la revendication 1(b), où ladite protéine est un fragment immunogène rP28 de la protéine P28, ledit fragment comprend la séquence d'acides aminés s'étendant de l'acide aminé 6 jusqu'à l'acide aminé 256 sur la Fig. 1, où le fragment immunogène rP28 se lie à des anticorps dans du sérum d'un humain infecté avec *E. chaffensis.*

20. Protéine de fusion comprenant le fragment immunogène de la revendication 1(b) et un peptide ou marqueur N-terminal ou C-terminal.

21. Polynucléotide isolé sélectionné dans le groupe:
(a) un polynucléotide isolé codant pour une protéine à membrane extérieure de *E. chaffeensis* ou un fragment immunogène de celle-ci selon la revendication 1(a);
(b) un polynucléotide isolé codant pour un fragment immunogène rP28 selon la revendication 1(b); et
(c) un polynucléotide isolé codant pour un fragment de protéine OMP-1A selon la revendication 1(c).

22. Polynucléotide isolé selon la revendication 21(a), où le polynucléotide code pour une protéine OMP-1B, un fragment immunogène de celle-ci ou une OMP-1B mature selon l'une quelconque des revendications 2, 3 ou 4.

23. Polynucléotide isolé selon la revendication 21(a), où le polynucléotide code pour une protéine OMP-1C, un fragment immunogène de celle-ci ou une OMP-1C mature selon l'une quelconque des revendications 5, 6 ou 7.

24. Polynucléotide isolé selon la revendication 21(a), où le polynucléotide code pour une protéine OMP-1D, un fragment immunogène de celle-ci ou une OMP-1D mature selon l'une quelconque des revendications 8, 9 ou 10.

25. Polynucléotide isolé selon la revendication 21(a), où le polynucléotide code pour une protéine OMP-1E, un fragment immunogène de celle-ci ou une OMP-1E mature selon l'une quelconque des revendications 11, 12 ou 13.

26. Polynucléotide isolé selon la revendication 21(a), où le polynucléotide code pour une protéine OMP-1F, un fragment immunogène de celle-ci ou une OMP-1F mature selon l'une quelconque des revendications 14, 15 ou 16.

27. Polynucléotide isolé selon la revendication 21(a) ou la revendication 21(b), où le polynucléotide code pour une protéine P28, un fragment immunogène de celle-ci ou un fragment immunogène rP28 selon l'une quelconque des revendications 17, 18 et 19.

28. Méthode de détection d'anticorps à *E. chaffensis* dans un échantillon comprenant les étapes de:
(a) réaliser une protéine à membrane extérieure selon la revendication 1(a) ou un fragment immunogène rP28 selon la revendication 1(b);
(b) mettre en contact l'échantillon avec la protéine à membrane extérieure ou le fragment immunogène rP28; et
(c) analyser la formation d'un complexe entre les anticorps dans l'échantillon et la protéine à membrane extérieure ou le fragment immunogène rP28, où la formation dudit complexe est indicative de la présence d'anticorps à *E. chaffensis.*

29. Anticorps immunospécifique à au moins une protéine à membrane extérieure de *E*. *chaffensis,* où ledit anticorps est produit par un immunogène sélectionné parmi une ou plusieurs des suivantes: une protéine OMP-1B selon la revendication 2; une protéine OMP-1C selon la revendication 5; une protéine OMP-1D selon la revendication 8; une protéine OMP-1E selon la revendication 11; une protéine OMP-1F selon la revendication 14 ou une protéine P28 selon la revendication 17.

30. Anticorps selon la revendication 29, où ledit anticorps est monoclonal.

31. Composition immunogène comprenant une protéine sélectionnée parmi:
(a) au moins une protéine à membrane extérieure de *E. chaffensis* selon la revendication 1(a);
(b) un fragment immunogène rP28 selon la revendication 1(b);
et comprenant en outre un adjuvant pharmaceutiquement acceptable, où ladite composition est apte à protéger un mammifère contre une infection avec *E*. *chaffensis.*

32. Vaccin comprenant une composition immunogène selon la revendication 31.


### SEQUENCE LISTING

<110> The Ohio State University Research Foundation
<120> Outer Membrane Protein of Ehrlichia canis and Ehrlichia chaffeensis
<130> SMK/FP5846274
<140> EP 98949384.6
   <141> 1998-09-18
<150> PCT/US98/19600
   <151> 1998-09-18
<150> US 60/059,353
   <151> 1997-09-19
<160> 64
<170> PatentIn version 3.0
<210> 1
   <211> 846
   <212> DNA
   <213> Ehrlichia chaffeensis
<220>
   <221> CDS
   <222> (1)..(846)
<400> 1
   atg aat tac aaa aaa gtt ttc ata aca agt gca ttg ata tca tta ata        48
   Met Asn Tyr Lys Lys Val Phe Ile Thr Ser Ala Leu Ile Ser Leu Ile
   1               5                   10                  15
   tct tct cta cct gga gta tca ttt tcc gac cca gca ggt agt ggt att        96
   Ser Ser Leu Pro Gly Val Ser Phe Ser Asp Pro Ala Gly Ser Gly Ile
               20                  25                  30
   aac ggt aat ttc tac atc agt gga aaa tac atg cca agt gct tcg cat       144
   Asn Gly Asn Phe Tyr Ile Ser Gly Lys Tyr Met Pro Ser Ala Ser His
           35                  40                  45
   ttt gga gta ttc tct gct aag gaa gaa aga aat aca aca gtt gga gtg       192
   Phe Gly Val Phe Ser Ala Lys Glu Glu Arg Asn Thr Thr Val Gly Val
       50                  55                  60
   ttt gga ctg aag caa aat tgg gac gga agc gca ata tcc aac tcc tcc      240
   Phe Gly Leu Lys Gln Asn Trp Asp Gly Ser Ala Ile Ser Asn Ser Ser
   65                  70                  75                  80
   cca aac gat gta ttc act gtc tca aat tat tca ttt aaa tat gaa aac      288
   Pro Asn Asp Val Phe Thr Val Ser Asn Tyr Ser Phe Lys Tyr Glu Asn
                   85                  90                  95
   aac ccg ttt tta ggt ttt gca gga gct att ggt tac tca atg gat ggt      336
   Asn Pro Phe Leu Gly Phe Ala Gly Ala Ile Gly Tyr Ser Met Asp Gly
               100                 105                 110
   cca aga ata gag ctt gaa gta tct tat gaa aca ttt gat gta aaa aat      384
   Pro Arg Ile Glu Leu Glu Val Ser Tyr Glu Thr Phe Asp Val Lys Asn
           115                 120                 125
   caa ggt aac aat tat aag aat gaa gca cat aga tat tgt gct cta tcc      432
   Gln Gly Asn Asn Tyr Lys Asn Glu Ala His Arg Tyr Cys Ala Leu Ser
       130                 135                 140
   cat aac tca gca gca gac atg agt agt gca agt aat aat ttt gtc ttt      480
   His Asn Ser Ala Ala Asp Met Ser Ser Ala Ser Asn Asn Phe Val Phe
   145                 150                 155                 160
   cta aaa aat gaa gga tta ctt gac ata tca ttt atg ctg aac gca tgc      528
   Leu Lys Asn Glu Gly Leu Leu Asp Ile Ser Phe Met Leu Asn Ala Cys
                   165                 170                 175
   tat gac gta gta ggc gaa ggc ata cct ttt tct cct tat ata tgc gca      576
   Tyr Asp Val Val Gly Glu Gly Ile Pro Phe Ser Pro Tyr Ile Cys Ala
               180                 185                 190
   ggt atc ggt act gat tta gta tcc atg ttt gaa gct aca aat cct aaa      624
   Gly Ile Gly Thr Asp Leu Val Ser Met Phe Glu Ala Thr Asn Pro Lys
           195                 200                 205
   att tct tac caa gga aag tta ggt tta agc tac tct ata agc cca gaa      672
   Ile Ser Tyr Gln Gly Lys Leu Gly Leu Ser Tyr Ser Ile Ser Pro Glu
       210                 215                 220
   gct tct gtg ttt att ggt ggg cac ttt cat aag gta tta ggg aac gaa      720
   Ala Ser Val Phe Ile Gly Gly His Phe His Lys Val Leu Gly Asn Glu
   225                 230                 235                 240
   ttt aga gat att cct act ata ata cct act gga tca aca ctt gca gga      768
   Phe Arg Asp Ile Pro Thr Ile Ile Pro Thr Gly Ser Thr Leu Ala Gly
                   245                 250                 255
   aaa gga aac tac cct gca ata gta ata ctg gat gta tgc cac ttt gga      816
   Lys Gly Asn Tyr Pro Ala Ile Val Ile Leu Asp Val Cys His Phe Gly
               260                 265                 270
   ata gaa ctt gga gga agg ttt gta ttc taa                              846
   Ile Glu Leu Gly Gly Arg Phe Val Phe
           275                 280
<210> 2
   <211> 281
   <212> PRT
   <213> Ehrlichia chaffeensis
<400> 2
   Met Asn Tyr Lys Lys Val Phe Ile Thr Ser Ala Leu Ile Ser Leu Ile
   1               5                   10                  15
   Ser Ser Leu Pro Gly Val Ser Phe Ser Asp Pro Ala Gly Ser Gly Ile
               20                  25                  30
   Asn Gly Asn Phe Tyr Ile Ser Gly Lys Tyr Met Pro Ser Ala Ser His
           35                  40                  45
   Phe Gly Val Phe Ser Ala Lys Glu Glu Arg Asn Thr Thr Val Gly Val
       50                  55                  60
   Phe Gly Leu Lys Gln Asn Trp Asp Gly Ser Ala Ile Ser Asn Ser Ser
   65                  70                  75                  80
   Pro Asn Asp Val Phe Thr Val Ser Asn Tyr Ser Phe Lys Tyr Glu Asn
                   85                  90                  95
   Asn Pro Phe Leu Gly Phe Ala Gly Ala Ile Gly Tyr Ser Met Asp Gly
               100                 105                 110
   Pro Arg Ile Glu Leu Glu Val Ser Tyr Glu Thr Phe Asp Val Lys Asn
   115 120 125
   Gln Gly Asn Asn Tyr Lys Asn Glu Ala His Arg Tyr Cys Ala Leu Ser
   130 135 140
   His Asn Ser Ala Ala Asp Met Ser Ser Ala Ser Asn Asn Phe Val Phe
   145 150 155 160
   Leu Lys Asn Glu Gly Leu Leu Asp Ile Ser Phe Met Leu Asn Ala Cys
   165 170 175
   Tyr Asp Val Val Gly Glu Gly Ile Pro Phe Ser Pro Tyr Ile Cys Ala
   180 185 190
   Gly Ile Gly Thr Asp Leu Val Ser Met Phe Glu Ala Thr Asn Pro Lys
   195 200 205
   Ile Ser Tyr Gln Gly Lys Leu Gly Leu Ser Tyr Ser Ile Ser Pro Glu
   210 215 220
   Ala Ser Val Phe Ile Gly Gly His Phe His Lys Val Leu Gly Asn Glu
   225 230 235 240
   Phe Arg Asp Ile Pro Thr Ile Ile Pro Thr Gly Ser Thr Leu Ala Gly
   245 250 255
   Lys Gly Asn Tyr Pro Ala Ile Val Ile Leu Asp Val Cys His Phe Gly
   260 265 270
   Ile Glu Leu Gly Gly Arg Phe Val Phe
   275 280
<210> 3
   <211> 852
   <212> DNA
   <213> Ehrlichia chaffeensis
<220>
   <221> CDS
   <222> (1)..(852)
<400> 3
   atg aat tac aag aaa att ttt gta agc agt gca tta att tca tta atg        48
   Met Asn Tyr Lys Lys Ile Phe Val Ser Ser Ala Leu Ile Ser Leu Met
   1               5                   10                  15
   tca atc tta cct tac caa tct ttt gca gat cct gta act tca aat gat        96
   Ser Ile Leu Pro Tyr Gln Ser Phe Ala Asp Pro Val Thr Ser Asn Asp
               20                  25                  30
   aca gga atc aac gac agc aga gaa ggc ttc tac att agt gta aag tat       144
   Thr Gly Ile Asn Asp Ser Arg Glu Gly Phe Tyr Ile Ser Val Lys Tyr
           35                  40                  45
   aat cca agc ata tca cac ttc aga aaa ttc tca gct gaa gaa gct ccc       192
   Asn Pro Ser Ile Ser His Phe Arg Lys Phe Ser Ala Glu Glu Ala Pro
       50                  55                  60
   atc aat gga aat act tct atc act aaa aag gtt ttc ggg ctg aaa aaa       240
   Ile Asn Gly Asn Thr Ser Ile Thr Lys Lys Val Phe Gly Leu Lys Lys
   65                  70                  75                  80
   gac gga gat ata gca caa tct gcg aat ttt aac agg aca gat cca gcc       288
   Asp Gly Asp Ile Ala Gln Ser Ala Asn Phe Asn Arg Thr Asp Pro Ala
                   85                  90                  95
   ctc gag ttt cag aat aac cta ata tca gga ttc tca gga agt att ggt       336
   Leu Glu Phe Gln Asn Asn Leu Ile Ser Gly Phe Ser Gly Ser Ile Gly
               100                 105                 110
   tat gct atg gat ggg cca aga ata gaa ctt gaa gct gca tac caa aaa       384
   Tyr Ala Met Asp Gly Pro Arg Ile Glu Leu Glu Ala Ala Tyr Gln Lys
           115                 120                 125
   ttt gat gca aaa aat cct gac aac aat gac act aat agc ggt gac tac       432
   Phe Asp Ala Lys Asn Pro Asp Asn Asn Asp Thr Asn Ser Gly Asp Tyr
       130                 135                 140
   tat aaa tac ttt gga cta tct cgt gaa gac gca ata gca gat aag aaa      480
   Tyr Lys Tyr Phe Gly Leu Ser Arg Glu Asp Ala Ile Ala Asp Lys Lys
   145                 150                 155                 160
   tat gtt gtc ctt aaa aat gaa ggc atc act ttt atg tca tta atg gtt      528
   Tyr Val Val Leu Lys Asn Glu Gly Ile Thr Phe Met Ser Leu Met Val
                   165                 170                 175
   aac act tgc tat gac att aca gct gaa gga gta cct ttc ata ccg tat      576
   Asn Thr Cys Tyr Asp Ile Thr Ala Glu Gly Val Pro Phe Ile Pro Tyr
               180                 185                 190
   gca tgt gca ggt gta gga gca gac ctt ata aac gta ttt aag gat ttt      624
   Ala Cys Ala Gly Val Gly Ala Asp Leu Ile Asn Val Phe Lys Asp Phe
           195                 200                 205
   aat tta aaa ttc tca tac caa ggg aaa ata ggt att agc tat cca atc      672
   Asn Leu Lys Phe Ser Tyr Gln Gly Lys Ile Gly Ile Ser Tyr Pro Ile
       210                 215                 220
   aca cca gaa gtt tcc gct ttt att gga gga tac tac cac gga gtt ata      720
   Thr Pro Glu Val Ser Ala Phe Ile Gly Gly Tyr Tyr His Gly Val Ile
   225                 230                 235                 240
   gga aat aat ttt aac aaa ata cct gta ata aca cct gta gta tta gaa      768
   Gly Asn Asn Phe Asn Lys Ile Pro Val Ile Thr Pro Val Val Leu Glu
                   245                 250                 255
   gga gct cct caa aca aca tct gcg cta gta act att gac act gga tac      816
   Gly Ala Pro Gln Thr Thr Ser Ala Leu Val Thr Ile Asp Thr Gly Tyr
               260                 265                 270
   ttt ggc gga gaa gtt gga gta agg ttc acc ttc tag                      852
   Phe Gly Gly Glu Val Gly Val Arg Phe Thr Phe
           275                 280
<210> 4
   <211> 283
   <212> PRT
   <213> Ehrlichia chaffeensis
<400> 4
   Met Asn Tyr Lys Lys Ile Phe Val Ser Ser Ala Leu Ile Ser Leu Met
   1               5                   10                  15
   Ser Ile Leu Pro Tyr Gln Ser Phe Ala Asp Pro Val Thr Ser Asn Asp
   20 25 30
   Thr Gly Ile Asn Asp Ser Arg Glu Gly Phe Tyr Ile Ser Val Lys Tyr
   35 40 45
   Asn Pro Ser Ile Ser His Phe Arg Lys Phe Ser Ala Glu Glu Ala Pro
   50 55 60
   Ile Asn Gly Asn Thr Ser Ile Thr Lys Lys Val Phe Gly Leu Lys Lys
   65 70 75 80
   Asp Gly Asp Ile Ala Gln Ser Ala Asn Phe Asn Arg Thr Asp Pro Ala
   85 90 95
   Leu Glu Phe Gln Asn Asn Leu Ile Ser Gly Phe Ser Gly Ser Ile Gly
   100 105 110
   Tyr Ala Met Asp Gly Pro Arg Ile Glu Leu Glu Ala Ala Tyr Gln Lys
   115 120 125
   Phe Asp Ala Lys Asn Pro Asp Asn Asn Asp Thr Asn Ser Gly Asp Tyr
   130 135 140
   Tyr Lys Tyr Phe Gly Leu Ser Arg Glu Asp Ala Ile Ala Asp Lys Lys
   145 150 155 160
   Tyr Val Val Leu Lys Asn Glu Gly Ile Thr Phe Met Ser Leu Met Val
   165 170 175
   Asn Thr Cys Tyr Asp Ile Thr Ala Glu Gly Val Pro Phe Ile Pro Tyr
   180 185 190
   Ala Cys Ala Gly Val Gly Ala Asp Leu Ile Asn Val Phe Lys Asp Phe
   195 200 205
   Asn Leu Lys Phe Ser Tyr Gln Gly Lys Ile Gly Ile Ser Tyr Pro Ile
   210 215 220
   Thr Pro Glu Val Ser Ala Phe Ile Gly Gly Tyr Tyr His Gly Val Ile
   225 230 235 240
   Gly Asn Asn Phe Asn Lys Ile Pro Val Ile Thr Pro Val Val Leu Glu
   245 250 255
   Gly Ala Pro Gln Thr Thr Ser Ala Leu Val Thr Ile Asp Thr Gly Tyr
   260 265 270
   Phe Gly Gly Glu Val Gly Val Arg Phe Thr Phe
   275 280
<210> 5
   <211> 843
   <212> DNA
   <213> Ehrlichia chaffeensis
<220>
   <221> CDS
   <222> (1)..(843)
<400> 5
   atg aac tgc aaa aaa ttt ttt ata aca act gca ttg gca ttg cca atg 48
   Met Asn Cys Lys Lys Phe Phe Ile Thr Thr Ala Leu Ala Leu Pro Met
   1 5 10 15
   tct ttc tta cct gga ata tta ctt tct gaa cca gta caa gat gac agt 96
   Ser Phe Leu Pro Gly Ile Leu Leu Ser Glu Pro Val Gln Asp Asp Ser
   20 25 30
   gtg agt ggc aat ttc tat att agt ggc aag tac atg cca agt gct tct 144
   Val Ser Gly Asn Phe Tyr Ile Ser Gly Lys Tyr Met Pro Ser Ala Ser
   35 40 45
   cat ttt gga gtt ttc tct gcc aaa gaa gaa aaa aat cct act gtc gcg      192
   His Phe Gly Val Phe Ser Ala Lys Glu Glu Lys Asn Pro Thr Val Ala
       50                  55                  60
   ttg tat ggt ttg aaa caa gat tgg aac ggt gtt agt gct tca agt cat      240
   Leu Tyr Gly Leu Lys Gln Asp Trp Asn Gly Val Ser Ala Ser Ser His
   65                  70                  75                  80
   gct gat gcg gac ttt aat aac aaa ggt tat tct ttt aaa tac gaa aac      288
   Ala Asp Ala Asp Phe Asn Asn Lys Gly Tyr Ser Phe Lys Tyr Glu Asn
                   85                  90                  95
   aat cca ttt cta ggt ttt gca gga gct att ggt tat tca atg ggt ggt      336
   Asn Pro Phe Leu Gly Phe Ala Gly Ala Ile Gly Tyr Ser Met Gly Gly
               100                 105                 110
   cca aga ata gag ttt gaa gtg tcc tat gaa aca ttt gac gtg aaa aat      384
   Pro Arg Ile Glu Phe Glu Val Ser Tyr Glu Thr Phe Asp Val Lys Asn
           115                 120                 125
   caa ggt ggt aat tac aaa aat gat gct cac aga tac tgt gcc tta gat      432
   Gln Gly Gly Asn Tyr Lys Asn Asp Ala His Arg Tyr Cys Ala Leu Asp
       130                 135                 140
   cgt aaa gca agc agc act aat gcc aca gct agt cac tac gtg cta cta      480
   Arg Lys Ala Ser Ser Thr Asn Ala Thr Ala Ser His Tyr Val Leu Leu
   145                 150                 155                 160
   aaa aat gaa gga cta ctt gat ata tca ctt atg ttg aat gca tgc tat      528
   Lys Asn Glu Gly Leu Leu Asp Ile Ser Leu Met Leu Asn Ala Cys Tyr
                   165                 170                 175
   gac gta gta agt gaa gga ata cct tcc tct cct tac ata tgt gca ggt      576
   Asp Val Val Ser Glu Gly Ile Pro Ser Ser Pro Tyr Ile Cys Ala Gly
               180                 185                 190
   gtt ggt acc gat tta ata tcc atg ttt gaa gct ata aac cct aaa att      624
   Val Gly Thr Asp Leu Ile Ser Met Phe Glu Ala Ile Asn Pro Lys Ile
           195                 200                 205
   tct tat caa gga aag tta ggt ttg agt tac tct ata aac cca gaa gct      672
   Ser Tyr Gln Gly Lys Leu Gly Leu Ser Tyr Ser Ile Asn Pro Glu Ala
       210                 215                 220
   tct gtc ttt gtt ggt gga cat ttt cat aaa gtt gca ggt aat gaa ttc      720
   Ser Val Phe Val Gly Gly His Phe His Lys Val Ala Gly Asn Glu Phe
   225                 230                 235                 240
   agg gac att tct act ctt aaa gcg ttt gct aca cca tca tct gca gct      768
   Arg Asp Ile Ser Thr Leu Lys Ala Phe Ala Thr Pro Ser Ser Ala Ala
                   245                 250                 255
   act cca gac tta gca aca gta aca ctg agt gtg tgt cac ttt gga gta      816
   Thr Pro Asp Leu Ala Thr Val Thr Leu Ser Val Cys His Phe Gly Val
               260                 265                 270
   gaa ctt gga gga aga ttt aac ttc taa                                  843
   Glu Leu Gly Gly Arg Phe Asn Phe
           275                 280
<210> 6
   <211> 280
   <212> PRT
   <213> Ehrlichia chaffeensis
<400> 6
   Met Asn Cys Lys Lys Phe Phe Ile Thr Thr Ala Leu Ala Leu Pro Met
   1               5                   10                  15
   Ser Phe Leu Pro Gly Ile Leu Leu Ser Glu Pro Val Gln Asp Asp Ser
               20                  25                  30
   Val Ser Gly Asn Phe Tyr Ile Ser Gly Lys Tyr Met Pro Ser Ala Ser
           35                  40                  45
   His Phe Gly Val Phe Ser Ala Lys Glu Glu Lys Asn Pro Thr Val Ala
       50                  55                  60
   Leu Tyr Gly Leu Lys Gln Asp Trp Asn Gly Val Ser Ala Ser Ser His
   65                  70                  75                  80
   Ala Asp Ala Asp Phe Asn Asn Lys Gly Tyr Ser Phe Lys Tyr Glu Asn
                   85                  90                  95
   Asn Pro Phe Leu Gly Phe Ala Gly Ala Ile Gly Tyr Ser Met Gly Gly
               100                 105                 110
   Pro Arg Ile Glu Phe Glu Val Ser Tyr Glu Thr Phe Asp Val Lys Asn
           115                 120                 125
   Gln Gly Gly Asn Tyr Lys Asn Asp Ala His Arg Tyr Cys Ala Leu Asp
       130                 135                 140
   Arg Lys Ala Ser Ser Thr Asn Ala Thr Ala Ser His Tyr Val Leu Leu
   145                 150                 155                 160
   Lys Asn Glu Gly Leu Leu Asp Ile Ser Leu Met Leu Asn Ala Cys Tyr
                   165                 170                 175
   Asp Val Val Ser Glu Gly Ile Pro Ser Ser Pro Tyr Ile Cys Ala Gly
               180                 185                 190
   Val Gly Thr Asp Leu Ile Ser Met Phe Glu Ala Ile Asn Pro Lys Ile
           195                 200                 205
   Ser Tyr Gln Gly Lys Leu Gly Leu Ser Tyr Ser Ile Asn Pro Glu Ala
       210                 215                 220
   Ser Val Phe Val Gly Gly His Phe His Lys Val Ala Gly Asn Glu Phe
   225                 230                 235                 240
   Arg Asp Ile Ser Thr Leu Lys Ala Phe Ala Thr Pro Ser Ser Ala Ala
                   245                 250                 255
   Thr Pro Asp Leu Ala Thr Val Thr Leu Ser Val Cys His Phe Gly Val
               260                 265                 270
   Glu Leu Gly Gly Arg Phe Asn Phe
           275                 280
<210> 7
   <211> 861
   <212> DNA
   <213> Ehrlichia chaffeensis
<220>
   <221> CDS
   <222> (1)..(861)
<400> 7
   atg aac tgc gaa aaa ttt ttt ata aca act gca tta aca tta cta atg       48
   Met Asn Cys Glu Lys Phe Phe Ile Thr Thr Ala Leu Thr Leu Leu Met
   1               5                   10                  15
   tcc ttc tta cct gga ata tca ctt tct gat cca gta cag gat gac aac       96
   Ser Phe Leu Pro Gly Ile Ser Leu Ser Asp Pro Val Gln Asp Asp Asn
               20                  25                  30
   att agt ggt aat ttc tac atc agt gga aag tat atg cca agc gct tcg      144
   Ile Ser Gly Asn Phe Tyr Ile Ser Gly Lys Tyr Met Pro Ser Ala Ser
           35                  40                  45
   cat ttt gga gtt ttt tct gcc aag gaa gaa aga aat aca aca gtt gga      192
   His Phe Gly Val Phe Ser Ala Lys Glu Glu Arg Asn Thr Thr Val Gly
       50                  55                  60
   gta ttt gga ata gag caa gat tgg gat aga tgt gta ata tct aga acc      240
   Val Phe Gly Ile Glu Gln Asp Trp Asp Arg Cys Val Ile Ser Arg Thr
   65                  70                  75                  80
   act tta agc cat ata ttc acc gtt cca aat tat tca ttt aag tat gaa      288
   Thr Leu Ser His Ile Phe Thr Val Pro Asn Tyr Ser Phe Lys Tyr Glu
                   85                  90                  95
   aat aat cta ttt tca gga ttt gca gga gct att ggc tac tca atg gat      336
   Asn Asn Leu Phe Ser Gly Phe Ala Gly Ala Ile Gly Tyr Ser Met Asp
               100                 105                 110
   ggc cca aga ata gag ctt gaa gta tct tat gaa gca ttc gat gtt aaa      384
   Gly Pro Arg Ile Glu Leu Glu Val Ser Tyr Glu Ala Phe Asp Val Lys
           115                 120                 125
   aat caa ggt aac aat tat aag aac gaa gca cat aga tat tat gct ctg      432
   Asn Gln Gly Asn Asn Tyr Lys Asn Glu Ala His Arg Tyr Tyr Ala Leu
       130                 135                 140
   tcc cat ctt ctc ggc aca gag aca cag ata gat ggt gca ggc agt gcg        480
   Ser His Leu Leu Gly Thr Glu Thr Gln Ile Asp Gly Ala Gly Ser Ala
   145                 150                 155                 160
   tct gtc ttt cta ata aat gaa gga cta ctt gat aaa tca ttt atg ctg        528
   Ser Val Phe Leu Ile Asn Glu Gly Leu Leu Asp Lys Ser Phe Met Leu
                   165                 170                 175
   aac gca tgt tat gat gta ata agt gaa ggc ata cct ttt tct cct tat        576
   Asn Ala Cys Tyr Asp Val Ile Ser Glu Gly Ile Pro Phe Ser Pro Tyr
               180                 185                 190
   ata tgt gca ggt att ggt att gat tta gta tcc atg ttt gaa gct ata        624
   Ile Cys Ala Gly Ile Gly Ile Asp Leu Val Ser Met Phe Glu Ala Ile
           195                 200                 205
   aat cct aaa att tct tat caa gga aaa tta ggc tta agt tac cct ata        672
   Asn Pro Lys Ile Ser Tyr Gln Gly Lys Leu Gly Leu Ser Tyr Pro Ile
       210                 215                 220
   agc cca gaa gct tct gtg ttt att ggt gga cat ttt cat aag gtg ata        720
   Ser Pro Glu Ala Ser Val Phe Ile Gly Gly His Phe His Lys Val Ile
   225                 230                 235                 240
   gga aac gaa ttt aga gat att cct act atg ata cct agt gaa tca gcg        768
   Gly Asn Glu Phe Arg Asp Ile Pro Thr Met Ile Pro Ser Glu Ser Ala
                   245                 250                 255
   ctt gca gga aaa gga aac tac cct gca ata gta aca ctg gac gtg ttc        816
   Leu Ala Gly Lys Gly Asn Tyr Pro Ala Ile Val Thr Leu Asp Val Phe
               260                 265                 270
   tac ttt ggc ata gaa ctt gga gga agg ttt aac ttc caa ctt tga            861
   Tyr Phe Gly Ile Glu Leu Gly Gly Arg Phe Asn Phe Gln Leu
           275                 280                 285
<210> 8
   <211> 286
   <212> PRT
   <213> Ehrlichia chaffeensis
<400> 8
   Met Asn Cys Glu Lys Phe Phe Ile Thr Thr Ala Leu Thr Leu Leu Met
   1               5                   10                  15
   Ser Phe Leu Pro Gly Ile Ser Leu Ser Asp Pro Val Gln Asp Asp Asn
               20                  25                  30
   Ile Ser Gly Asn Phe Tyr Ile Ser Gly Lys Tyr Met Pro Ser Ala Ser
           35                  40                  45
   His Phe Gly Val Phe Ser Ala Lys Glu Glu Arg Asn Thr Thr Val Gly
       50                  55                  60
   Val Phe Gly Ile Glu Gln Asp Trp Asp Arg Cys Val Ile Ser Arg Thr
   65                  70                  75                  80
   Thr Leu Ser His Ile Phe Thr Val Pro Asn Tyr Ser Phe Lys Tyr Glu
                   85                  90                  95
   Asn Asn Leu Phe Ser Gly Phe Ala Gly Ala Ile Gly Tyr Ser Met Asp
               100                 105                 110
   Gly Pro Arg Ile Glu Leu Glu Val Ser Tyr Glu Ala Phe Asp Val Lys
           115                 120                 125
   Asn Gln Gly Asn Asn Tyr Lys Asn Glu Ala His Arg Tyr Tyr Ala Leu
       130                 135                 140
   Ser His Leu Leu Gly Thr Glu Thr Gln Ile Asp Gly Ala Gly Ser Ala
   145                 150                 155                 160
   Ser Val Phe Leu Ile Asn Glu Gly Leu Leu Asp Lys Ser Phe Met Leu
                   165                 170                 175
   Asn Ala Cys Tyr Asp Val Ile Ser Glu Gly Ile Pro Phe Ser Pro Tyr
               180                 185                 190
   Ile Cys Ala Gly Ile Gly Ile Asp Leu Val Ser Met Phe Glu Ala Ile
   195 200 205
   Asn Pro Lys Ile Ser Tyr Gln Gly Lys Leu Gly Leu Ser Tyr Pro Ile
   210 215 220
   Ser Pro Glu Ala Ser Val Phe Ile Gly Gly His Phe His Lys Val Ile
   225 230 235 240
   Gly Asn Glu Phe Arg Asp Ile Pro Thr Met Ile Pro Ser Glu Ser Ala
   245 250 255
   Leu Ala Gly Lys Gly Asn Tyr Pro Ala Ile Val Thr Leu Asp Val Phe
   260 265 270
   Tyr Phe Gly Ile Glu Leu Gly Gly Arg Phe Asn Phe Gln Leu
   275 280 285
<210> 9
   <211> 837
   <212> DNA
   <213> Ehrlichia chaffeensis
<220>
   <221> CDS
   <222> (1)..(837)
<400> 9
   atg aat tgc aaa aaa ttt ttt ata aca act gca tta gta tca cta atg 48
   Met Asn Cys Lys Lys Phe Phe Ile Thr Thr Ala Leu Val Ser Leu Met
   1 5 10 15
   tcc ttt cta cct gga ata tca ttt tct gat cca gtg caa ggt gac aat 96
   Ser Phe Leu Pro Gly Ile Ser Phe Ser Asp Pro Val Gln Gly Asp Asn
   20 25 30
   att agt ggt aat ttc tat gtt agt ggc aag tat atg cca agt gct tcg 144
   Ile Ser Gly Asn Phe Tyr Val Ser Gly Lys Tyr Met Pro Ser Ala Ser
   35 40 45
   cat ttt ggc atg ttt tct gcc aaa gaa gaa aaa aat cct act gtt gca      192
   His Phe Gly Met Phe Ser Ala Lys Glu Glu Lys Asn Pro Thr Val Ala
       50                  55                  60
   ttg tat ggc tta aaa caa gat tgg gaa ggg att agc tca tca agt cac      240
   Leu Tyr Gly Leu Lys Gln Asp Trp Glu Gly Ile Ser Ser Ser Ser His
   65                  70                  75                  80
   aat gat aat cat ttc aat aac aag ggt tat tca ttt aaa tat gaa aat      288
   Asn Asp Asn His Phe Asn Asn Lys Gly Tyr Ser Phe Lys Tyr Glu Asn
                   85                  90                  95
   aac cca ttt tta ggg ttt gca gga gct att ggt tat tca atg ggt ggt      336
   Asn Pro Phe Leu Gly Phe Ala Gly Ala Ile Gly Tyr Ser Met Gly Gly
               100                 105                 110
   cca aga gta gag ttt gaa gtg tcc tat gaa aca ttt gac gtt aaa aat      384
   Pro Arg Val Glu Phe Glu Val Ser Tyr Glu Thr Phe Asp Val Lys Asn
           115                 120                 125
   cag ggt aat aac tat aaa aat gat gct cac aga tac tgt gct tta ggt      432
   Gln Gly Asn Asn Tyr Lys Asn Asp Ala His Arg Tyr Cys Ala Leu Gly
       130                 135                 140
   caa caa gac aac agc gga ata cct aaa act agt aaa tac gta ctg tta      480
   Gln Gln Asp Asn Ser Gly Ile Pro Lys Thr Ser Lys Tyr Val Leu Leu
   145                 150                 155                 160
   aaa agc gaa gga ttg ctt gac ata tca ttt atg cta aat gca tgc tat      528
   Lys Ser Glu Gly Leu Leu Asp Ile Ser Phe Met Leu Asn Ala Cys Tyr
                   165                 170                 175
   gat ata ata aac gag agc ata cct ttg tct cct tac ata tgt gca ggt      576
   Asp Ile Ile Asn Glu Ser Ile Pro Leu Ser Pro Tyr Ile Cys Ala Gly
               180                 185                 190
   gtt ggt act gat tta ata tcc atg ttt gaa gct aca aat cct aaa att      624
   Val Gly Thr Asp Leu Ile Ser Met Phe Glu Ala Thr Asn Pro Lys Ile
           195                 200                 205
   tct tac caa ggg aag tta ggt cta agt tac tct ata aac cca gaa gct      672
   Ser Tyr Gln Gly Lys Leu Gly Leu Ser Tyr Ser Ile Asn Pro Glu Ala
       210                 215                 220
   tct gta ttt att ggt gga cat ttt cat aag gtg ata gga aac gaa ttt        720
   Ser Val Phe Ile Gly Gly His Phe His Lys Val Ile Gly Asn Glu Phe
   225                 230                 235                 240
   agg gac att cct act ctg aaa gca ttt gtt acg tca tca gct act cca        768
   Arg Asp Ile Pro Thr Leu Lys Ala Phe Val Thr Ser Ser Ala Thr Pro
                   245                 250                 255
   gat cta gca ata gta aca cta agt gta tgt cat ttt gga ata gaa ctt        816
   Asp Leu Ala Ile Val Thr Leu Ser Val Cys His Phe Gly Ile Glu Leu
               260                 265                 270
   gga gga agg ttt aac ttc taa                                            837
   Gly Gly Arg Phe Asn Phe
           275
<210> 10
   <211> 278
   <212> PRT
   <213> Ehrlichia chaffeensis
<400> 10
   Met Asn Cys Lys Lys Phe Phe Ile Thr Thr Ala Leu Val Ser Leu Met
   1 5 10 15
   Ser Phe Leu Pro Gly Ile Ser Phe Ser Asp Pro Val Gln Gly Asp Asn
   20 25 30
   Ile Ser Gly Asn Phe Tyr Val Ser Gly Lys Tyr Met Pro Ser Ala Ser
   35 40 45
   His Phe Gly Met Phe Ser Ala Lys Glu Glu Lys Asn Pro Thr Val Ala
   50 55 60
   Leu Tyr Gly Leu Lys Gln Asp Trp Glu Gly Ile Ser Ser Ser Ser His
   65 70 75 80
   Asn Asp Asn His Phe Asn Asn Lys Gly Tyr Ser Phe Lys Tyr Glu Asn
   85 90 95
   Asn Pro Phe Leu Gly Phe Ala Gly Ala Ile Gly Tyr Ser Met Gly Gly
   100 105 110
   Pro Arg Val Glu Phe Glu Val Ser Tyr Glu Thr Phe Asp Val Lys Asn
   115 120 125
   Gln Gly Asn Asn Tyr Lys Asn Asp Ala His Arg Tyr Cys Ala Leu Gly
   130 135 140
   Gln Gln Asp Asn Ser Gly Ile Pro Lys Thr Ser Lys Tyr Val Leu Leu
   145 150 155 160
   Lys Ser Glu Gly Leu Leu Asp Ile Ser Phe Met Leu Asn Ala Cys Tyr
   165 170 175
   Asp Ile Ile Asn Glu Ser Ile Pro Leu Ser Pro Tyr Ile Cys Ala Gly
   180 185 190
   Val Gly Thr Asp Leu Ile Ser Met Phe Glu Ala Thr Asn Pro Lys Ile
   195 200 205
   Ser Tyr Gln Gly Lys Leu Gly Leu Ser Tyr Ser Ile Asn Pro Glu Ala
   210 215 220
   Ser Val Phe Ile Gly Gly His Phe His Lys Val Ile Gly Asn Glu Phe
   225 230 235 240
   Arg Asp Ile Pro Thr Leu Lys Ala Phe Val Thr Ser Ser Ala Thr Pro
   245 250 255
   Asp Leu Ala Ile Val Thr Leu Ser Val Cys His Phe Gly Ile Glu Leu
   260 265 270
   Gly Gly Arg Phe Asn Phe
   275
<210> 11
   <211> 843
   <212> DNA
   <213> Ehrlichia chaffeensis
<220>
   <221> CDS
   <222> (1)..(843)
<400> 11
   atg aat tgc aaa aaa ttt ttt ata aca act aca tta gta tcg cta atg        48
   Met Asn Cys Lys Lys Phe Phe Ile Thr Thr Thr Leu Val Ser Leu Met
   1               5                   10                  15
   tcc ttc tta cct gga ata tca ttt tct gat gca gta cag aac gac aat        96
   Ser Phe Leu Pro Gly Ile Ser Phe Ser Asp Ala Val Gln Asn Asp Asn
               20                  25                  30
   gtt ggt ggt aat ttc tat atc agt ggg aaa tat gta cca agt gtt tca       144
   Val Gly Gly Asn Phe Tyr Ile Ser Gly Lys Tyr Val Pro Ser Val Ser
           35                  40                  45
   cat ttt ggc gta ttc tct gct aaa cag gaa aga aat aca aca acc gga       192
   His Phe Gly Val Phe Ser Ala Lys Gln Glu Arg Asn Thr Thr Thr Gly
       50                  55                  60
   gta ttt gga tta aag caa gat tgg gat ggc agc aca ata tct aaa aat       240
   Val Phe Gly Leu Lys Gln Asp Trp Asp Gly Ser Thr Ile Ser Lys Asn
   65                  70                  75                  80
   tct cca gaa aat aca ttt aac gtt cca aat tat tca ttt aaa tat gaa       288
   Ser Pro Glu Asn Thr Phe Asn Val Pro Asn Tyr Ser Phe Lys Tyr Glu
                   85                  90                  95
   aat aat cca ttt cta ggt ttt gca gga gct gtt ggt tat tta atg aat       336
   Asn Asn Pro Phe Leu Gly Phe Ala Gly Ala Val Gly Tyr Leu Met Asn
               100                 105                 110
   ggt cca aga ata gag tta gaa atg tcc tat gaa aca ttt gat gtg aaa       384
   Gly Pro Arg Ile Glu Leu Glu Met Ser Tyr Glu Thr Phe Asp Val Lys
           115                 120                 125
   aac cag ggt aat aac tat aag aac gat gct cac aaa tat tat gct tta       432
   Asn Gln Gly Asn Asn Tyr Lys Asn Asp Ala His Lys Tyr Tyr Ala Leu
       130                 135                 140
   acc cat aac agt ggg gga aag cta agc aat gca ggt gat aag ttt gtt      480
   Thr His Asn Ser Gly Gly Lys Leu Ser Asn Ala Gly Asp Lys Phe Val
   145                 150                 155                 160
   ttt cta aaa aat gaa gga cta ctt gat ata tca ctt atg ttg aat gca      528
   Phe Leu Lys Asn Glu Gly Leu Leu Asp Ile Ser Leu Met Leu Asn Ala
                   165                 170                 175
   tgc tat gat gta ata agt gaa gga ata cct ttc tct cct tac ata tgt      576
   Cys Tyr Asp Val Ile Ser Glu Gly Ile Pro Phe Ser Pro Tyr Ile Cys
               180                 185                 190
   gca ggt gtt ggt act gat tta ata tcc atg ttt gaa gct ata aac cct      624
   Ala Gly Val Gly Thr Asp Leu Ile Ser Met Phe Glu Ala Ile Asn Pro
           195                 200                 205
   aaa att tct tat caa gga aag tta ggt ttg agt tac tcc ata agc cca      672
   Lys Ile Ser Tyr Gln Gly Lys Leu Gly Leu Ser Tyr Ser Ile Ser Pro
       210                 215                 220
   gaa gct tct gtt ttt gtt ggt gga cat ttt cat aag gtg ata ggg aat      720
   Glu Ala Ser Val Phe Val Gly Gly His Phe His Lys Val Ile Gly Asn
   225                 230                 235                 240
   gaa ttc aga gat att cct gct atg ata ccc agt acc tca act ctc aca      768
   Glu Phe Arg Asp Ile Pro Ala Met Ile Pro Ser Thr Ser Thr Leu Thr
                   245                 250                 255
   ggt aat cac ttt act ata gta aca cta agt gta tgc cac ttt gga gtg      816
   Gly Asn His Phe Thr Ile Val Thr Leu Ser Val Cys His Phe Gly Val
               260                 265                 270
   gaa ctt gga gga agg ttt aac ttt taa                                  843
   Glu Leu Gly Gly Arg Phe Asn Phe
           275                 280
<210> 12
   <211> 280
   <212> PRT
   <213> Ehrlichia chaffeensis
<400> 12
   Met Asn Cys Lys Lys Phe Phe Ile Thr Thr Thr Leu Val Ser Leu Met
   1               5                   10                  15
   Ser Phe Leu Pro Gly Ile Ser Phe Ser Asp Ala Val Gln Asn Asp Asn
               20                  25                  30
   Val Gly Gly Asn Phe Tyr Ile Ser Gly Lys Tyr Val Pro Ser Val Ser
           35                  40                  45
   His Phe Gly Val Phe Ser Ala Lys Gln Glu Arg Asn Thr Thr Thr Gly
       50                  55                  60
   Val Phe Gly Leu Lys Gln Asp Trp Asp Gly Ser Thr Ile Ser Lys Asn
   65                  70                  75                  80
   Ser Pro Glu Asn Thr Phe Asn Val Pro Asn Tyr Ser Phe Lys Tyr Glu
                   85                  90                  95
   Asn Asn Pro Phe Leu Gly Phe Ala Gly Ala Val Gly Tyr Leu Met Asn
               100                 105                 110
   Gly Pro Arg Ile Glu Leu Glu Met Ser Tyr Glu Thr Phe Asp Val Lys
           115                 120                 125
   Asn Gln Gly Asn Asn Tyr Lys Asn Asp Ala His Lys Tyr Tyr Ala Leu
       130                 135                 140
   Thr His Asn Ser Gly Gly Lys Leu Ser Asn Ala Gly Asp Lys Phe Val
   145                 150                 155                 160
   Phe Leu Lys Asn Glu Gly Leu Leu Asp Ile Ser Leu Met Leu Asn Ala
                   165                 170                 175
   Cys Tyr Asp Val Ile Ser Glu Gly Ile Pro Phe Ser Pro Tyr Ile Cys
               180                 185                 190
   Ala Gly Val Gly Thr Asp Leu Ile Ser Met Phe Glu Ala Ile Asn Pro
           195                 200                 205
   Lys Ile Ser Tyr Gln Gly Lys Leu Gly Leu Ser Tyr Ser Ile Ser Pro
       210                 215                 220
   Glu Ala Ser Val Phe Val Gly Gly His Phe His Lys Val Ile Gly Asn
   225                 230                 235                 240
   Glu Phe Arg Asp Ile Pro Ala Met Ile Pro Ser Thr Ser Thr Leu Thr
                   245                 250                 255
   Gly Asn His Phe Thr Ile Val Thr Leu Ser Val Cys His Phe Gly Val
               260                 265                 270
   Glu Leu Gly Gly Arg Phe Asn Phe
           275                 280
<210> 13
   <211> 894
   <212> DNA
   <213> Ehrlichia chaffeensis
<400> 13
   atggaaaatc tcatgaataa gaaaaacaaa ttctttacaa taagtacagc aatggtatgc 60
   ttattgttat tacctggtat atcattttca gaaactataa acaacagtgc taaaaaacag 120
   cctgggttat atatcagtgg gcagtacaaa cctagtgttt cagtttttag taatttttca 180
   gtaaaagaaa ctaatgttcc cacaaagcag ttaatagcac ttaaaaaaga cattaattct 240
   gttgcagttg gtagtaatgc tactacaggt attagcaatc caggtaattt cacaattcct 300
   tatactgcag aatttcaaga taatgttgcc aatttcaatg gggctgttgg ttactctttt 360
   cctgatagtc taagaattga aatagaggga tttcatgaaa aatttgatgt caaaaaccct 420
   ggaggttaca cacaagtaaa agatgcgtac cgttattttg cactagcacg tgatttaaaa 480
   gatggcttct ttgaacctaa agcggaagat acaggtgttt atcatactgt tatgaaaaat 540
   gatggattat ctattttatc tactatggtt aacgtctgtt acgatttttc tgtagatgaa 600
   ttaccagtct taccttatat atgtgcaggt atgggtataa acgccataga attcttcgac 660
   gctttacatg taaaatttgc ttaccaaggc aaactaggta ttagctatca actatttact 720
   aaagtaaatt tattccttga tgggtattac catcaagtaa taggcaatca atccaaaaac 780
   ttaaacgtaa accatgttta cacacttaaa gaatctccta aagtcacatc tgcagtagct 840
   acacttgaca ttgcatactt tggtgccgaa gttggaataa gattcacatt ttaa 894
<210> 14
   <211> 280
   <212> PRT
   <213> Ehrlichia chaffeensis
<400> 14
   Met Val Cys Leu Leu Leu Leu Pro Gly Ile Ser Phe Ser Glu Thr Ile
   1               5                   10                  15
   Asn Asn Ser Ala Lys Lys Gln Pro Gly Leu Tyr Ile Ser Gly Gln Tyr
               20                  25                  30
   Lys Pro Ser Val Ser Val Phe Ser Asn Phe Ser Val Lys Glu Thr Asn
           35                  40                  45
   Val Pro Thr Lys Gln Leu Ile Ala Leu Lys Lys Asp Ile Asn Ser Val
       50                  55                  60
   Ala Val Gly Ser Asn Ala Thr Thr Gly Ile Ser Asn Pro Gly Asn Phe
   65                  70                  75                  80
   Thr Ile Pro Tyr Thr Ala Glu Phe Gln Asp Asn Val Ala Asn Phe Asn
                   85                  90                  95
   Gly Ala Val Gly Tyr Ser Phe Pro Asp Ser Leu Arg Ile Glu Ile Glu
               100                 105                 110
   Gly Phe His Glu Lys Phe Asp Val Lys Asn Pro Gly Gly Tyr Thr Gln
           115                 120                 125
   Val Lys Asp Ala Tyr Arg Tyr Phe Ala Leu Ala Arg Asp Leu Lys Asp
       130                 135                 140
   Gly Phe Phe Glu Pro Lys Ala Glu Asp Thr Gly Val Tyr His Thr Val
   145 150 155 160
   Met Lys Asn Asp Gly Leu Ser Ile Leu Ser Thr Met Val Asn Val Cys
   165 170 175
   Tyr Asp Phe Ser Val Asp Glu Leu Pro Val Leu Pro Tyr Ile Cys Ala
   180 185 190
   Gly Met Gly Ile Asn Ala Ile Glu Phe Phe Asp Ala Leu His Val Lys
   195 200 205
   Phe Ala Tyr Gln Gly Lys Leu Gly Ile Ser Tyr Gln Leu Phe Thr Lys
   210 215 220
   Val Asn Leu Phe Leu Asp Gly Tyr Tyr His Gln Val Ile Gly Asn Gln
   225 230 235 240
   Phe Lys Asn Leu Asn Val Asn His Val Tyr Thr Leu Lys Glu Ser Pro
   245 250 255
   Lys Val Thr Ser Ala Val Ala Thr Leu Asp Ile Ala Tyr Phe Gly Gly
   260 265 270
   Glu Val Gly Ile Arg Phe Thr Phe
   275 280
<210> 15
   <211> 591
   <212> DNA
   <213> Ehrlichia chaffeensis
<220>
   <221> CDS
   <222> (1)..(591)
<400> 15
   atg ata tat aaa gaa aaa ctt act aga gtg gga gaa tat atc tta gca 48
   Met Ile Tyr Lys Glu Lys Leu Thr Arg Val Gly Glu Tyr Ile Leu Ala
   1 5 10 15
   tat tta tca ttt att ctt tct act tat atc ttt cta gtg ctg gta aat 96
   Tyr Leu Ser Phe Ile Leu Ser Thr Tyr Ile Phe Leu Val Leu Val Asn
   20 25 30
   att att aga tat aac agc ctt gct ata tgt gtt atc agt cta cta aga        144
   Ile Ile Arg Tyr Asn Ser Leu Ala Ile Cys Val Ile Ser Leu Leu Arg
           35                  40                  45
   act aat atc ttt aac gtt agc aca aaa aaa tta ata aaa gat aaa tgt        192
   Thr Asn Ile Phe Asn Val Ser Thr Lys Lys Leu Ile Lys Asp Lys Cys
       50                  55                  60
   cgt gat act aag ttt agt aac atg aaa tgt tat ttg tac ggt aaa ccg        240
   Arg Asp Thr Lys Phe Ser Asn Met Lys Cys Tyr Leu Tyr Gly Lys Pro
   65                  70                  75                  80
   tta aat tta caa att ttt tat gga ata ttt tcc ttt att aga aac ttt        288
   Leu Asn Leu Gln Ile Phe Tyr Gly Ile Phe Ser Phe Ile Arg Asn Phe
                   85                  90                  95
   caa aat aac aca cta ata att cct aat gat agt aaa tgc ggc ttc tat        336
   Gln Asn Asn Thr Leu Ile Ile Pro Asn Asp Ser Lys Cys Gly Phe Tyr
               100                 105                 110
   acc acg tta tgg gat aat cca gca cta cat tat aca tat aca ctt act        384
   Thr Thr Leu Trp Asp Asn Pro Ala Leu His Tyr Thr Tyr Thr Leu Thr
           115                 120                 125
   ggc agt gag tac cgt aat ttt ttt gac att cta tat gaa aac att atc        432
   Gly Ser Glu Tyr Arg Asn Phe Phe Asp Ile Leu Tyr Glu Asn Ile Ile
       130                 135                 140
   tgt caa tgt aaa tta ctt att aac tat aac cgt tct gta tta aac caa        480
   Cys Gln Cys Lys Leu Leu Ile Asn Tyr Asn Arg Ser Val Leu Asn Gln
   145                 150                 155                 160
   cat aat aaa aat act ctc gta ata ata cca ata cct aat gct aga gag        528
   His Asn Lys Asn Thr Leu Val Ile Ile Pro Ile Pro Asn Ala Arg Glu
                   165                 170                 175
   ttc agt aat gaa att cga gta agg aat ata tca ata aat aag gaa agt        576
   Phe Ser Asn Glu Ile Arg Val Arg Asn Ile Ser Ile Asn Lys Glu Ser
               180                 185                 190
   tct tat gag tgc taa                                                    591
   Ser Tyr Glu Cys
           195
<210> 16
   <211> 196
   <212> PRT
   <213> Ehrlichia chaffeensis
<400> 16
   Met Ile Tyr Lys Glu Lys Leu Thr Arg Val Gly Glu Tyr Ile Leu Ala
   1               5                   10                  15
   Tyr Leu Ser Phe Ile Leu Ser Thr Tyr Ile Phe Leu Val Leu Val Asn
               20                  25                  30
   Ile Ile Arg Tyr Asn Ser Leu Ala Ile Cys Val Ile Ser Leu Leu Arg
           35                  40                  45
   Thr Asn Ile Phe Asn Val Ser Thr Lys Lys Leu Ile Lys Asp Lys Cys
       50                  55                  60
   Arg Asp Thr Lys Phe Ser Asn Met Lys Cys Tyr Leu Tyr Gly Lys Pro
   65                  70                  75                  80
   Leu Asn Leu Gln Ile Phe Tyr Gly Ile Phe Ser Phe Ile Arg Asn Phe
                   85                  90                  95
   Gln Asn Asn Thr Leu Ile Ile Pro Asn Asp Ser Lys Cys Gly Phe Tyr
               100                 105                 110
   Thr Thr Leu Trp Asp Asn Pro Ala Leu His Tyr Thr Tyr Thr Leu Thr
           115                 120                 125
   Gly Ser Glu Tyr Arg Asn Phe Phe Asp Ile Leu Tyr Glu Asn Ile Ile
       130                 135                 140
   Cys Gln Cys Lys Leu Leu Ile Asn Tyr Asn Arg Ser Val Leu Asn Gln
   145                 150                 155                 160
   His Asn Lys Asn Thr Leu Val Ile Ile Pro Ile Pro Asn Ala Arg Glu
   165 170 175
   Phe Ser Asn Glu Ile Arg Val Arg Asn Ile Ser Ile Asn Lys Glu Ser
   180 185 190
   Ser Tyr Glu Cys
   195
<210> 17
   <211> 493
   <212> DNA
   <213> Ehrlichia chaffeensis
<220>
   <221> CDS
   <222> (1)..(492)
<400> 17
   atg aat aaa aaa aac aag ttt att ata gct aca gca ttg gta tat tta 48
   Met Asn Lys Lys Asn Lys Phe Ile Ile Ala Thr Ala Leu Val Tyr Leu
   1 5 10 15
   ctg tca tta cct agt gta tcg ttt tca gag gtt aca aac agc agt att 96
   Leu Ser Leu Pro Ser Val Ser Phe Ser Glu Val Thr Asn Ser Ser Ile
   20 25 30
   aaa aaa cac tct ggg tta tat att agt gga caa tac aaa cca agt gtt 144
   Lys Lys His Ser Gly Leu Tyr Ile Ser Gly Gln Tyr Lys Pro Ser Val
   35 40 45
   tct gtt ttt agt agt ttc tca att aaa gaa act aac act atc aca aaa 192
   Ser Val Phe Ser Ser Phe Ser Ile Lys Glu Thr Asn Thr Ile Thr Lys
   50 55 60
   aat ctt ata gcg tta aaa aaa gat att aac tct ctt gaa gtt aac gcc 240
   Asn Leu Ile Ala Leu Lys Lys Asp Ile Asn Ser Leu Glu Val Asn Ala
   65 70 75 80
   gat gct agt caa ggt att agt cat cca gga aat ttt act ata cct tat 288
   Asp Ala Ser Gln Gly Ile Ser His Pro Gly Asn Phe Thr Ile Pro Tyr
   85 90 95
   ata gca gca ttt gaa gat aat gct ttt aat ttc aac ggt gct att ggt       336
   Ile Ala Ala Phe Glu Asp Asn Ala Phe Asn Phe Asn Gly Ala Ile Gly
               100                 105                 110
   tac att act gaa ggt cta agg att gaa ata gaa ggt tcc tat gaa gaa       384
   Tyr Ile Thr Glu Gly Leu Arg Ile Glu Ile Glu Gly Ser Tyr Glu Glu
           115                 120                 125
   ttt gat gct aaa aac cct gga ggt tat ggt cta aat gat gcc ttt cgg       432
   Phe Asp Ala Lys Asn Pro Gly Gly Tyr Gly Leu Asn Asp Ala Phe Arg
       130                 135                 140
   tac ttt gct tta gca cgt gat atg gaa agc aac aag ttc caa cca aaa       480
   Tyr Phe Ala Leu Ala Arg Asp Met Glu Ser Asn Lys Phe Gln Pro Lys
   145                 150                 155                 160
   gca caa agc tca c                                                     493
   Ala Gln Ser Ser
<210> 18
   <211> 164
   <212> PRT
   <213> Ehrlichia chaffeensis
<400> 18
   Met Asn Lys Lys Asn Lys Phe Ile Ile Ala Thr Ala Leu Val Tyr Leu
   1 5 10 15
   Leu Ser Leu Pro Ser Val Ser Phe Ser Glu Val Thr Asn Ser Ser Ile
   20 25 30
   Lys Lys His Ser Gly Leu Tyr Ile Ser Gly Gln Tyr Lys Pro Ser Val
   35 40 45
   Ser Val Phe Ser Ser Phe Ser Ile Lys Glu Thr Asn Thr Ile Thr Lys
   50 55 60
   Asn Leu Ile Ala Leu Lys Lys Asp Ile Asn Ser Leu Glu Val Asn Ala
   65 70 75 80
   Asp Ala Ser Gln Gly Ile Ser His Pro Gly Asn Phe Thr Ile Pro Tyr
   85 90 95
   Ile Ala Ala Phe Glu Asp Asn Ala Phe Asn Phe Asn Gly Ala Ile Gly
   100 105 110
   Tyr Ile Thr Glu Gly Leu Arg Ile Glu Ile Glu Gly Ser Tyr Glu Glu
   115 120 125
   Phe Asp Ala Lys Asn Pro Gly Gly Tyr Gly Leu Asn Asp Ala Phe Arg
   130 135 140
   Tyr Phe Ala Leu Ala Arg Asp Met Glu Ser Asn Lys Phe Gln Pro Lys
   145 150 155 160
   Ala Gln Ser Ser
<210> 19
   <211> 396
   <212> DNA
   <213> Ehrlichia chaffeensis
<220>
   <221> CDS
   <222> (1)..(396)
<400> 19
   tct aga ata cat gat gaa aat tat gct att aca aca aat aat aaa tta        48
   Ser Arg Ile His Asp Glu Asn Tyr Ala Ile Thr Thr Asn Asn Lys Leu
   1               5                   10                  15
   tcc atc gca tct att atg gtt aac acc tgc tat gat att tca att aat        96
   Ser Ile Ala Ser Ile Met Val Asn Thr Cys Tyr Asp Ile Ser Ile Asn
               20                  25                  30
   aat aca tca ata gta ccg tat tta tgc aca ggc att ggt gaa gat ctt       144
   Asn Thr Ser Ile Val Pro Tyr Leu Cys Thr Gly Ile Gly Glu Asp Leu
           35                  40                  45
   gta ggg ctt ttt aat aca ata cat ttt aaa ctt gca tat caa ggg aaa       192
   Val Gly Leu Phe Asn Thr Ile His Phe Lys Leu Ala Tyr Gln Gly Lys
       50                  55                  60
   gtt gga atg agt tat ttg ata aat aac aat atc cta tta ttt tct gac       240
   Val Gly Met Ser Tyr Leu Ile Asn Asn Asn Ile Leu Leu Phe Ser Asp
   65                  70                  75                  80
   ata tat tat cat aaa gtc atg ggt aac aga ttt aaa aat ttg tac atg       288
   Ile Tyr Tyr His Lys Val Met Gly Asn Arg Phe Lys Asn Leu Tyr Met
                   85                  90                  95
   caa tat gta gct gat cct aat att tct gaa gaa act ata cct ata tta       336
   Gln Tyr Val Ala Asp Pro Asn Ile Ser Glu Glu Thr Ile Pro Ile Leu
               100                 105                 110
   gca aaa ctt gat att ggt tat ttt gga agt gaa att gga ata agg ttt       384
   Ala Lys Leu Asp Ile Gly Tyr Phe Gly Ser Glu Ile Gly Ile Arg Phe
           115                 120                 125
   atg ttt aac taa                                                       396
   Met Phe Asn
       130
<210> 20
   <211> 131
   <212> PRT
   <213> Ehrlichia chaffeensis
<400> 20
   Ser Arg Ile His Asp Glu Asn Tyr Ala Ile Thr Thr Asn Asn Lys Leu
   1               5                   10                  15
   Ser Ile Ala Ser Ile Met Val Asn Thr Cys Tyr Asp Ile Ser Ile Asn
               20                  25                  30
   Asn Thr Ser Ile Val Pro Tyr Leu Cys Thr Gly Ile Gly Glu Asp Leu
           35                  40                  45
   Val Gly Leu Phe Asn Thr Ile His Phe Lys Leu Ala Tyr Gln Gly Lys
       50                  55                  60
   Val Gly Met Ser Tyr Leu Ile Asn Asn Asn Ile Leu Leu Phe Ser Asp
   65                  70                  75                  80
   Ile Tyr Tyr His Lys Val Met Gly Asn Arg Phe Lys Asn Leu Tyr Met
                   85                  90                  95
   Gln Tyr Val Ala Asp Pro Asn Ile Ser Glu Glu Thr Ile Pro Ile Leu
               100                 105                 110
   Ala Lys Leu Asp Ile Gly Tyr Phe Gly Ser Glu Ile Gly Ile Arg Phe
           115                 120                 125
   Met Phe Asn
       130
<210> 21
   <211> 888
   <212> DNA
   <213> Ehrlichia chaffeensis
<220>
   <221> CDS
   <222> (1)..(888)
<400> 21
   atg aca aag aaa ttt aat ttt gta aat gtt ata tta aca ttt ttg tta      48
   Met Thr Lys Lys Phe Asn Phe Val Asn Val Ile Leu Thr Phe Leu Leu
   1               5                   10                  15
   ttt ctt ttc cca ctt aag tca ttt aca aca tat gca aat aat aac aca      96
   Phe Leu Phe Pro Leu Lys Ser Phe Thr Thr Tyr Ala Asn Asn Asn Thr
               20                  25                  30
   atc act caa aaa gtt gga ttg tac ata agt ggt caa tat aag cca agt     144
   Ile Thr Gln Lys Val Gly Leu Tyr Ile Ser Gly Gln Tyr Lys Pro Ser
           35                  40                  45
   att cct cat ttc aag aat ttt tca gta gaa gaa aat gac aaa gta gta     192
   Ile Pro His Phe Lys Asn Phe Ser Val Glu Glu Asn Asp Lys Val Val
       50                  55                  60
   gat ttg ata ggt ctt aca act gat gtt aca tat atc aca gaa cat ata      240
   Asp Leu Ile Gly Leu Thr Thr Asp Val Thr Tyr Ile Thr Glu His Ile
   65                  70                  75                  80
   tta cga gat aat aca aaa ttc aac act cat tat att gca aag ttc aag      288
   Leu Arg Asp Asn Thr Lys Phe Asn Thr His Tyr Ile Ala Lys Phe Lys
                   85                  90                  95
   aac aat ttt ata aat ttc agc agt gca att ggt tat tat tct ggg caa      336
   Asn Asn Phe Ile Asn Phe Ser Ser Ala Ile Gly Tyr Tyr Ser Gly Gln
               100                 105                 110
   gaa cca agg tta gaa ata gaa agc tct tat ggg gat ttt gat gtt gta      384
   Glu Pro Arg Leu Glu Ile Glu Ser Ser Tyr Gly Asp Phe Asp Val Val
           115                 120                 125
   aat tat aaa aat tat gca gta caa gat gtt aat aga tat ttt gct tta      432
   Asn Tyr Lys Asn Tyr Ala Val Gln Asp Val Asn Arg Tyr Phe Ala Leu
       130                 135                 140
   gta cgt gaa aaa aat ggt tca aat ttc tct cca aaa cca cat gaa act      480
   Val Arg Glu Lys Asn Gly Ser Asn Phe Ser Pro Lys Pro His Glu Thr
   145                 150                 155                 160
   agt caa ccc tct gac agt aat cct aaa aag tct ttt tat act tta atg      528
   Ser Gln Pro Ser Asp Ser Asn Pro Lys Lys Ser Phe Tyr Thr Leu Met
                   165                 170                 175
   aag aat aat ggg gta ttt gtt gca tca gta ata atc aac ggt tgt tat      576
   Lys Asn Asn Gly Val Phe Val Ala Ser Val Ile Ile Asn Gly Cys Tyr
               180                 185                 190
   gat ttt tct ttt aat aac aca aca ata tca cct tac gta tgt ata gga      624
   Asp Phe Ser Phe Asn Asn Thr Thr Ile Ser Pro Tyr Val Cys Ile Gly
           195                 200                 205
   gtt gga gga gat ttt ata gag ttt ttt gaa gta atg cat atc aag ttt      672
   Val Gly Gly Asp Phe Ile Glu Phe Phe Glu Val Met His Ile Lys Phe
       210                 215                 220
   gct tgc caa agt aag gtt ggt att agc tat cca ata tct ccc tct att      720
   Ala Cys Gln Ser Lys Val Gly Ile Ser Tyr Pro Ile Ser Pro Ser Ile
   225                 230                 235                 240
   act att ttt gct gat gca cat tat cac aag gtc ata aat aat aaa ttt        768
   Thr Ile Phe Ala Asp Ala His Tyr His Lys Val Ile Asn Asn Lys Phe
                   245                 250                 255
   aac aac cta cat gtt aag tat tca tat gaa ctt aaa aac tca cct acc        816
   Asn Asn Leu His Val Lys Tyr Ser Tyr Glu Leu Lys Asn Ser Pro Thr
               260                 265                 270
   att acc tct gca aca gcc aaa cta aac att gaa tat ttt ggt ggt gaa        864
   Ile Thr Ser Ala Thr Ala Lys Leu Asn Ile Glu Tyr Phe Gly Gly Glu
           275                 280                 285
   gtt ggg atg aga ttt ata ttt taa                                        888
   Val Gly Met Arg Phe Ile Phe
       290                 295
<210> 22
   <211> 295
   <212> PRT
   <213> Ehrlichia chaffeensis
<400> 22
   Met Thr Lys Lys Phe Asn Phe Val Asn Val Ile Leu Thr Phe Leu Leu
   1               5                   10                  15
   Phe Leu Phe Pro Leu Lys Ser Phe Thr Thr Tyr Ala Asn Asn Asn Thr
               20                  25                  30
   Ile Thr Gln Lys Val Gly Leu Tyr Ile Ser Gly Gln Tyr Lys Pro Ser
           35                  40                  45
   Ile Pro His Phe Lys Asn Phe Ser Val Glu Glu Asn Asp Lys Val Val
       50                  55                  60
   Asp Leu Ile Gly Leu Thr Thr Asp Val Thr Tyr Ile Thr Glu His Ile
   65                  70                  75                  80
   Leu Arg Asp Asn Thr Lys Phe Asn Thr His Tyr Ile Ala Lys Phe Lys
                   85                  90                  95
   Asn Asn Phe Ile Asn Phe Ser Ser Ala Ile Gly Tyr Tyr Ser Gly Gln
   100 105 110
   Glu Pro Arg Leu Glu Ile Glu Ser Ser Tyr Gly Asp Phe Asp Val Val
   115 120 125
   Asn Tyr Lys Asn Tyr Ala Val Gln Asp Val Asn Arg Tyr Phe Ala Leu
   130 135 140
   Val Arg Glu Lys Asn Gly Ser Asn Phe Ser Pro Lys Pro His Glu Thr
   145 150 155 160
   Ser Gln Pro Ser Asp Ser Asn Pro Lys Lys Ser Phe Tyr Thr Leu Met
   165 170 175
   Lys Asn Asn Gly Val Phe Val Ala Ser Val Ile Ile Asn Gly Cys Tyr
   180 185 190
   Asp Phe Ser Phe Asn Asn Thr Thr Ile Ser Pro Tyr Val Cys Ile Gly
   195 200 205
   Val Gly Gly Asp Phe Ile Glu Phe Phe Glu Val Met His Ile Lys Phe
   210 215 220
   Ala Cys Gln Ser Lys Val Gly Ile Ser Tyr Pro Ile Ser Pro Ser Ile
   225 230 235 240
   Thr Ile Phe Ala Asp Ala His Tyr His Lys Val Ile Asn Asn Lys Phe
   245 250 255
   Asn Asn Leu His Val Lys Tyr Ser Tyr Glu Leu Lys Asn Ser Pro Thr
   260 265 270
   Ile Thr Ser Ala Thr Ala Lys Leu Asn Ile Glu Tyr Phe Gly Gly Glu
   275 280 285
   Val Gly Met Arg Phe Ile Phe
   290 295
<210> 23
   <211> 840
   <212> DNA
   <213> Ehrlichia chaffeensis
<220>
   <221> CDS
   <222> (1)..(840)
<400> 23
   atg agc aaa aaa aag ttt att aca ata gga aca gta ctt gca tct cta      48
   Met Ser Lys Lys Lys Phe Ile Thr Ile Gly Thr Val Leu Ala Ser Leu
   1               5                   10                  15
   tta tca ttc tta tct att gaa tcc ttt tca gct ata aat cat aat cat      96
   Leu Ser Phe Leu Ser Ile Glu Ser Phe Ser Ala Ile Asn His Asn His
               20                  25                  30
   aca gga aat aac act agt ggt ata tat att aca ggg cag tat aga cca     144
   Thr Gly Asn Asn Thr Ser Gly Ile Tyr Ile Thr Gly Gln Tyr Arg Pro
           35                  40                  45
   gga gta tcc cat ttt agc aat ttc tca gta aaa gaa act aat gtt gat     192
   Gly Val Ser His Phe Ser Asn Phe Ser Val Lys Glu Thr Asn Val Asp
       50                  55                  60
   aca ata caa cta gta gga tat aaa aaa agt gcg tct tct atc gat cct     240
   Thr Ile Gln Leu Val Gly Tyr Lys Lys Ser Ala Ser Ser Ile Asp Pro
   65                  70                  75                  80
   aac act tat tca aac ttt caa ggt cca tat act gtt aca ttt caa gat     288
   Asn Thr Tyr Ser Asn Phe Gln Gly Pro Tyr Thr Val Thr Phe Gln Asp
                   85                  90                  95
   aat gct gct agt ttc agt gga gca att gga tat tct tac ccc gaa agt     336
   Asn Ala Ala Ser Phe Ser Gly Ala Ile Gly Tyr Ser Tyr Pro Glu Ser
               100                 105                 110
   cta aga ctt gaa ctt gaa ggt tct tac gaa aaa ttt gat gtc aaa gat     384
   Leu Arg Leu Glu Leu Glu Gly Ser Tyr Glu Lys Phe Asp Val Lys Asp
           115                 120                 125
   cct aaa gac tac tca gca aaa gat gct ttt agg ttt ttt gct cta gca      432
   Pro Lys Asp Tyr Ser Ala Lys Asp Ala Phe Arg Phe Phe Ala Leu Ala
       130                 135                 140
   cgt aat acg tct act act gtt cct gat gct caa aaa tat aca gtt atg      480
   Arg Asn Thr Ser Thr Thr Val Pro Asp Ala Gln Lys Tyr Thr Val Met
   145                 150                 155                 160
   aag aat aat ggc tta tct gtt gca tca atc atg atc aat ggt tgt tat      528
   Lys Asn Asn Gly Leu Ser Val Ala Ser Ile Met Ile Asn Gly Cys Tyr
                   165                 170                 175
   gat cta tct ttt aat aat tta gtc gta tca cct tat ata tgt gca ggt      576
   Asp Leu Ser Phe Asn Asn Leu Val Val Ser Pro Tyr Ile Cys Ala Gly
               180                 185                 190
   aat ggt gaa gat ttc att gaa ttt ttt gat act ttg cac att aaa ctt      624
   Asn Gly Glu Asp Phe Ile Glu Phe Phe Asp Thr Leu His Ile Lys Leu
           195                 200                 205
   gct tat caa gga aaa cta ggt att agt tat tac ttc ttt cct aag att      672
   Ala Tyr Gln Gly Lys Leu Gly Ile Ser Tyr Tyr Phe Phe Pro Lys Ile
       210                 215                 220
   aat gta ttt gct ggt ggg tac tat cat aga gtt ata ggg aat aaa ttt      720
   Asn Val Phe Ala Gly Gly Tyr Tyr His Arg Val Ile Gly Asn Lys Phe
   225                 230                 235                 240
   aaa aat tta aat gtt aac cat gtt gtt aca ctt gat gaa ttt cct aaa      768
   Lys Asn Leu Asn Val Asn His Val Val Thr Leu Asp Glu Phe Pro Lys
                   245                 250                 255
   gca act tct gca gta gct aca ctt ata gtt gct tat ttt ggt ggt gaa      816
   Ala Thr Ser Ala Val Ala Thr Leu Ile Val Ala Tyr Phe Gly Gly Glu
               260                 265                 270
   gct gga gta aag ttt aca ttt taa                                      840
   Ala Gly Val Lys Phe Thr Phe
           275
<210> 24
   <211> 279
   <212> PRT
   <213> Ehrlichia chaffeensis
<400> 24
   Met Ser Lys Lys Lys Phe Ile Thr Ile Gly Thr Val Leu Ala Ser Leu
   1 5 10 15
   Leu Ser Phe Leu Ser Ile Glu Ser Phe Ser Ala Ile Asn His Asn His
   20 25 30
   Thr Gly Asn Asn Thr Ser Gly Ile Tyr Ile Thr Gly Gln Tyr Arg Pro
   35 40 45
   Gly Val Ser His Phe Ser Asn Phe Ser Val Lys Glu Thr Asn Val Asp
   50 55 60
   Thr Ile Gln Leu Val Gly Tyr Lys Lys Ser Ala Ser Ser Ile Asp Pro
   65 70 75 80
   Asn Thr Tyr Ser Asn Phe Gln Gly Pro Tyr Thr Val Thr Phe Gln Asp
   85 90 95
   Asn Ala Ala Ser Phe Ser Gly Ala Ile Gly Tyr Ser Tyr Pro Glu Ser
   100 105 110
   Leu Arg Leu Glu Leu Glu Gly Ser Tyr Glu Lys Phe Asp Val Lys Asp
   115 120 125
   Pro Lys Asp Tyr Ser Ala Lys Asp Ala Phe Arg Phe Phe Ala Leu Ala
   130 135 140
   Arg Asn Thr Ser Thr Thr Val Pro Asp Ala Gln Lys Tyr Thr Val Met
   145 150 155 160
   Lys Asn Asn Gly Leu Ser Val Ala Ser Ile Met Ile Asn Gly Cys Tyr
   165 170 175
   Asp Leu Ser Phe Asn Asn Leu Val Val Ser Pro Tyr Ile Cys Ala Gly
   180 185 190
   Asn Gly Glu Asp Phe Ile Glu Phe Phe Asp Thr Leu His Ile Lys Leu
   195 200 205
   Ala Tyr Gln Gly Lys Leu Gly Ile Ser Tyr Tyr Phe Phe Pro Lys Ile
   210 215 220
   Asn Val Phe Ala Gly Gly Tyr Tyr His Arg Val Ile Gly Asn Lys Phe
   225 230 235 240
   Lys Asn Leu Asn Val Asn His Val Val Thr Leu Asp Glu Phe Pro Lys
   245 250 255
   Ala Thr Ser Ala Val Ala Thr Leu Ile Val Ala Tyr Phe Gly Gly Glu
   260 265 270
   Ala Gly Val Lys Phe Thr Phe
   275
<210> 25
   <211> 852
   <212> DNA
   <213> Ehrlichia chaffeensis
<220>
   <221> CDS
   <222> (1)..(852)
<400> 25
   atg agt gct aaa aaa aag ctt ttt ata ata ggg tca gtg tta gta tgt 48
   Met Ser Ala Lys Lys Lys Leu Phe Ile Ile Gly Ser Val Leu Val Cys
   1 5 10 15
   tta gtg tca tac tta cct act aaa tct ttg tca aac tta aat aat att        96
   Leu Val Ser Tyr Leu Pro Thr Lys Ser Leu Ser Asn Leu Asn Asn Ile
               20                  25                  30
   aat aat aac act aag tgc act ggg cta tat gtc agt gga caa tat aaa       144
   Asn Asn Asn Thr Lys Cys Thr Gly Leu Tyr Val Ser Gly Gln Tyr Lys
           35                  40                  45
   cct act gtt tct cac ttt agt aat ttt tca ctt aaa gaa act tat act       192
   Pro Thr Val Ser His Phe Ser Asn Phe Ser Leu Lys Glu Thr Tyr Thr
       50                  55                  60
   gac act aaa gag tta tta gga cta gca aaa gat att aag tct att aca       240
   Asp Thr Lys Glu Leu Leu Gly Leu Ala Lys Asp Ile Lys Ser Ile Thr
   65                  70                  75                  80
   gat ata aca aca aat aaa aaa ttc aac att cct tat aac aca aaa ttt       288
   Asp Ile Thr Thr Asn Lys Lys Phe Asn Ile Pro Tyr Asn Thr Lys Phe
                   85                  90                  95
   caa gat aat gct gtt agc ttc agt gca gct gtt gga tat att tcc caa       336
   Gln Asp Asn Ala Val Ser Phe Ser Ala Ala Val Gly Tyr Ile Ser Gln
               100                 105                 110
   gac agt cca agg gtt gag gta gaa tgg tct tat gaa gaa ttt gac gtt       384
   Asp Ser Pro Arg Val Glu Val Glu Trp Ser Tyr Glu Glu Phe Asp Val
           115                 120                 125
   aaa aat cct ggt aat tac gta gta agt gaa gcc ttc agg tat att gct       432
   Lys Asn Pro Gly Asn Tyr Val Val Ser Glu Ala Phe Arg Tyr Ile Ala
       130                 135                 140
   tta gca aga gga att gat aat ctt caa aaa tat cct gaa aca aat aag       480
   Leu Ala Arg Gly Ile Asp Asn Leu Gln Lys Tyr Pro Glu Thr Asn Lys
   145                 150                 155                 160
   tat gtt gtt ata aag aac aat ggc tta tct gtc gca tcc att ata atc       528
   Tyr Val Val Ile Lys Asn Asn Gly Leu Ser Val Ala Ser Ile Ile Ile
                   165                 170                 175
   aat ggc tgt tat gat ttt tct tta aac aat tta aaa gta tca cct tac       576
   Asn Gly Cys Tyr Asp Phe Ser Leu Asn Asn Leu Lys Val Ser Pro Tyr
               180                 185                 190
   ata tgc gta ggg ttt ggt ggg gac att ata gaa ttt ttt agt gct gta 624
   Ile Cys Val Gly Phe Gly Gly Asp Ile Ile Glu Phe Phe Ser Ala Val
   195 200 205
   agt ttt aaa ttt gct tat caa ggt aag gta ggt atc agt tat cca tta 672
   Ser Phe Lys Phe Ala Tyr Gln Gly Lys Val Gly Ile Ser Tyr Pro Leu
   210 215 220
   ttc tct aat atg att ata ttt gct gac gga tat tac cat aag gtc ata 720
   Phe Ser Asn Met Ile Ile Phe Ala Asp Gly Tyr Tyr His Lys Val Ile
   225 230 235 240
   gga aat aaa ttt aac aat tta aat gtt caa cac gtt gtt agt ctt aac 768
   Gly Asn Lys Phe Asn Asn Leu Asn Val Gln His Val Val Ser Leu Asn
   245 250 255
   agt cat cct aag tct act ttt gca gta gct act ctt aat gtt gag tat 816
   Ser His Pro Lys Ser Thr Phe Ala Val Ala Thr Leu Asn Val Glu Tyr
   260 265 270
   ttc ggt agt gaa ttt ggg tta aaa ttt ata ttt taa 852
   Phe Gly Ser Glu Phe Gly Leu Lys Phe Ile Phe
   275 280
<210> 26
   <211> 283
   <212> PRT
   <213> Ehrlichia chaffeensis
<400> 26
   Met Ser Ala Lys Lys Lys Leu Phe Ile Ile Gly Ser Val Leu Val Cys
   1 5 10 15
   Leu Val Ser Tyr Leu Pro Thr Lys Ser Leu Ser Asn Leu Asn Asn Ile
   20 25 30
   Asn Asn Asn Thr Lys Cys Thr Gly Leu Tyr Val Ser Gly Gln Tyr Lys
   35 40 45
   Pro Thr Val Ser His Phe Ser Asn Phe Ser Leu Lys Glu Thr Tyr Thr
   50 55 60
   Asp Thr Lys Glu Leu Leu Gly Leu Ala Lys Asp Ile Lys Ser Ile Thr
   65 70 75 80
   Asp Ile Thr Thr Asn Lys Lys Phe Asn Ile Pro Tyr Asn Thr Lys Phe
   85 90 95
   Gln Asp Asn Ala Val Ser Phe Ser Ala Ala Val Gly Tyr Ile Ser Gln
   100 105 110
   Asp Ser Pro Arg Val Glu Val Glu Trp Ser Tyr Glu Glu Phe Asp Val
   115 120 125
   Lys Asn Pro Gly Asn Tyr Val Val Ser Glu Ala Phe Arg Tyr Ile Ala
   130 135 140
   Leu Ala Arg Gly Ile Asp Asn Leu Gln Lys Tyr Pro Glu Thr Asn Lys
   145 150 155 160
   Tyr Val Val Ile Lys Asn Asn Gly Leu Ser Val Ala Ser Ile Ile Ile
   165 170 175
   Asn Gly Cys Tyr Asp Phe Ser Leu Asn Asn Leu Lys Val Ser Pro Tyr
   180 185 190
   Ile Cys Val Gly Phe Gly Gly Asp Ile Ile Glu Phe Phe Ser Ala Val
   195 200 205
   Ser Phe Lys Phe Ala Tyr Gln Gly Lys Val Gly Ile Ser Tyr Pro Leu
   210 215 220
   Phe Ser Asn Met Ile Ile Phe Ala Asp Gly Tyr Tyr His Lys Val Ile
   225 230 235 240
   Gly Asn Lys Phe Asn Asn Leu Asn Val Gln His Val Val Ser Leu Asn
   245 250 255
   Ser His Pro Lys Ser Thr Phe Ala Val Ala Thr Leu Asn Val Glu Tyr
   260 265 270
   Phe Gly Ser Glu Phe Gly Leu Lys Phe Ile Phe
   275 280
<210> 27
   <211> 828
   <212> DNA
   <213> Ehrlichia chaffeensis
<220>
   <221> CDS
   <222> (1)..(828)
<400> 27
   atg agt aaa aaa aat ttt att aca ata gga gca aca ctt att cat atg 48
   Met Ser Lys Lys Asn Phe Ile Thr Ile Gly Ala Thr Leu Ile His Met
   1 5 10 15
   ttg tta cct aac ata tct ttt cca gaa act att aac aat aac act gat 96
   Leu Leu Pro Asn Ile Ser Phe Pro Glu Thr Ile Asn Asn Asn Thr Asp
   20 25 30
   aaa ctt tct ggg tta tat ata agt ggg caa tat aaa cca ggg att tct 144
   Lys Leu Ser Gly Leu Tyr Ile Ser Gly Gln Tyr Lys Pro Gly Ile Ser
   35 40 45
   cat ttc agc aaa ttt tca gtc aaa gaa atc tat aat gat aac att caa 192
   His Phe Ser Lys Phe Ser Val Lys Glu Ile Tyr Asn Asp Asn Ile Gln
   50 55 60
   cta att ggg tta aga cac aac gca att tct act agt acc ctt aat att 240
   Leu Ile Gly Leu Arg His Asn Ala Ile Ser Thr Ser Thr Leu Asn Ile
   65 70 75 80
   aat aca gat ttt aat atc ccc tat aaa gta aca ttt caa aat aac att 288
   Asn Thr Asp Phe Asn Ile Pro Tyr Lys Val Thr Phe Gln Asn Asn Ile
   85 90 95
   acc agc ttt agt gga gct att ggt tat tct gat ccc aca ggg gca aga 336
   Thr Ser Phe Ser Gly Ala Ile Gly Tyr Ser Asp Pro Thr Gly Ala Arg
   100 105 110
   ttt gag ctt gaa ggt tct tat gaa gaa ttt gat gtg aca gat cct gga      384
   Phe Glu Leu Glu Gly Ser Tyr Glu Glu Phe Asp Val Thr Asp Pro Gly
           115                 120                 125
   gac tgc tta ata aaa gat acc tat aga tat ttc gct tta gct aga aac      432
   Asp Cys Leu Ile Lys Asp Thr Tyr Arg Tyr Phe Ala Leu Ala Arg Asn
       130                 135                 140
   cca tca ggt tct agc cct acc tca aac aac tat act gtt atg aga aat      480
   Pro Ser Gly Ser Ser Pro Thr Ser Asn Asn Tyr Thr Val Met Arg Asn
   145                 150                 155                 160
   gat ggt gtt tcc att act tct gtt ata ttt aat ggc tgt tat gac atc      528
   Asp Gly Val Ser Ile Thr Ser Val Ile Phe Asn Gly Cys Tyr Asp Ile
                   165                 170                 175
   ttt tta aag gat tta gaa gta tca cct tat gta tgt gtt ggt gta ggt      576
   Phe Leu Lys Asp Leu Glu Val Ser Pro Tyr Val Cys Val Gly Val Gly
               180                 185                 190
   gga gat ttt ata gaa ttt ttt gac gca tta cac att aaa tta gca tac      624
   Gly Asp Phe Ile Glu Phe Phe Asp Ala Leu His Ile Lys Leu Ala Tyr
           195                 200                 205
   caa ggc aag tta ggt atc aat tat cac tta tcg act caa gca agc gta      672
   Gln Gly Lys Leu Gly Ile Asn Tyr His Leu Ser Thr Gln Ala Ser Val
       210                 215                 220
   ttt att gat gga tat tat cat aag gtt ata gga aat caa ttc aac aat      720
   Phe Ile Asp Gly Tyr Tyr His Lys Val Ile Gly Asn Gln Phe Asn Asn
   225                 230                 235                 240
   cta aat gtt caa cac gtg gct agt aca gat ttt gga cct gta tac gca      768
   Leu Asn Val Gln His Val Ala Ser Thr Asp Phe Gly Pro Val Tyr Ala
                   245                 250                 255
   gta gcc aca ctt aac att ggt tat ttt ggt ggt gaa atc gga att aga      816
   Val Ala Thr Leu Asn Ile Gly Tyr Phe Gly Gly Glu Ile Gly Ile Arg
               260                 265                 270
   ctt aca ttt taa                                                      828
   Leu Thr Phe
           275
<210> 28
   <211> 275
   <212> PRT
   <213> Ehrlichia chaffeensis
<400> 28
   Met Ser Lys Lys Asn Phe Ile Thr Ile Gly Ala Thr Leu Ile His Met
   1               5                   10                  15
   Leu Leu Pro Asn Ile Ser Phe Pro Glu Thr Ile Asn Asn Asn Thr Asp
               20                  25                  30
   Lys Leu Ser Gly Leu Tyr Ile Ser Gly Gln Tyr Lys Pro Gly Ile Ser
           35                  40                  45
   His Phe Ser Lys Phe Ser Val Lys Glu Ile Tyr Asn Asp Asn Ile Gln
       50                  55                  60
   Leu Ile Gly Leu Arg His Asn Ala Ile Ser Thr Ser Thr Leu Asn Ile
   65                  70                  75                  80
   Asn Thr Asp Phe Asn Ile Pro Tyr Lys Val Thr Phe Gln Asn Asn Ile
                   85                  90                  95
   Thr Ser Phe Ser Gly Ala Ile Gly Tyr Ser Asp Pro Thr Gly Ala Arg
               100                 105                 110
   Phe Glu Leu Glu Gly Ser Tyr Glu Glu Phe Asp Val Thr Asp Pro Gly
           115                 120                 125
   Asp Cys Leu Ile Lys Asp Thr Tyr Arg Tyr Phe Ala Leu Ala Arg Asn
       130                 135                 140
   Pro Ser Gly Ser Ser Pro Thr Ser Asn Asn Tyr Thr Val Met Arg Asn
   145                 150                 155                 160
   Asp Gly Val Ser Ile Thr Ser Val Ile Phe Asn Gly Cys Tyr Asp Ile
                   165                 170                 175
   Phe Leu Lys Asp Leu Glu Val Ser Pro Tyr Val Cys Val Gly Val Gly
               180                 185                 190
   Gly Asp Phe Ile Glu Phe Phe Asp Ala Leu His Ile Lys Leu Ala Tyr
           195                 200                 205
   Gln Gly Lys Leu Gly Ile Asn Tyr His Leu Ser Thr Gln Ala Ser Val
       210                 215                 220
   Phe Ile Asp Gly Tyr Tyr His Lys Val Ile Gly Asn Gln Phe Asn Asn
   225                 230                 235                 240
   Leu Asn Val Gln His Val Ala Ser Thr Asp Phe Gly Pro Val Tyr Ala
                   245                 250                 255
   Val Ala Thr Leu Asn Ile Gly Tyr Phe Gly Gly Glu Ile Gly Ile Arg
               260                 265                 270
   Leu Thr Phe
           275
<210> 29
   <211> 858
   <212> DNA
   <213> Ehrlichia chaffeensis
<220>
   <221> CDS
   <222> (1)..(858)
<400> 29
   atg aat aat aga aaa agt ttt ttt ata ata ggt gca tca tta cta gca      48
   Met Asn Asn Arg Lys Ser Phe Phe Ile Ile Gly Ala Ser Leu Leu Ala
   1               5                   10                  15
   agc tta tta ttc aca tct gag gcc tct tct aca gga aat gta agt aac        96
   Ser Leu Leu Phe Thr Ser Glu Ala Ser Ser Thr Gly Asn Val Ser Asn
               20                  25                  30
   cat act tat ttt aaa cct agg tta tat atc agt gga caa tat aga cca       144
   His Thr Tyr Phe Lys Pro Arg Leu Tyr Ile Ser Gly Gln Tyr Arg Pro
           35                  40                  45
   gga gtt tct cat ttt agc aaa ttt tca gtc aaa gaa acc aac tac aat       192
   Gly Val Ser His Phe Ser Lys Phe Ser Val Lys Glu Thr Asn Tyr Asn
       50                  55                  60
   act act caa cta gtt ggg ctt aaa aag gac atc agt gtc ata ggg aac       240
   Thr Thr Gln Leu Val Gly Leu Lys Lys Asp Ile Ser Val Ile Gly Asn
   65                  70                  75                  80
   agt aat atc aca acc tac aca aat ttc aac ttt cct tac att gca gaa       288
   Ser Asn Ile Thr Thr Tyr Thr Asn Phe Asn Phe Pro Tyr Ile Ala Glu
                   85                  90                  95
   ttt caa gac aat gcc ata agt ttc agt ggg gca att gga tac ttg tat       336
   Phe Gln Asp Asn Ala Ile Ser Phe Ser Gly Ala Ile Gly Tyr Leu Tyr
               100                 105                 110
   tcc gag aat ttt aga att gaa gta gag gct tct tat gaa gaa ttt gat       384
   Ser Glu Asn Phe Arg Ile Glu Val Glu Ala Ser Tyr Glu Glu Phe Asp
           115                 120                 125
   gtt aaa aat cca gaa gga tct gct aca gac gca tac agg tat ttt gca       432
   Val Lys Asn Pro Glu Gly Ser Ala Thr Asp Ala Tyr Arg Tyr Phe Ala
       130                 135                 140
   cta gca cgt gct atg gat ggc act aat aaa tct agt cct gat gac aca       480
   Leu Ala Arg Ala Met Asp Gly Thr Asn Lys Ser Ser Pro Asp Asp Thr
   145                 150                 155                 160
   aga aaa ttc act gtc atg aga aat gac ggg ttt tca att tca tca gta       528
   Arg Lys Phe Thr Val Met Arg Asn Asp Gly Phe Ser Ile Ser Ser Val
                   165                 170                 175
   atg ata aat ggg tgt tac aat ttt aca tta gat gat ata cca gta gta       576
   Met Ile Asn Gly Cys Tyr Asn Phe Thr Leu Asp Asp Ile Pro Val Val
               180                 185                 190
   ccg tat gta tgc gca gga ata gga gga gat ttc ata gag ttt ttt aat      624
   Pro Tyr Val Cys Ala Gly Ile Gly Gly Asp Phe Ile Glu Phe Phe Asn
           195                 200                 205
   gat tta cat gtt aag ttt cgt cat caa ggc aag gta ggt att agt tat      672
   Asp Leu His Val Lys Phe Arg His Gln Gly Lys Val Gly Ile Ser Tyr
       210                 215                 220
   tct ata tcc cct gaa gta agt tta ttt ctt aac gga tat tac cat aaa      720
   Ser Ile Ser Pro Glu Val Ser Leu Phe Leu Asn Gly Tyr Tyr His Lys
   225                 230                 235                 240
   gta aca ggt aac aga ttt aaa aac tta cac gtt caa cac gta agt gat      768
   Val Thr Gly Asn Arg Phe Lys Asn Leu His Val Gln His Val Ser Asp
                   245                 250                 255
   tta agt gac gct cct aag ttc aca tct gca gtt gct aca ctc aat gtt      816
   Leu Ser Asp Ala Pro Lys Phe Thr Ser Ala Val Ala Thr Leu Asn Val
               260                 265                 270
   ggg tac ttt ggt ggc gaa att gga gta aga ttt ata ttt taa              858
   Gly Tyr Phe Gly Gly Glu Ile Gly Val Arg Phe Ile Phe
           275                 280                 285
<210> 30
   <211> 285
   <212> PRT
   <213> Ehrlichia chaffeensis
<400> 30
   Met Asn Asn Arg Lys Ser Phe Phe Ile Ile Gly Ala Ser Leu Leu Ala
   1               5                   10                  15
   Ser Leu Leu Phe Thr Ser Glu Ala Ser Ser Thr Gly Asn Val Ser Asn
               20                  25                  30
   His Thr Tyr Phe Lys Pro Arg Leu Tyr Ile Ser Gly Gln Tyr Arg Pro
           35                  40                  45
   Gly Val Ser His Phe Ser Lys Phe Ser Val Lys Glu Thr Asn Tyr Asn
       50                  55                  60
   Thr Thr Gln Leu Val Gly Leu Lys Lys Asp Ile Ser Val Ile Gly Asn
   65 70 75 80
   Ser Asn Ile Thr Thr Tyr Thr Asn Phe Asn Phe Pro Tyr Ile Ala Glu
   85 90 95
   Phe Gln Asp Asn Ala Ile Ser Phe Ser Gly Ala Ile Gly Tyr Leu Tyr
   100 105 110
   Ser Glu Asn Phe Arg Ile Glu Val Glu Ala Ser Tyr Glu Glu Phe Asp
   115 120 125
   Val Lys Asn Pro Glu Gly Ser Ala Thr Asp Ala Tyr Arg Tyr Phe Ala
   130 135 140
   Leu Ala Arg Ala Met Asp Gly Thr Asn Lys Ser Ser Pro Asp Asp Thr
   145 150 155 160
   Arg Lys Phe Thr Val Met Arg Asn Asp Gly Phe Ser Ile Ser Ser Val
   165 170 175
   Met Ile Asn Gly Cys Tyr Asn Phe Thr Leu Asp Asp Ile Pro Val Val
   180 185 190
   Pro Tyr Val Cys Ala Gly Ile Gly Gly Asp Phe Ile Glu Phe Phe Asn
   195 200 205
   Asp Leu His Val Lys Phe Arg His Gln Gly Lys Val Gly Ile Ser Tyr
   210 215 220
   Ser Ile Ser Pro Glu Val Ser Leu Phe Leu Asn Gly Tyr Tyr His Lys
   225 230 235 240
   Val Thr Gly Asn Arg Phe Lys Asn Leu His Val Gln His Val Ser Asp
   245 250 255
   Leu Ser Asp Ala Pro Lys Phe Thr Ser Ala Val Ala Thr Leu Asn Val
               260                 265                 270
   Gly Tyr Phe Gly Gly Glu Ile Gly Val Arg Phe Ile Phe
           275                 280                 285
<210> 31
   <211> 867
   <212> DNA
   <213> Ehrlichia canis
<220>
   <221> CDS
   <222> (1)..(867)
<400> 31
   atg aat tgc aaa aga ttt ttc ata gca agt gca ttg ata tca cta atg       48
   Met Asn Cys Lys Arg Phe Phe Ile Ala Ser Ala Leu Ile Ser Leu Met
   1               5                   10                  15
   tct ttc tta cct agc gta tct ttt tct gaa tca ata cat gaa gat aat       96
   Ser Phe Leu Pro Ser Val Ser Phe Ser Glu Ser Ile His Glu Asp Asn
               20                  25                  30
   ata aat ggt aac ttt tac att agt gca aag tat atg cca agt gcc tca      144
   Ile Asn Gly Asn Phe Tyr Ile Ser Ala Lys Tyr Met Pro Ser Ala Ser
           35                  40                  45
   cac ttt ggc gta ttt tca gtt aaa gaa gag aaa aac aca aca act gga      192
   His Phe Gly Val Phe Ser Val Lys Glu Glu Lys Asn Thr Thr Thr Gly
       50                  55                  60
   gtt ttc gga tta aaa caa gat tgg gac gga gca aca ata aag gat gca      240
   Val Phe Gly Leu Lys Gln Asp Trp Asp Gly Ala Thr Ile Lys Asp Ala
   65                  70                  75                  80
   agc agc agc cac aca ata gac cca agt aca ata ttc tcc att tca aat      288
   Ser Ser Ser His Thr Ile Asp Pro Ser Thr Ile Phe Ser Ile Ser Asn
                   85                  90                  95
   tat tca ttt aaa tat gaa aac aat cca ttt tta ggg ttt gca gga gct      336
   Tyr Ser Phe Lys Tyr Glu Asn Asn Pro Phe Leu Gly Phe Ala Gly Ala
               100                 105                 110
   att ggc tac tca atg ggt ggt cca agg gta gag ttt gaa gtg tct tac       384
   Ile Gly Tyr Ser Met Gly Gly Pro Arg Val Glu Phe Glu Val Ser Tyr
           115                 120                 125
   gaa ata ttt gat gta aaa aac caa ggt aac agt tac aag aac gat gct       432
   Glu Ile Phe Asp Val Lys Asn Gln Gly Asn Ser Tyr Lys Asn Asp Ala
       130                 135                 140
   cac aaa tat tgc gct tta tca aga cac acc gga ggt atg cca caa gcc       480
   His Lys Tyr Cys Ala Leu Ser Arg His Thr Gly Gly Met Pro Gln Ala
   145                 150                 155                 160
   ggt cat caa aat aaa ttt gtc ttc cta aaa aat gaa gga tta ctt gac       528
   Gly His Gln Asn Lys Phe Val Phe Leu Lys Asn Glu Gly Leu Leu Asp
                   165                 170                 175
   ata tca ctt atg ata aac gca tgt tat gat ata aca atc gac agc atg       576
   Ile Ser Leu Met Ile Asn Ala Cys Tyr Asp Ile Thr Ile Asp Ser Met
               180                 185                 190
   cca ttt tct cca tat ata tgt gca ggt att ggt agt gac tta gtt tcg       624
   Pro Phe Ser Pro Tyr Ile Cys Ala Gly Ile Gly Ser Asp Leu Val Ser
           195                 200                 205
   atg ttt gaa act aca aat cct aaa att tct tat caa gga aaa tta ggt       672
   Met Phe Glu Thr Thr Asn Pro Lys Ile Ser Tyr Gln Gly Lys Leu Gly
       210                 215                 220
   gta agt tac tcc ata agc cca gaa gca tct gtt ttt gtt gga gga cac       720
   Val Ser Tyr Ser Ile Ser Pro Glu Ala Ser Val Phe Val Gly Gly His
   225                 230                 235                 240
   ttt cac aga gtt ata ggt aat gaa ttt aaa gac att cct gca ata act       768
   Phe His Arg Val Ile Gly Asn Glu Phe Lys Asp Ile Pro Ala Ile Thr
                   245                 250                 255
   cct gct gga gca aca gaa att aaa ggc aca cag ttt aca aca gta aca       816
   Pro Ala Gly Ala Thr Glu Ile Lys Gly Thr Gln Phe Thr Thr Val Thr
               260                 265                 270
   tta aac ata tgc cac ttc gga cta gag ctt gga ggc agg ttt act ttt       864
   Leu Asn Ile Cys His Phe Gly Leu Glu Leu Gly Gly Arg Phe Thr Phe
           275                 280                 285
   taa                                                                   867
<210> 32
   <211> 288
   <212> PRT
   <213> Ehrlichia canis
<400> 32
   Met Asn Cys Lys Arg Phe Phe Ile Ala Ser Ala Leu Ile Ser Leu Met
   1               5                   10                  15
   Ser Phe Leu Pro Ser Val Ser Phe Ser Glu Ser Ile His Glu Asp Asn
               20                  25                  30
   Ile Asn Gly Asn Phe Tyr Ile Ser Ala Lys Tyr Met Pro Ser Ala Ser
           35                  40                  45
   His Phe Gly Val Phe Ser Val Lys Glu Glu Lys Asn Thr Thr Thr Gly
       50                  55                  60
   Val Phe Gly Leu Lys Gln Asp Trp Asp Gly Ala Thr Ile Lys Asp Ala
   65                  70                  75                  80
   Ser Ser Ser His Thr Ile Asp Pro Ser Thr Ile Phe Ser Ile Ser Asn
                   85                  90                  95
   Tyr Ser Phe Lys Tyr Glu Asn Asn Pro Phe Leu Gly Phe Ala Gly Ala
               100                 105                 110
   Ile Gly Tyr Ser Met Gly Gly Pro Arg Val Glu Phe Glu Val Ser Tyr
           115                 120                 125
   Glu Ile Phe Asp Val Lys Asn Gln Gly Asn Ser Tyr Lys Asn Asp Ala
       130                 135                 140
   His Lys Tyr Cys Ala Leu Ser Arg His Thr Gly Gly Met Pro Gln Ala
   145                 150                 155                 160
   Gly His Gln Asn Lys Phe Val Phe Leu Lys Asn Glu Gly Leu Leu Asp
                   165                 170                 175
   Ile Ser Leu Met Ile Asn Ala Cys Tyr Asp Ile Thr Ile Asp Ser Met
               180                 185                 190
   Pro Phe Ser Pro Tyr Ile Cys Ala Gly Ile Gly Ser Asp Leu Val Ser
           195                 200                 205
   Met Phe Glu Thr Thr Asn Pro Lys Ile Ser Tyr Gln Gly Lys Leu Gly
       210                 215                 220
   Val Ser Tyr Ser Ile Ser Pro Glu Ala Ser Val Phe Val Gly Gly His
   225                 230                 235                 240
   Phe His Arg Val Ile Gly Asn Glu Phe Lys Asp Ile Pro Ala Ile Thr
                   245                 250                 255
   Pro Ala Gly Ala Thr Glu Ile Lys Gly Thr Gln Phe Thr Thr Val Thr
               260                 265                 270
   Leu Asn Ile Cys His Phe Gly Leu Glu Leu Gly Gly Arg Phe Thr Phe
           275                 280                 285
<210> 33
   <211> 864
   <212> DNA
   <213> Ehrlichia chaffeensis
<220>
   <221> CDS
   <222> (1)..(864)
<400> 33
   atg aaa tat aaa aaa act ttt aca gta act gca tta gta tta tta act      48
   Met Lys Tyr Lys Lys Thr Phe Thr Val Thr Ala Leu Val Leu Leu Thr
   1               5                   10                  15
   tcc ttt aca cat ttt ata cct ttt tat agt cca gca cgt gcc agt aca      96
   Ser Phe Thr His Phe Ile Pro Phe Tyr Ser Pro Ala Arg Ala Ser Thr
               20                  25                  30
   att cac aac ttc tac att agt gga aaa tat atg cca aca gcg tca cat     144
   Ile His Asn Phe Tyr Ile Ser Gly Lys Tyr Met Pro Thr Ala Ser His
           35                  40                  45
   ttt gga att ttt tca gct aaa gaa gaa caa agt ttt act aag gta tta     192
   Phe Gly Ile Phe Ser Ala Lys Glu Glu Gln Ser Phe Thr Lys Val Leu
       50                  55                  60
   gtt ggg tta gat caa cga tta tca cat aat att ata aac aat aat gat     240
   Val Gly Leu Asp Gln Arg Leu Ser His Asn Ile Ile Asn Asn Asn Asp
   65                  70                  75                  80
   aca gca aag agt ctt aag gtt caa aat tat tca ttt aaa tac aaa aat     288
   Thr Ala Lys Ser Leu Lys Val Gln Asn Tyr Ser Phe Lys Tyr Lys Asn
                   85                  90                  95
   aac cca ttt cta gga ttt gca aga gct att ggt tat tca ata ggc aat     336
   Asn Pro Phe Leu Gly Phe Ala Arg Ala Ile Gly Tyr Ser Ile Gly Asn
               100                 105                 110
   tca aga ata gaa cta gaa gta tca cat gaa ata ttt gat act aaa aac     384
   Ser Arg Ile Glu Leu Glu Val Ser His Glu Ile Phe Asp Thr Lys Asn
           115                 120                 125
   cca gga aac aat tat tta aat gac tct cac aaa tat tgc gct tta tct     432
   Pro Gly Asn Asn Tyr Leu Asn Asp Ser His Lys Tyr Cys Ala Leu Ser
       130                 135                 140
   cat gga agt cac ata tgc agt gat gga aat agc gga gat tgg tac act     480
   His Gly Ser His Ile Cys Ser Asp Gly Asn Ser Gly Asp Trp Tyr Thr
   145                 150                 155                 160
   gca aaa act gat aag ttt gta ctt ctg aaa aat gaa ggt tta ctt gac     528
   Ala Lys Thr Asp Lys Phe Val Leu Leu Lys Asn Glu Gly Leu Leu Asp
                   165                 170                 175
   gtc tca ttt atg tta aac gca tgt tat gac ata aca act gaa aaa atg     576
   Val Ser Phe Met Leu Asn Ala Cys Tyr Asp Ile Thr Thr Glu Lys Met
               180                 185                 190
   cct ttt tca cct tat ata tgt gca ggt att ggt act gat ctc ata tct      624
   Pro Phe Ser Pro Tyr Ile Cys Ala Gly Ile Gly Thr Asp Leu Ile Ser
           195                 200                 205
   atg ttt gag aca aca caa aac aaa ata tct tat caa gga aag tta ggt      672
   Met Phe Glu Thr Thr Gln Asn Lys Ile Ser Tyr Gln Gly Lys Leu Gly
       210                 215                 220
   tta aac tat act ata aac tca aga gtt tct gtt ttt gca ggt ggg cac      720
   Leu Asn Tyr Thr Ile Asn Ser Arg Val Ser Val Phe Ala Gly Gly His
   225                 230                 235                 240
   ttt cat aaa gta ata ggt aat gaa ttt aaa ggt att cct act cta tta      768
   Phe His Lys Val Ile Gly Asn Glu Phe Lys Gly Ile Pro Thr Leu Leu
                   245                 250                 255
   cct gat gga tca aac att aaa gta caa cag tct gca aca gta aca tta      816
   Pro Asp Gly Ser Asn Ile Lys Val Gln Gln Ser Ala Thr Val Thr Leu
               260                 265                 270
   gat gtg tgc cat ttc ggg tta gag att gga agt aga ttt ttc ttt taa      864
   Asp Val Cys His Phe Gly Leu Glu Ile Gly Ser Arg Phe Phe Phe
           275                 280                 285
<210> 34
   <211> 287
   <212> PRT
   <213> Ehrlichia chaffeensis
<400> 34
   Met Lys Tyr Lys Lys Thr Phe Thr Val Thr Ala Leu Val Leu Leu Thr
   1               5                   10                  15
   Ser Phe Thr His Phe Ile Pro Phe Tyr Ser Pro Ala Arg Ala Ser Thr
               20                  25                  30
   Ile His Asn Phe Tyr Ile Ser Gly Lys Tyr Met Pro Thr Ala Ser His
           35                  40                  45
   Phe Gly Ile Phe Ser Ala Lys Glu Glu Gln Ser Phe Thr Lys Val Leu
       50                  55                  60
   Val Gly Leu Asp Gln Arg Leu Ser His Asn Ile Ile Asn Asn Asn Asp
   65 70 75 80
   Thr Ala Lys Ser Leu Lys Val Gln Asn Tyr Ser Phe Lys Tyr Lys Asn
   85 90 95
   Asn Pro Phe Leu Gly Phe Ala Arg Ala Ile Gly Tyr Ser Ile Gly Asn
   100 105 110
   Ser Arg Ile Glu Leu Glu Val Ser His Glu Ile Phe Asp Thr Lys Asn
   115 120 125
   Pro Gly Asn Asn Tyr Leu Asn Asp Ser His Lys Tyr Cys Ala Leu Ser
   130 135 140
   His Gly Ser His Ile Cys Ser Asp Gly Asn Ser Gly Asp Trp Tyr Thr
   145 150 155 160
   Ala Lys Thr Asp Lys Phe Val Leu Leu Lys Asn Glu Gly Leu Leu Asp
   165 170 175
   Val Ser Phe Met Leu Asn Ala Cys Tyr Asp Ile Thr Thr Glu Lys Met
   180 185 190
   Pro Phe Ser Pro Tyr Ile Cys Ala Gly Ile Gly Thr Asp Leu Ile Ser
   195 200 205
   Met Phe Glu Thr Thr Gln Asn Lys Ile Ser Tyr Gln Gly Lys Leu Gly
   210 215 220
   Leu Asn Tyr Thr Ile Asn Ser Arg Val Ser Val Phe Ala Gly Gly His
   225 230 235 240
   Phe His Lys Val Ile Gly Asn Glu Phe Lys Gly Ile Pro Thr Leu Leu
   245 250 255
   Pro Asp Gly Ser Asn Ile Lys Val Gln Gln Ser Ala Thr Val Thr Leu
               260                 265                 270
   Asp Val Cys His Phe Gly Leu Glu Ile Gly Ser Arg Phe Phe Phe
           275                 280                 285
<210> 35
   <211> 924
   <212> DNA
   <213> Ehrlichia canis
<220>
   <221> CDS
   <222> (1)..(924)
<400> 35
   atg ttt tat act aat ata tat att ctg gct tgt att tac ttt gca ctt       48
   Met Phe Tyr Thr Asn Ile Tyr Ile Leu Ala Cys Ile Tyr Phe Ala Leu
   1               5                   10                  15
   cca cta ttg tta att tat ttt cac tat ttt agg tgt aat atg aat tgc       96
   Pro Leu Leu Leu Ile Tyr Phe His Tyr Phe Arg Cys Asn Met Asn Cys
               20                  25                  30
   aaa aaa att ctt ata aca act gca tta ata tca tta atg tac tct att      144
   Lys Lys Ile Leu Ile Thr Thr Ala Leu Ile Ser Leu Met Tyr Ser Ile
           35                  40                  45
   cca agc ata tct ttt tct gat act ata caa gat ggt aac atg ggt ggt      192
   Pro Ser Ile Ser Phe Ser Asp Thr Ile Gln Asp Gly Asn Met Gly Gly
       50                  55                  60
   aac ttc tat att agt gga aag tat gta cca agt gtc tca cat ttt ggt      240
   Asn Phe Tyr Ile Ser Gly Lys Tyr Val Pro Ser Val Ser His Phe Gly
   65                  70                  75                  80
   agc ttc tca gct aaa gaa gaa agc aaa tca act gtt gga gtt ttt gga      288
   Ser Phe Ser Ala Lys Glu Glu Ser Lys Ser Thr Val Gly Val Phe Gly
                   85                  90                  95
   tta aaa cat gat tgg gat gga agt cca ata ctt aag aat aaa cac gct      336
   Leu Lys His Asp Trp Asp Gly Ser Pro Ile Leu Lys Asn Lys His Ala
               100                 105                 110
   gac ttt act gtt cca aac tat tcg ttc aga tac gag aac aat cca ttt      384
   Asp Phe Thr Val Pro Asn Tyr Ser Phe Arg Tyr Glu Asn Asn Pro Phe
           115                 120                 125
   cta ggg ttt gca gga gct atc ggt tac tca atg ggt ggc cca aga ata      432
   Leu Gly Phe Ala Gly Ala Ile Gly Tyr Ser Met Gly Gly Pro Arg Ile
       130                 135                 140
   gaa ttc gaa ata tct tat gaa gca ttc gac gta aaa agt cct aat atc      480
   Glu Phe Glu Ile Ser Tyr Glu Ala Phe Asp Val Lys Ser Pro Asn Ile
   145                 150                 155                 160
   aat tat caa aat gac gcg cac agg tac tgc gct cta tct cat cac aca      528
   Asn Tyr Gln Asn Asp Ala His Arg Tyr Cys Ala Leu Ser His His Thr
                   165                 170                 175
   tcg gca gcc atg gaa gct gat aaa ttt gtc ttc tta aaa aac gaa ggg      576
   Ser Ala Ala Met Glu Ala Asp Lys Phe Val Phe Leu Lys Asn Glu Gly
               180                 185                 190
   tta att gac ata tca ctt gca ata aat gca tgt tat gat ata ata aat      624
   Leu Ile Asp Ile Ser Leu Ala Ile Asn Ala Cys Tyr Asp Ile Ile Asn
           195                 200                 205
   gac aaa gta cct gtt tct cct tat ata tgc gca ggt att ggt act gat      672
   Asp Lys Val Pro Val Ser Pro Tyr Ile Cys Ala Gly Ile Gly Thr Asp
       210                 215                 220
   ttg att tct atg ttt gaa gct aca agt cct aaa att tcc tac caa gga      720
   Leu Ile Ser Met Phe Glu Ala Thr Ser Pro Lys Ile Ser Tyr Gln Gly
   225                 230                 235                 240
   aaa ctg ggc att agt tac tct att aat ccg gaa acc tct gtt ttc atc      768
   Lys Leu Gly Ile Ser Tyr Ser Ile Asn Pro Glu Thr Ser Val Phe Ile
                   245                 250                 255
   ggt ggg cat ttc cac agg atc ata ggt aat gag ttt aga gat att cct      816
   Gly Gly His Phe His Arg Ile Ile Gly Asn Glu Phe Arg Asp Ile Pro
               260                 265                 270
   gca ata gta cct agt aac tca act aca ata agt gga cca caa ttt gca      864
   Ala Ile Val Pro Ser Asn Ser Thr Thr Ile Ser Gly Pro Gln Phe Ala
           275                 280                 285
   aca gta aca cta aat gtg tgt cac ttt ggt tta gaa ctt gga gga aga      912
   Thr Val Thr Leu Asn Val Cys His Phe Gly Leu Glu Leu Gly Gly Arg
       290                 295                 300
   ttt aac ttc taa                                                      924
   Phe Asn Phe
   305
<210> 36
   <211> 307
   <212> PRT
   <213> Ehrlichia canis
<400> 36
   Met Phe Tyr Thr Asn Ile Tyr Ile Leu Ala Cys Ile Tyr Phe Ala Leu
   1               5                   10                  15
   Pro Leu Leu Leu Ile Tyr Phe His Tyr Phe Arg Cys Asn Met Asn Cys
               20                  25                  30
   Lys Lys Ile Leu Ile Thr Thr Ala Leu Ile Ser Leu Met Tyr Ser Ile
           35                  40                  45
   Pro Ser Ile Ser Phe Ser Asp Thr Ile Gln Asp Gly Asn Met Gly Gly
       50                  55                  60
   Asn Phe Tyr Ile Ser Gly Lys Tyr Val Pro Ser Val Ser His Phe Gly
   65                  70                  75                  80
   Ser Phe Ser Ala Lys Glu Glu Ser Lys Ser Thr Val Gly Val Phe Gly
                   85                  90                  95
   Leu Lys His Asp Trp Asp Gly Ser Pro Ile Leu Lys Asn Lys His Ala
               100                 105                 110
   Asp Phe Thr Val Pro Asn Tyr Ser Phe Arg Tyr Glu Asn Asn Pro Phe
           115                 120                 125
   Leu Gly Phe Ala Gly Ala Ile Gly Tyr Ser Met Gly Gly Pro Arg Ile
       130                 135                 140
   Glu Phe Glu Ile Ser Tyr Glu Ala Phe Asp Val Lys Ser Pro Asn Ile
   145                 150                 155                 160
   Asn Tyr Gln Asn Asp Ala His Arg Tyr Cys Ala Leu Ser His His Thr
                   165                 170                 175
   Ser Ala Ala Met Glu Ala Asp Lys Phe Val Phe Leu Lys Asn Glu Gly
               180                 185                 190
   Leu Ile Asp Ile Ser Leu Ala Ile Asn Ala Cys Tyr Asp Ile Ile Asn
           195                 200                 205
   Asp Lys Val Pro Val Ser Pro Tyr Ile Cys Ala Gly Ile Gly Thr Asp
       210                 215                 220
   Leu Ile Ser Met Phe Glu Ala Thr Ser Pro Lys Ile Ser Tyr Gln Gly
   225                 230                 235                 240
   Lys Leu Gly Ile Ser Tyr Ser Ile Asn Pro Glu Thr Ser Val Phe Ile
                   245                 250                 255
   Gly Gly His Phe His Arg Ile Ile Gly Asn Glu Phe Arg Asp Ile Pro
               260                 265                 270
   Ala Ile Val Pro Ser Asn Ser Thr Thr Ile Ser Gly Pro Gln Phe Ala
           275                 280                 285
   Thr Val Thr Leu Asn Val Cys His Phe Gly Leu Glu Leu Gly Gly Arg
       290                 295                 300
   Phe Asn Phe
   305
<210> 37
   <211> 843
   <212> DNA
   <213> Ehrlichia canis
<220>
   <221> CDS
   <222> (1)..(843)
<400> 37
   atg aat tgc aaa aaa att ctt ata aca act gca tta atg tca tta atg      48
   Met Asn Cys Lys Lys Ile Leu Ile Thr Thr Ala Leu Met Ser Leu Met
   1               5                   10                  15
   tac tat gct cca agc ata tct ttt tct gat act ata caa gac gat aac      96
   Tyr Tyr Ala Pro Ser Ile Ser Phe Ser Asp Thr Ile Gln Asp Asp Asn
               20                  25                  30
   act ggt agc ttc tac atc agt gga aaa tat gta cca agt gtt tca cat     144
   Thr Gly Ser Phe Tyr Ile Ser Gly Lys Tyr Val Pro Ser Val Ser His
           35                  40                  45
   ttt ggt gtt ttc tca gct aaa gaa gaa aga aac tca act gtt gga gtt     192
   Phe Gly Val Phe Ser Ala Lys Glu Glu Arg Asn Ser Thr Val Gly Val
       50                  55                  60
   ttt gga tta aaa cat gat tgg aat gga ggt aca ata tct aac tct tct     240
   Phe Gly Leu Lys His Asp Trp Asn Gly Gly Thr Ile Ser Asn Ser Ser
   65                  70                  75                  80
   cca gaa aat ata ttc aca gtt caa aat tat tcg ttt aaa tac gaa aac     288
   Pro Glu Asn Ile Phe Thr Val Gln Asn Tyr Ser Phe Lys Tyr Glu Asn
                   85                  90                  95
   aac cca ttc tta ggg ttt gca gga gct att ggt tat tca atg ggt ggc     336
   Asn Pro Phe Leu Gly Phe Ala Gly Ala Ile Gly Tyr Ser Met Gly Gly
               100                 105                 110
   cca aga ata gaa ctt gaa gtt ctg tac gag aca ttc gat gtg aaa aat     384
   Pro Arg Ile Glu Leu Glu Val Leu Tyr Glu Thr Phe Asp Val Lys Asn
           115                 120                 125
   cag aac aat aat tat aag aac ggc gca cac aga tac tgt gct tta tct     432
   Gln Asn Asn Asn Tyr Lys Asn Gly Ala His Arg Tyr Cys Ala Leu Ser
       130                 135                 140
   cat cat agt tca gca aca aac atg tcc tcc gca agt aac aaa ttt gtt      480
   His His Ser Ser Ala Thr Asn Met Ser Ser Ala Ser Asn Lys Phe Val
   145                 150                 155                 160
   ttc tta aaa aat gaa ggg tta att gac tta tca ttt atg ata aat gca      528
   Phe Leu Lys Asn Glu Gly Leu Ile Asp Leu Ser Phe Met Ile Asn Ala
                   165                 170                 175
   tgc tat gac ata ata att gaa gga atg cct ttt tca cct tat att tgt      576
   Cys Tyr Asp Ile Ile Ile Glu Gly Met Pro Phe Ser Pro Tyr Ile Cys
               180                 185                 190
   gca ggt gtt ggt act gat gtt gtt tcc atg ttt gaa gct ata aat cct      624
   Ala Gly Val Gly Thr Asp Val Val Ser Met Phe Glu Ala Ile Asn Pro
           195                 200                 205
   aaa att tct tac caa gga aaa cta gga tta ggt tat agt ata agt tca      672
   Lys Ile Ser Tyr Gln Gly Lys Leu Gly Leu Gly Tyr Ser Ile Ser Ser
       210                 215                 220
   gaa gcc tct gtt ttt atc ggt gga cac ttt cac aga gtc ata ggt aat      720
   Glu Ala Ser Val Phe Ile Gly Gly His Phe His Arg Val Ile Gly Asn
   225                 230                 235                 240
   gaa ttt aga gac atc cct gct atg gtt cct agt gga tca aat ctt cca      768
   Glu Phe Arg Asp Ile Pro Ala Met Val Pro Ser Gly Ser Asn Leu Pro
                   245                 250                 255
   gaa aac caa ttt gca ata gta aca cta aat gtg tgt cac ttt ggt tta      816
   Glu Asn Gln Phe Ala Ile Val Thr Leu Asn Val Cys His Phe Gly Leu
               260                 265                 270
   gaa ctt gga gga aga ttt aac ttc tga                                  843
   Glu Leu Gly Gly Arg Phe Asn Phe
           275                 280
<210> 38
   <211> 280
   <212> PRT
   <213> Ehrlichia canis
<400> 38
   Met Asn Cys Lys Lys Ile Leu Ile Thr Thr Ala Leu Met Ser Leu Met
   1               5                   10                  15
   Tyr Tyr Ala Pro Ser Ile Ser Phe Ser Asp Thr Ile Gln Asp Asp Asn
               20                  25                  30
   Thr Gly Ser Phe Tyr Ile Ser Gly Lys Tyr Val Pro Ser Val Ser His
           35                  40                  45
   Phe Gly Val Phe Ser Ala Lys Glu Glu Arg Asn Ser Thr Val Gly Val
       50                  55                  60
   Phe Gly Leu Lys His Asp Trp Asn Gly Gly Thr Ile Ser Asn Ser Ser
   65                  70                  75                  80
   Pro Glu Asn Ile Phe Thr Val Gln Asn Tyr Ser Phe Lys Tyr Glu Asn
                   85                  90                  95
   Asn Pro Phe Leu Gly Phe Ala Gly Ala Ile Gly Tyr Ser Met Gly Gly
               100                 105                 110
   Pro Arg Ile Glu Leu Glu Val Leu Tyr Glu Thr Phe Asp Val Lys Asn
           115                 120                 125
   Gln Asn Asn Asn Tyr Lys Asn Gly Ala His Arg Tyr Cys Ala Leu Ser
       130                 135                 140
   His His Ser Ser Ala Thr Asn Met Ser Ser Ala Ser Asn Lys Phe Val
   145                 150                 155                 160
   Phe Leu Lys Asn Glu Gly Leu Ile Asp Leu Ser Phe Met Ile Asn Ala
                   165                 170                 175
   Cys Tyr Asp Ile Ile Ile Glu Gly Met Pro Phe Ser Pro Tyr Ile Cys
               180                 185                 190
   Ala Gly Val Gly Thr Asp Val Val Ser Met Phe Glu Ala Ile Asn Pro
           195                 200                 205
   Lys Ile Ser Tyr Gln Gly Lys Leu Gly Leu Gly Tyr Ser Ile Ser Ser
       210                 215                 220
   Glu Ala Ser Val Phe Ile Gly Gly His Phe His Arg Val Ile Gly Asn
   225                 230                 235                 240
   Glu Phe Arg Asp Ile Pro Ala Met Val Pro Ser Gly Ser Asn Leu Pro
                   245                 250                 255
   Glu Asn Gln Phe Ala Ile Val Thr Leu Asn Val Cys His Phe Gly Leu
               260                 265                 270
   Glu Leu Gly Gly Arg Phe Asn Phe
           275                 280
<210> 39
   <211> 852
   <212> DNA
   <213> Ehrlichia canis
<220>
   <221> CDS
   <222> (1)..(852)
<400> 39
   atg aat tgt aaa aaa gtt ttc aca ata agt gca ttg ata tca tcc ata       48
   Met Asn Cys Lys Lys Val Phe Thr Ile Ser Ala Leu Ile Ser Ser Ile
   1               5                   10                  15
   tac ttc cta cct aat gtc tca tac tct aac cca gta tat ggt aac agt       96
   Tyr Phe Leu Pro Asn Val Ser Tyr Ser Asn Pro Val Tyr Gly Asn Ser
               20                  25                  30
   atg tat ggt aat ttt tac ata tca gga aag tac atg cca agt gtt cct      144
   Met Tyr Gly Asn Phe Tyr Ile Ser Gly Lys Tyr Met Pro Ser Val Pro
           35                  40                  45
   cat ttt gga att ttt tca gct gaa gaa gag aaa aaa aag aca act gta      192
   His Phe Gly Ile Phe Ser Ala Glu Glu Glu Lys Lys Lys Thr Thr Val
       50                  55                  60
   gta tat ggc tta aaa gga aaa ctg gca gga gat gca ata tct agt caa      240
   Val Tyr Gly Leu Lys Gly Lys Leu Ala Gly Asp Ala Ile Ser Ser Gln
   65                  70                  75                  80
   agt cca gat gat aat ttt acc att cga aat tac tca ttc aag tat gca      288
   Ser Pro Asp Asp Asn Phe Thr Ile Arg Asn Tyr Ser Phe Lys Tyr Ala
                   85                  90                  95
   agc aac aag ttt tta ggg ttt gca gta gct att ggt tac tcg ata ggc      336
   Ser Asn Lys Phe Leu Gly Phe Ala Val Ala Ile Gly Tyr Ser Ile Gly
               100                 105                 110
   agt cca aga ata gaa gtt gag atg tct tat gaa gca ttt gat gtg aaa      384
   Ser Pro Arg Ile Glu Val Glu Met Ser Tyr Glu Ala Phe Asp Val Lys
           115                 120                 125
   aat cca ggt gat aat tac aaa aac ggt gct tac agg tat tgt gct tta      432
   Asn Pro Gly Asp Asn Tyr Lys Asn Gly Ala Tyr Arg Tyr Cys Ala Leu
       130                 135                 140
   tct cat caa gat gat gcg gat gat gac atg act agt gca act gac aaa      480
   Ser His Gln Asp Asp Ala Asp Asp Asp Met Thr Ser Ala Thr Asp Lys
   145                 150                 155                 160
   ttt gta tat tta att aat gaa gga tta ctt aac ata tca ttt atg aca      528
   Phe Val Tyr Leu Ile Asn Glu Gly Leu Leu Asn Ile Ser Phe Met Thr
                   165                 170                 175
   aac ata tgt tat gaa aca gca agc aaa aat ata cct ctc tct cct tac      576
   Asn Ile Cys Tyr Glu Thr Ala Ser Lys Asn Ile Pro Leu Ser Pro Tyr
               180                 185                 190
   ata tgt gca ggt att ggt act gat tta att cac atg ttt gaa act aca      624
   Ile Cys Ala Gly Ile Gly Thr Asp Leu Ile His Met Phe Glu Thr Thr
           195                 200                 205
   cat cct aaa att tct tat caa gga aag cta ggg ttg gcc tac ttc gta      672
   His Pro Lys Ile Ser Tyr Gln Gly Lys Leu Gly Leu Ala Tyr Phe Val
       210                 215                 220
   agt gca gag tct tcg gtt tct ttt ggt ata tat ttt cat aaa att ata      720
   Ser Ala Glu Ser Ser Val Ser Phe Gly Ile Tyr Phe His Lys Ile Ile
   225                 230                 235                 240
   aat aat aag ttt aaa aat gtt cca gcc atg gta cct att aac tca gac       768
   Asn Asn Lys Phe Lys Asn Val Pro Ala Met Val Pro Ile Asn Ser Asp
                   245                 250                 255
   gag ata gta gga cca cag ttt gca aca gta aca tta aat gta tgc tac       816
   Glu Ile Val Gly Pro Gln Phe Ala Thr Val Thr Leu Asn Val Cys Tyr
               260                 265                 270
   ttt gga tta gaa ctt gga tgt agg ttc aac ttc taa                       852
   Phe Gly Leu Glu Leu Gly Cys Arg Phe Asn Phe
           275                 280
<210> 40
   <211> 283
   <212> PRT
   <213> Ehrlichia canis
<400> 40
   Met Asn Cys Lys Lys Val Phe Thr Ile Ser Ala Leu Ile Ser Ser Ile
   1               5                   10                  15
   Tyr Phe Leu Pro Asn Val Ser Tyr Ser Asn Pro Val Tyr Gly Asn Ser
               20                  25                  30
   Met Tyr Gly Asn Phe Tyr Ile Ser Gly Lys Tyr Met Pro Ser Val Pro
           35                  40                  45
   His Phe Gly Ile Phe Ser Ala Glu Glu Glu Lys Lys Lys Thr Thr Val
       50                  55                  60
   Val Tyr Gly Leu Lys Gly Lys Leu Ala Gly Asp Ala Ile Ser Ser Gln
   65                  70                  75                  80
   Ser Pro Asp Asp Asn Phe Thr Ile Arg Asn Tyr Ser Phe Lys Tyr Ala
                   85                  90                  95
   Ser Asn Lys Phe Leu Gly Phe Ala Val Ala Ile Gly Tyr Ser Ile Gly
               100                 105                 110
   Ser Pro Arg Ile Glu Val Glu Met Ser Tyr Glu Ala Phe Asp Val Lys
   115 120 125
   Asn Pro Gly Asp Asn Tyr Lys Asn Gly Ala Tyr Arg Tyr Cys Ala Leu
   130 135 140
   Ser His Gln Asp Asp Ala Asp Asp Asp Met Thr Ser Ala Thr Asp Lys
   145 150 155 160
   Phe Val Tyr Leu Ile Asn Glu Gly Leu Leu Asn Ile Ser Phe Met Thr
   165 170 175
   Asn Ile Cys Tyr Glu Thr Ala Ser Lys Asn Ile Pro Leu Ser Pro Tyr
   180 185 190
   Ile Cys Ala Gly Ile Gly Thr Asp Leu Ile His Met Phe Glu Thr Thr
   195 200 205
   His Pro Lys Ile Ser Tyr Gln Gly Lys Leu Gly Leu Ala Tyr Phe Val
   210 215 220
   Ser Ala Glu Ser Ser Val Ser Phe Gly Ile Tyr Phe His Lys Ile Ile
   225 230 235 240
   Asn Asn Lys Phe Lys Asn Val Pro Ala Met Val Pro Ile Asn Ser Asp
   245 250 255
   Glu Ile Val Gly Pro Gln Phe Ala Thr Val Thr Leu Asn Val Cys Tyr
   260 265 270
   Phe Gly Leu Glu Leu Gly Cys Arg Phe Asn Phe
   275 280
<210> 41
   <211> 831
   <212> DNA
   <213> Ehrlichia canis
<220>
   <221> CDS
   <222> (1)..(831)
<400> 41
   atg aac tgt aaa aaa ttt ctt ata aca act aca ttg gta tca cta aca        48
   Met Asn Cys Lys Lys Phe Leu Ile Thr Thr Thr Leu Val Ser Leu Thr
   1               5                   10                  15
   att ctt tta cct ggc ata tct ttc tcc aaa cca ata cat gaa aac aat        96
   Ile Leu Leu Pro Gly Ile Ser Phe Ser Lys Pro Ile His Glu Asn Asn
               20                  25                  30
   act aca gga aac ttt tac att att gga aaa tat gta cca agt att tca       144
   Thr Thr Gly Asn Phe Tyr Ile Ile Gly Lys Tyr Val Pro Ser Ile Ser
           35                  40                  45
   cat ttt ggg aac ttt tca gct aaa gaa gaa aaa aac aca act act gga       192
   His Phe Gly Asn Phe Ser Ala Lys Glu Glu Lys Asn Thr Thr Thr Gly
       50                  55                  60
   att ttt gga tta aaa gaa tca tgg act ggt ggt atc atc ctt gat aaa       240
   Ile Phe Gly Leu Lys Glu Ser Trp Thr Gly Gly Ile Ile Leu Asp Lys
   65                  70                  75                  80
   gaa cat gca gct ttt aat atc cca aat tat tca ttt aaa tat gaa aat       288
   Glu His Ala Ala Phe Asn Ile Pro Asn Tyr Ser Phe Lys Tyr Glu Asn
                   85                  90                  95
   aat cca ttt tta gga ttt gca ggg gta att ggc tat tca ata ggt agt       336
   Asn Pro Phe Leu Gly Phe Ala Gly Val Ile Gly Tyr Ser Ile Gly Ser
               100                 105                 110
   cca aga ata gaa ttt gaa gta tca tac gag aca ttc gat gta caa aat       384
   Pro Arg Ile Glu Phe Glu Val Ser Tyr Glu Thr Phe Asp Val Gln Asn
           115                 120                 125
   cca gga gat aag ttt aac aat gat gca cat aag tat tgt gct tta tcc       432
   Pro Gly Asp Lys Phe Asn Asn Asp Ala His Lys Tyr Cys Ala Leu Ser
       130                 135                 140
   aat gat tcc agt aaa aca atg aaa agt ggt aaa ttc gtt ttt ctc aaa        480
   Asn Asp Ser Ser Lys Thr Met Lys Ser Gly Lys Phe Val Phe Leu Lys
   145                 150                 155                 160
   aat gaa gga tta agt gac ata tca ctc atg tta aat gta tgt tat gat        528
   Asn Glu Gly Leu Ser Asp Ile Ser Leu Met Leu Asn Val Cys Tyr Asp
                   165                 170                 175
   ata ata aac aaa aga atg cct ttt tca cct tac ata tgt gca ggc att        576
   Ile Ile Asn Lys Arg Met Pro Phe Ser Pro Tyr Ile Cys Ala Gly Ile
               180                 185                 190
   ggt act gac tta ata ttc atg ttt gac gct ata aac cat aaa gct gct        624
   Gly Thr Asp Leu Ile Phe Met Phe Asp Ala Ile Asn His Lys Ala Ala
           195                 200                 205
   tat caa gga aaa tta ggt ttt aat tat cca ata agc cca gaa gct aac        672
   Tyr Gln Gly Lys Leu Gly Phe Asn Tyr Pro Ile Ser Pro Glu Ala Asn
       210                 215                 220
   att tct atg ggt gtg cac ttt cac aaa gta aca aac aac gag ttt aga        720
   Ile Ser Met Gly Val His Phe His Lys Val Thr Asn Asn Glu Phe Arg
   225                 230                 235                 240
   gtt cct gtt cta tta act gct gga gga ctc gct cca gat aat cta ttt        768
   Val Pro Val Leu Leu Thr Ala Gly Gly Leu Ala Pro Asp Asn Leu Phe
                   245                 250                 255
   gca ata gta aag ttg agt ata tgt cat ttt ggg tta gaa ttt ggg tac        816
   Ala Ile Val Lys Leu Ser Ile Cys His Phe Gly Leu Glu Phe Gly Tyr
               260                 265                 270
   agg gtc agt ttt taa                                                    831
   Arg Val Ser Phe
           275
<210> 42
   <211> 276
   <212> PRT
   <213> Ehrlichia canis
<400> 42
   Met Asn Cys Lys Lys Phe Leu Ile Thr Thr Thr Leu Val Ser Leu Thr
   1               5                   10                  15
   Ile Leu Leu Pro Gly Ile Ser Phe Ser Lys Pro Ile His Glu Asn Asn
               20                  25                  30
   Thr Thr Gly Asn Phe Tyr Ile Ile Gly Lys Tyr Val Pro Ser Ile Ser
           35                  40                  45
   His Phe Gly Asn Phe Ser Ala Lys Glu Glu Lys Asn Thr Thr Thr Gly
       50                  55                  60
   Ile Phe Gly Leu Lys Glu Ser Trp Thr Gly Gly Ile Ile Leu Asp Lys
   65                  70                  75                  80
   Glu His Ala Ala Phe Asn Ile Pro Asn Tyr Ser Phe Lys Tyr Glu Asn
                   85                  90                  95
   Asn Pro Phe Leu Gly Phe Ala Gly Val Ile Gly Tyr Ser Ile Gly Ser
               100                 105                 110
   Pro Arg Ile Glu Phe Glu Val Ser Tyr Glu Thr Phe Asp Val Gln Asn
           115                 120                 125
   Pro Gly Asp Lys Phe Asn Asn Asp Ala His Lys Tyr Cys Ala Leu Ser
       130                 135                 140
   Asn Asp Ser Ser Lys Thr Met Lys Ser Gly Lys Phe Val Phe Leu Lys
   145                 150                 155                 160
   Asn Glu Gly Leu Ser Asp Ile Ser Leu Met Leu Asn Val Cys Tyr Asp
                   165                 170                 175
   Ile Ile Asn Lys Arg Met Pro Phe Ser Pro Tyr Ile Cys Ala Gly Ile
               180                 185                 190
   Gly Thr Asp Leu Ile Phe Met Phe Asp Ala Ile Asn His Lys Ala Ala
           195                 200                 205
   Tyr Gln Gly Lys Leu Gly Phe Asn Tyr Pro Ile Ser Pro Glu Ala Asn
   210 215 220
   Ile Ser Met Gly Val His Phe His Lys Val Thr Asn Asn Glu Phe Arg
   225 230 235 240
   Val Pro Val Leu Leu Thr Ala Gly Gly Leu Ala Pro Asp Asn Leu Phe
   245 250 255
   Ala Ile Val Lys Leu Ser Ile Cys His Phe Gly Leu Glu Phe Gly Tyr
   260 265 270
   Arg Val Ser Phe
   275
<210> 43
   <211> 882
   <212> DNA
   <213> Ehrlichia canis
<220>
   <221> CDS
   <222> (1)..(882)
<400> 43
   atg aat aat aaa ctc aaa ttt act ata ata aac aca gta tta gta tgc 48
   Met Asn Asn Lys Leu Lys Phe Thr Ile Ile Asn Thr Val Leu Val Cys
   1 5 10 15
   tta ttg tca tta cct aat ata tct tcc tca aag gcc ata aac aat aac 96
   Leu Leu Ser Leu Pro Asn Ile Ser Ser Ser Lys Ala Ile Asn Asn Asn
   20 25 30
   gct aaa aag tac tac gga tta tat atc agt gga caa tat aaa ccc agt 144
   Ala Lys Lys Tyr Tyr Gly Leu Tyr Ile Ser Gly Gln Tyr Lys Pro Ser
   35 40 45
   gtt tct gtt ttc agt aat ttt tca gtt aaa gaa acc aat gtc ata act 192
   Val Ser Val Phe Ser Asn Phe Ser Val Lys Glu Thr Asn Val Ile Thr
   50 55 60
   aaa aac ctt ata gct tta aaa aaa gat gtt gac tct att gaa acc aag      240
   Lys Asn Leu Ile Ala Leu Lys Lys Asp Val Asp Ser Ile Glu Thr Lys
   65                  70                  75                  80
   act gat gcc agt gta ggt att agt aac cca tca aat ttt act atc ccc      288
   Thr Asp Ala Ser Val Gly Ile Ser Asn Pro Ser Asn Phe Thr Ile Pro
                   85                  90                  95
   tat aca gct gta ttt caa gat aat tct gtc aat ttc aat gga act att      336
   Tyr Thr Ala Val Phe Gln Asp Asn Ser Val Asn Phe Asn Gly Thr Ile
               100                 105                 110
   ggt tac acc ttt gct gaa ggt aca aga gtt gaa ata gaa ggt tct tat      384
   Gly Tyr Thr Phe Ala Glu Gly Thr Arg Val Glu Ile Glu Gly Ser Tyr
           115                 120                 125
   gag gaa ttt gat gtt aaa aac cct gga ggc tat aca cta agt gat gcc      432
   Glu Glu Phe Asp Val Lys Asn Pro Gly Gly Tyr Thr Leu Ser Asp Ala
       130                 135                 140
   tat cgc tat ttt gca tta gca cgt gaa atg aaa ggt aat agt ttt aca      480
   Tyr Arg Tyr Phe Ala Leu Ala Arg Glu Met Lys Gly Asn Ser Phe Thr
   145                 150                 155                 160
   cct aaa gaa aaa gtt tct aat agt ttt ttt cac act gta atg aga aat      528
   Pro Lys Glu Lys Val Ser Asn Ser Phe Phe His Thr Val Met Arg Asn
                   165                 170                 175
   gat gga tta tct ata ata tct gtt ata gta aat gtt tgc tac gat ttc      576
   Asp Gly Leu Ser Ile Ile Ser Val Ile Val Asn Val Cys Tyr Asp Phe
               180                 185                 190
   tct ttg aac aat ttg tca ata tcg cct tac ata tgt gga gga gca ggg      624
   Ser Leu Asn Asn Leu Ser Ile Ser Pro Tyr Ile Cys Gly Gly Ala Gly
           195                 200                 205
   gta gat gct ata gaa ttc ttc gat gta tta cac att aag ttt gca tat      672
   Val Asp Ala Ile Glu Phe Phe Asp Val Leu His Ile Lys Phe Ala Tyr
       210                 215                 220
   caa agc aag cta ggt att gct tat tct cta cca tct aac att agt ctc      720
   Gln Ser Lys Leu Gly Ile Ala Tyr Ser Leu Pro Ser Asn Ile Ser Leu
   225                 230                 235                 240
   ttt gct agt tta tat tac cat aaa gta atg ggc aat caa ttt aaa aat        768
   Phe Ala Ser Leu Tyr Tyr His Lys Val Met Gly Asn Gln Phe Lys Asn
                   245                 250                 255
   tta aat gtc caa gat gtt gct gaa ctt gca agt ata cct aaa att aca        816
   Leu Asn Val Gln Asp Val Ala Glu Leu Ala Ser Ile Pro Lys Ile Thr
               260                 265                 270
   tcc gca gtt gct aca ctt aat att ggt tat ttt gga ggt gaa att ggt        864
   Ser Ala Val Ala Thr Leu Asn Ile Gly Tyr Phe Gly Gly Glu Ile Gly
           275                 280                 285
   gca aga ttg aca ttt taa                                                882
   Ala Arg Leu Thr Phe
       290
<210> 44
   <211> 293
   <212> PRT
   <213> Ehrlichia canis
<400> 44
   Met Asn Asn Lys Leu Lys Phe Thr Ile Ile Asn Thr Val Leu Val Cys
   1               5                   10                  15
   Leu Leu Ser Leu Pro Asn Ile Ser Ser Ser Lys Ala Ile Asn Asn Asn
               20                  25                  30
   Ala Lys Lys Tyr Tyr Gly Leu Tyr Ile Ser Gly Gln Tyr Lys Pro Ser
           35                  40                  45
   Val Ser Val Phe Ser Asn Phe Ser Val Lys Glu Thr Asn Val Ile Thr
       50                  55                  60
   Lys Asn Leu Ile Ala Leu Lys Lys Asp Val Asp Ser Ile Glu Thr Lys
   65                  70                  75                  80
   Thr Asp Ala Ser Val Gly Ile Ser Asn Pro Ser Asn Phe Thr Ile Pro
                   85                  90                  95
   Tyr Thr Ala Val Phe Gln Asp Asn Ser Val Asn Phe Asn Gly Thr Ile
   100 105 110
   Gly Tyr Thr Phe Ala Glu Gly Thr Arg Val Glu Ile Glu Gly Ser Tyr
   115 120 125
   Glu Glu Phe Asp Val Lys Asn Pro Gly Gly Tyr Thr Leu Ser Asp Ala
   130 135 140
   Tyr Arg Tyr Phe Ala Leu Ala Arg Glu Met Lys Gly Asn Ser Phe Thr
   145 150 155 160
   Pro Lys Glu Lys Val Ser Asn Ser Phe Phe His Thr Val Met Arg Asn
   165 170 175
   Asp Gly Leu Ser Ile Ile Ser Val Ile Val Asn Val Cys Tyr Asp Phe
   180 185 190
   Ser Leu Asn Asn Leu Ser Ile Ser Pro Tyr Ile Cys Gly Gly Ala Gly
   195 200 205
   Val Asp Ala Ile Glu Phe Phe Asp Val Leu His Ile Lys Phe Ala Tyr
   210 215 220
   Gln Ser Lys Leu Gly Ile Ala Tyr Ser Leu Pro Ser Asn Ile Ser Leu
   225 230 235 240
   Phe Ala Ser Leu Tyr Tyr His Lys Val Met Gly Asn Gln Phe Lys Asn
   245 250 255
   Leu Asn Val Gln Asp Val Ala Glu Leu Ala Ser Ile Pro Lys Ile Thr
   260 265 270
   Ser Ala Val Ala Thr Leu Asn Ile Gly Tyr Phe Gly Gly Glu Ile Gly
   275 280 285
   Ala Arg Leu Thr Phe
   290
<210> 45
   <211> 900
   <212> DNA
   <213> Ehrlichia canis
<220>
   <221> CDS
   <222> (1)..(900)
<400> 45
   atg aat agc aag agt aag ttc ttt aca ata tgt aca tcg tta ata tgc       48
   Met Asn Ser Lys Ser Lys Phe Phe Thr Ile Cys Thr Ser Leu Ile Cys
   1               5                   10                  15
   tta tta tca tca cct aac aca tct ctc tca aac ttc ata ggc aat agt       96
   Leu Leu Ser Ser Pro Asn Thr Ser Leu Ser Asn Phe Ile Gly Asn Ser
               20                  25                  30
   aca aaa cat tct gga tta tat gtt agc gga cat tat aag ccc agc gtt      144
   Thr Lys His Ser Gly Leu Tyr Val Ser Gly His Tyr Lys Pro Ser Val
           35                  40                  45
   tcc att ttt agc aaa ttt tca gta aaa gaa aca aat aca cat aca gta      192
   Ser Ile Phe Ser Lys Phe Ser Val Lys Glu Thr Asn Thr His Thr Val
       50                  55                  60
   cag tta gta gct ctt aaa aaa gat gtt aat tct att tct atg aac atc      240
   Gln Leu Val Ala Leu Lys Lys Asp Val Asn Ser Ile Ser Met Asn Ile
   65                  70                  75                  80
   agt aat ggt gct aca ggc att agc aaa gca aca aat ttt aat ctt cct      288
   Ser Asn Gly Ala Thr Gly Ile Ser Lys Ala Thr Asn Phe Asn Leu Pro
                   85                  90                  95
   tat gtt gca gaa ttt caa gac aat gcc ttc aac ttc agt gga gct att      336
   Tyr Val Ala Glu Phe Gln Asp Asn Ala Phe Asn Phe Ser Gly Ala Ile
               100                 105                 110
   ggt tat tca ctt ttt gaa caa cta aac att gaa gtt gaa ggt tct tat      384
   Gly Tyr Ser Leu Phe Glu Gln Leu Asn Ile Glu Val Glu Gly Ser Tyr
           115                 120                 125
   gaa gaa ttc gat gcc aaa aat cct ggt ggt tat att tta aat gat gca      432
   Glu Glu Phe Asp Ala Lys Asn Pro Gly Gly Tyr Ile Leu Asn Asp Ala
       130                 135                 140
   ttc cgc tat ttt gca ttg gca cgt gaa atg gga caa gaa aaa aat gat      480
   Phe Arg Tyr Phe Ala Leu Ala Arg Glu Met Gly Gln Glu Lys Asn Asp
   145                 150                 155                 160
   aat aag cat ctt agt cct aag gag gag cat gat ata agt aaa aca tat      528
   Asn Lys His Leu Ser Pro Lys Glu Glu His Asp Ile Ser Lys Thr Tyr
                   165                 170                 175
   tac aca gtc atg aga aat aat ggg tta tct ata tta tct att atg ata      576
   Tyr Thr Val Met Arg Asn Asn Gly Leu Ser Ile Leu Ser Ile Met Ile
               180                 185                 190
   aat ggc tgc tat aat cta cct ctc aat gat tta tca ata tca cct tat      624
   Asn Gly Cys Tyr Asn Leu Pro Leu Asn Asp Leu Ser Ile Ser Pro Tyr
           195                 200                 205
   ttt tgt aca gga ata ggt gta gat gct ata gaa ttt ttt gat gca ctg      672
   Phe Cys Thr Gly Ile Gly Val Asp Ala Ile Glu Phe Phe Asp Ala Leu
       210                 215                 220
   cat ctt aaa ctt gct ttg caa agt aaa ata gga gct act tac caa tta      720
   His Leu Lys Leu Ala Leu Gln Ser Lys Ile Gly Ala Thr Tyr Gln Leu
   225                 230                 235                 240
   tca gac aac att agt tta ttt aca aat gga tat atc cat caa gta ata      768
   Ser Asp Asn Ile Ser Leu Phe Thr Asn Gly Tyr Ile His Gln Val Ile
                   245                 250                 255
   ggt gat caa ttt aaa aac tta aaa gtc caa tat ata ggt gaa ctt aaa      816
   Gly Asp Gln Phe Lys Asn Leu Lys Val Gln Tyr Ile Gly Glu Leu Lys
               260                 265                 270
   gag aac ccg aaa att aca tct gca gtt gct act ctc aat gtt gga tac      864
   Glu Asn Pro Lys Ile Thr Ser Ala Val Ala Thr Leu Asn Val Gly Tyr
           275                 280                 285
   ttt gga ggt gaa att gga gta aga ctc aca ctt taa                      900
   Phe Gly Gly Glu Ile Gly Val Arg Leu Thr Leu
       290                 295
<210> 46
   <211> 299
   <212> PRT
   <213> Ehrlichia canis
<400> 46
   Met Asn Ser Lys Ser Lys Phe Phe Thr Ile Cys Thr Ser Leu Ile Cys
   1               5                   10                  15
   Leu Leu Ser Ser Pro Asn Thr Ser Leu Ser Asn Phe Ile Gly Asn Ser
               20                  25                  30
   Thr Lys His Ser Gly Leu Tyr Val Ser Gly His Tyr Lys Pro Ser Val
           35                  40                  45
   Ser Ile Phe Ser Lys Phe Ser Val Lys Glu Thr Asn Thr His Thr Val
       50                  55                  60
   Gln Leu Val Ala Leu Lys Lys Asp Val Asn Ser Ile Ser Met Asn Ile
   65                  70                  75                  80
   Ser Asn Gly Ala Thr Gly Ile Ser Lys Ala Thr Asn Phe Asn Leu Pro
                   85                  90                  95
   Tyr Val Ala Glu Phe Gln Asp Asn Ala Phe Asn Phe Ser Gly Ala Ile
               100                 105                 110
   Gly Tyr Ser Leu Phe Glu Gln Leu Asn Ile Glu Val Glu Gly Ser Tyr
           115                 120                 125
   Glu Glu Phe Asp Ala Lys Asn Pro Gly Gly Tyr Ile Leu Asn Asp Ala
       130                 135                 140
   Phe Arg Tyr Phe Ala Leu Ala Arg Glu Met Gly Gln Glu Lys Asn Asp
   145                 150                 155                 160
   Asn Lys His Leu Ser Pro Lys Glu Glu His Asp Ile Ser Lys Thr Tyr
                   165                 170                 175
   Tyr Thr Val Met Arg Asn Asn Gly Leu Ser Ile Leu Ser Ile Met Ile
               180                 185                 190
   Asn Gly Cys Tyr Asn Leu Pro Leu Asn Asp Leu Ser Ile Ser Pro Tyr
           195                 200                 205
   Phe Cys Thr Gly Ile Gly Val Asp Ala Ile Glu Phe Phe Asp Ala Leu
       210                 215                 220
   His Leu Lys Leu Ala Leu Gln Ser Lys Ile Gly Ala Thr Tyr Gln Leu
   225                 230                 235                 240
   Ser Asp Asn Ile Ser Leu Phe Thr Asn Gly Tyr Ile His Gln Val Ile
                   245                 250                 255
   Gly Asp Gln Phe Lys Asn Leu Lys Val Gln Tyr Ile Gly Glu Leu Lys
               260                 265                 270
   Glu Asn Pro Lys Ile Thr Ser Ala Val Ala Thr Leu Asn Val Gly Tyr
           275                 280                 285
   Phe Gly Gly Glu Ile Gly Val Arg Leu Thr Leu
       290                 295
<210> 47
   <211> 495
   <212> DNA
   <213> Ehrlichia canis
<220>
   <221> CDS
   <222> (1)..(495)
<400> 47
   atg aat tat aag aaa att cta gta aga agc gcg tta atc tca tta atg      48
   Met Asn Tyr Lys Lys Ile Leu Val Arg Ser Ala Leu Ile Ser Leu Met
   1               5                   10                  15
   tca atc tta cca tat cag ttc ttt gca gat cct cta ggt tca aga act      96
   Ser Ile Leu Pro Tyr Gln Phe Phe Ala Asp Pro Leu Gly Ser Arg Thr
               20                  25                  30
   aat gat aac aaa gaa ggc ttc tac att agt gca aag tac aat cca agt      144
   Asn Asp Asn Lys Glu Gly Phe Tyr Ile Ser Ala Lys Tyr Asn Pro Ser
           35                  40                  45
   ata tca cac ttt aga aaa ttc tct gct gaa gaa act cct att aat gga      192
   Ile Ser His Phe Arg Lys Phe Ser Ala Glu Glu Thr Pro Ile Asn Gly
       50                  55                  60
   aca aat tct ctc act aaa aaa gtt ttc gga cta aag aaa gat ggt gat      240
   Thr Asn Ser Leu Thr Lys Lys Val Phe Gly Leu Lys Lys Asp Gly Asp
   65                  70                  75                  80
   ata aca aaa aaa gac gat ttt aca aga gta gct cca ggc att gat ttt      288
   Ile Thr Lys Lys Asp Asp Phe Thr Arg Val Ala Pro Gly Ile Asp Phe
                   85                  90                  95
   caa aat aac tta ata tca gga ttt tca gga agt att ggt tac tct atg      336
   Gln Asn Asn Leu Ile Ser Gly Phe Ser Gly Ser Ile Gly Tyr Ser Met
               100                 105                 110
   gac gga cca aga ata gaa ctt gaa gct gca tat cac att tta atc caa      384
   Asp Gly Pro Arg Ile Glu Leu Glu Ala Ala Tyr His Ile Leu Ile Gln
           115                 120                 125
   aaa cac gat aac aat gat act gat aat ggt gaa tac tat aaa cat ttt      432
   Lys His Asp Asn Asn Asp Thr Asp Asn Gly Glu Tyr Tyr Lys His Phe
       130                 135                 140
   gca tat ctc gta aag atg cca tgg aag atc agc cat atg ttg ttc tta      480
   Ala Tyr Leu Val Lys Met Pro Trp Lys Ile Ser His Met Leu Phe Leu
   145                 150                 155                 160
   aaa atg acg gca tac                                                  495
   Lys Met Thr Ala Tyr
                   165
<210> 48
   <211> 165
   <212> PRT
   <213> Ehrlichia canis
<400> 48
   Met Asn Tyr Lys Lys Ile Leu Val Arg Ser Ala Leu Ile Ser Leu Met
   1               5                   10                  15
   Ser Ile Leu Pro Tyr Gln Phe Phe Ala Asp Pro Leu Gly Ser Arg Thr
               20                  25                  30
   Asn Asp Asn Lys Glu Gly Phe Tyr Ile Ser Ala Lys Tyr Asn Pro Ser
           35                  40                  45
   Ile Ser His Phe Arg Lys Phe Ser Ala Glu Glu Thr Pro Ile Asn Gly
       50                  55                  60
   Thr Asn Ser Leu Thr Lys Lys Val Phe Gly Leu Lys Lys Asp Gly Asp
   65                  70                  75                  80
   Ile Thr Lys Lys Asp Asp Phe Thr Arg Val Ala Pro Gly Ile Asp Phe
                   85                  90                  95
   Gln Asn Asn Leu Ile Ser Gly Phe Ser Gly Ser Ile Gly Tyr Ser Met
               100                 105                 110
   Asp Gly Pro Arg Ile Glu Leu Glu Ala Ala Tyr His Ile Leu Ile Gln
           115                 120                 125
   Lys His Asp Asn Asn Asp Thr Asp Asn Gly Glu Tyr Tyr Lys His Phe
       130                 135                 140
   Ala Tyr Leu Val Lys Met Pro Trp Lys Ile Ser His Met Leu Phe Leu
   145                 150                 155                 160
   Lys Met Thr Ala Tyr
                   165
<210> 49
   <211> 34
   <212> DNA
   <213> Artificial/Unknown
<220>
   <221> misc feature
   <222> ()..()
   <223> Reverse primer
<220>
   <221> misc_feature
   <222> 17, 26
   <223> n= a/t/g/c
<400> 49
   cgggatccga attcggnath aayggnaayt tyta 34
<210> 50
   <211> 30
   <212> DNA
   <213> Artificial/Unknown
<220>
   <221> misc_feature
   <222> ()..()
   <223> Reverse primer
<400> 50
   agcggccgct taraayasra aycttsctcc 30
<210> 51
   <211> 756
   <212> DNA
   <213> Ehrlichia chaffeensis
<400> 51
   ggcataaatg ggaatttcta catcagtgga aaatacatgc caagtgcttc gcattttgga      60
   gtattctctg ctaaggaaga aagaaataca acagttggag tgtttggact gaagcaaaat     120
   tgggacggaa gcgcaatatc caactcctcc ccaaacgatg tattcactgt ctcaaattat     180
   tcatttaaat atgaaaacaa cccgttttta ggttttgcag gagctattgg ttactcaatg     240
   gatggtccaa gaatagagct tgaagtatct tatgaaacat ttgatgtaaa aaatcaaggt     300
   aacaattata agaatgaagc acatagatat tgtgctctat cccataactc agcagcagac     360
   atgagtagtg caagtaataa ttttgtcttt ctaaaaaatg aaggattact tgacatatca      420
   tttatgctga acgcatgcta tgacgtagta ggcgaaggca tacctttttc tccttatata     480
   tgcgcaggta tcggtactga tttagtatcc atgtttgaag ctacaaatcc taaaatttct     540
   taccaaggaa agttaggttt aagctactct ataagcccag aagcttctgt gtttattggt     600
   gggcactttc ataaggtaat agggaacgaa tttagagata ttcctactat aatacctact     660
   ggatcaacac ttgcaggaaa aggaaactac cctgcaatag taatactgga tgtatgccac     720
   tttggaatag aacttggagg aaggtttgta ttctaa                               756
<210> 52
   <211> 256
   <212> PRT
   <213> Ehrlichia chaffeensis
<400> 52
   Asp Pro Ala Gly Ser Gly Ile Asn Gly Asn Phe Tyr Ile Ser Gly Lys
   1 5 10 15
   Tyr Met Pro Ser Ala Ser His Phe Gly Val Phe Ser Ala Lys Glu Glu
   20 25 30
   Arg Asn Thr Thr Val Gly Val Phe Gly Leu Lys Gln Asn Trp Asp Gly
   35 40 45
   Ser Ala Ile Ser Asn Ser Ser Pro Asn Asp Val Phe Thr Val Ser Asn
   50 55 60
   Tyr Ser Phe Lys Tyr Glu Asn Asn Pro Phe Leu Gly Phe Ala Gly Ala
   65 70 75 80
   Ile Gly Tyr Ser Met Asp Gly Pro Arg Ile Glu Leu Glu Val Ser Tyr
   85 90 95
   Glu Thr Phe Asp Val Lys Asn Gln Gly Asn Asn Tyr Lys Asn Glu Ala
   100 105 110
   His Arg Tyr Cys Ala Leu Ser His Asn Ser Ala Ala Asp Met Ser Ser
   115 120 125
   Ala Ser Asn Asn Phe Val Phe Leu Lys Asn Glu Gly Leu Leu Asp Ile
   130 135 140
   Ser Phe Met Leu Asn Ala Cys Tyr Asp Val Val Gly Glu Gly Ile Pro
   145 150 155 160
   Phe Ser Pro Tyr Ile Cys Ala Gly Ile Gly Thr Asp Leu Val Ser Met
   165 170 175
   Phe Glu Ala Thr Asn Pro Lys Ile Ser Tyr Gln Gly Lys Leu Gly Leu
   180 185 190
   Ser Tyr Ser Ile Ser Pro Glu Ala Ser Val Phe Ile Gly Gly His Phe
   195 200 205
   His Lys Val Ile Gly Asn Glu Phe Arg Asp Ile Pro Thr Ile Ile Pro
   210 215 220
   Thr Gly Ser Thr Leu Ala Gly Lys Gly Asn Tyr Pro Ala Ile Val Ile
   225 230 235 240
   Leu Asp Val Cys His Phe Gly Ile Glu Leu Gly Gly Arg Phe Val Phe
   245 250 255
<210> 53
   <211> 817
   <212> DNA
   <213> Ehrlichia canis
<400> 53
   tagcagcact aaaaaacagt ttgggttata tgttagtgga caacaccagc ctagtgtttc 60
   tatttttagc aatttctcag taaaggaaac taattttcct acaaagtatt ctagcagctt 120
   cttaaaaaaa gacattaatt ctgtcgaatt tgacgatagt gttactgctg gcattagtta 180
   cccacttaat ttcagtactc cttatatagc tgtatttcaa gataatattt ctaattttaa 240
   tggcgctatt gggtacactt ttgttgaagg cccaagaatt gaaatagaag gttcttatga 300
   agaattcgat gtcaaagacc ctggaagata tacagaaata caagatgcat accgttactt 360
   tgctttagca cgtgatatag actctattcc tactagccca aaaaatagaa cttcacatga 420
   tggcaacagt tcatataagg tataccacac tgtaatgaaa aatgaaggac tatctataat 480
   atccattatg gtcaatggct gctatgattt ttcttcagat aatttatcaa tattacctta 540
   tgtatgtggt ggtataggtg taaatgctat agagtttttc gatgcattac atgttaaatt 600
   cgcgtgtcag ggtaaattag gtattactta tccattatct tccaacgtta gtttatttgc 660
   tggtggatat tatcaccaag taatgggcaa ccaatttaaa aatctaaatg ttcaacatgt 720
   agctgaactt aatgacgcac ccaaagttac atctgcagta gctacacttg acattgggta 780
   ttttggtggt gaaattggag caaggcttat attttaa 817
<210> 54
   <211> 271
   <212> PRT
   <213> Ehrlichia canis
<400> 54
   Ser Ser Thr Lys Lys Gln Phe Gly Leu Tyr Val Ser Gly Gln His Gln
   1               5                   10                  15
   Pro Ser Val Ser Ile Phe Ser Asn Phe Ser Val Lys Glu Thr Asn Phe
               20                  25                  30
   Pro Thr Lys Tyr Ser Ser Ser Phe Leu Lys Lys Asp Ile Asn Ser Val
           35                  40                  45
   Glu Phe Asp Asp Ser Val Thr Ala Gly Ile Ser Tyr Pro Leu Asn Phe
       50                  55                  60
   Ser Thr Pro Tyr Ile Ala Val Phe Gln Asp Asn Ile Ser Asn Phe Asn
   65                  70                  75                  80
   Gly Ala Ile Gly Tyr Thr Phe Val Glu Gly Pro Arg Ile Glu Ile Glu
                   85                  90                  95
   Gly Ser Tyr Glu Glu Phe Asp Val Lys Asp Pro Gly Arg Tyr Thr Glu
               100                 105                 110
   Ile Gln Asp Ala Tyr Arg Tyr Phe Ala Leu Ala Arg Asp Ile Asp Ser
           115                 120                 125
   Ile Pro Thr Ser Pro Lys Asn Arg Thr Ser His Asp Gly Asn Ser Ser
       130                 135                 140
   Tyr Lys Val Tyr His Thr Val Met Lys Asn Glu Gly Leu Ser Ile Ile
   145                 150                 155                 160
   Ser Ile Met Val Asn Gly Cys Tyr Asp Phe Ser Ser Asp Asn Leu Ser
                   165                 170                 175
   Ile Leu Pro Tyr Val Cys Gly Gly Ile Gly Val Asn Ala Ile Glu Phe
               180                 185                 190
   Phe Asp Ala Leu His Val Lys Phe Ala Cys Gln Gly Lys Leu Gly Ile
           195                 200                 205
   Thr Tyr Pro Leu Ser Ser Asn Val Ser Leu Phe Ala Gly Gly Tyr Tyr
       210                 215                 220
   His Gln Val Met Gly Asn Gln Phe Lys Asn Leu Asn Val Gln His Val
   225                 230                 235                 240
   Ala Glu Leu Asn Asp Ala Pro Lys Val Thr Ser Ala Val Ala Thr Leu
                   245                 250                 255
   Asp Ile Gly Tyr Phe Gly Gly Glu Ile Gly Ala Arg Leu Ile Phe
               260                 265                 270
<210> 55
   <211> 882
   <212> DNA
   <213> Ehrlichia canis
<400> 55
   atggcaaatt ttatgtacaa aaaatacaaa ctaatgacag caggtgtagt attatttcac 60
   atgttatttc tacctcatgt ttctttcgca aaaaatacaa acagcaataa acttggatta 120
   tacatcagtg gacagtataa ccctagtgtt tctgttttta gcaatttttc agcaaaagaa 180
   accaatgttc atacagtaca actcatggcg cttaaaaaag acattgattc tattgaagtt 240
   gatactggaa atagcgcagg tattagcaaa ccacaaaatt tcacagttct ttatactcca 300
   aaatttcaag ataatgttgc tggtcttagc ggtgcacttg gattctttta ttctaaagga 360
   ttaaggattg aaatggggtt ttcttatgaa aaatttgatg ctaaagacct tggtgagtac 420
   accaaaataa aagatgctta tagatatttt gctctagtac gtgaaatgca tgttagtctc 480
   atttatccaa aagataataa cacaggaaca cattatactg ttatgagaaa tgatggtata 540
   tctatttctt ctgctacagt aaatggctgc tatgattctt ttttccagtt tatctttgtc 600
   acctatatgt gtataggcat cggtatagat gctatagaat ttcttaatgc atacatatta 660
   agtttgcttg ccaaggtagt taaggtgtta acttattctg tatctcccaa tgttaattta 720
   tttgcagatg gatattatca taaagtgatg ggcaataaat ttaaaaattt acctgttcaa 780
   tacgttaata ctttagaaga gtatccaaga gttacatctg caattgctac acttgatatt 840
   ggctacctcg gtggtgaaat tggcataaga tttatatttt aa 882
<210> 56
   <211> 289
   <212> PRT
   <213> Ehrlichia canis
<400> 56
   Met Tyr Lys Lys Tyr Lys Leu Met Thr Ala Gly Val Val Leu Phe His
   1 5 10 15
   Met Leu Phe Leu Pro His Val Ser Phe Ala Lys Asn Thr Asn Ser Asn
   20 25 30
   Lys Leu Gly Leu Tyr Ile Ser Gly Gln Tyr Asn Pro Ser Val Ser Val
   35 40 45
   Phe Ser Asn Phe Ser Ala Lys Glu Thr Asn Val His Thr Val Gln Leu
   50 55 60
   Met Ala Leu Lys Lys Asp Ile Asp Ser Ile Glu Val Asp Thr Gly Asn
   65 70 75 80
   Ser Ala Gly Ile Ser Lys Pro Gln Asn Phe Thr Val Leu Tyr Thr Pro
   85 90 95
   Lys Phe Gln Asp Asn Val Ala Gly Leu Ser Gly Ala Leu Gly Phe Phe
   100 105 110
   Tyr Ser Lys Gly Leu Arg Ile Glu Met Gly Phe Ser Tyr Glu Lys Phe
   115 120 125
   Asp Ala Lys Asp Leu Gly Glu Tyr Thr Lys Ile Lys Asp Ala Tyr Arg
   130 135 140
   Tyr Phe Ala Leu Val Arg Glu Met His Val Ser Leu Ile Tyr Pro Lys
   145 150 155 160
   Asp Asn Asn Thr Gly Thr His Tyr Thr Val Met Arg Asn Asp Gly Ile
   165 170 175
   Ser Ile Ser Ser Ala Thr Val Asn Gly Cys Tyr Asp Ser Phe Phe Gln
   180 185 190
   Phe Ile Phe Val Thr Tyr Met Cys Ile Gly Ile Gly Ile Asp Ala Ile
           195                 200                 205
   Glu Phe Leu Asn Ala Tyr Ile Leu Ser Leu Leu Ala Lys Val Val Lys
       210                 215                 220
   Val Leu Thr Tyr Ser Val Ser Pro Asn Val Asn Leu Phe Ala Asp Gly
   225                 230                 235                 240
   Tyr Tyr His Lys Val Met Gly Asn Lys Phe Lys Asn Leu Pro Val Gln
                   245                 250                 255
   Tyr Val Asn Thr Leu Glu Glu Tyr Pro Arg Val Thr Ser Ala Ile Ala
               260                 265                 270
   Thr Leu Asp Ile Gly Tyr Leu Gly Gly Glu Ile Gly Ile Arg Phe Ile
           275                 280                 285
   Phe
<210> 57
   <211> 891
   <212> DNA
   <213> Ehrlichia canis
<400> 57
   atgggaaatt ctatgaataa taaaagtcaa ttcttaataa gatttatatt tttaacatgc 60
   atgctgtcat tacctaatat atctctttca aaagtaaata acgaaaaaca ttctggtttg 120
   tatattagcg ggcaatacaa acccagtgtt tctgttttca gtaatttttc agttaaagaa 180
   accaactttc atacaaaaca tctcatagct cttaaacaag atgttgattc tgttgaaatt 240
   gatactggta gtaatacagc aggtattagt aacccatcta actttacaat cccttatact 300
   gcagaatttc aagacaacca tactaactgc aatggctcta ttggttatgc ttttgctgaa 360
   ggtccaagaa ttgaaataga attatcatat gaaaaatttg atgttaaaaa tcccacaggg 420
   tatactacag taaaagatgc ttatagatac tttgctttag cacgtgaaat aaatatttct 480
   ctattccaac caaaacaaaa agaaggtagt ggaatttacc atgtcgtaat gaaaaacgat 540
   gggttatcta tcttatccaa tatagttaat atttgctacg atttttcttt aaataattta 600
   cctatatcac cttatttatg cggaggaatg ggtataaatg ccatagaatt ctttgacgct 660
   ttacatgtga aatttgctta tcaaagcaag gcaggaatta gttatcaact attacgtaaa 720
   atcaacttat ttattgatgt atattactac gaagtaataa gtaataaatt taaaaacctg 780
   aaagtccaac atgtacatga acttaaagat aatccaaaag tcacatctgc agttgctaca 840
   cttgatatag catattttgg tagtgaagct ggcataagaa ttatatttta a 891
<210> 58
   <211> 292
   <212> PRT
   <213> Ehrlichia canis
<400> 58
   Met Asn Asn Lys Ser Gln Phe Leu Ile Arg Phe Ile Phe Leu Thr Cys
   1               5                   10                  15
   Met Leu Ser Leu Pro Asn Ile Ser Leu Ser Lys Val Asn Asn Glu Lys
               20                  25                  30
   His Ser Gly Leu Tyr Ile Ser Gly Gln Tyr Lys Pro Ser Val Ser Val
           35                  40                  45
   Phe Ser Asn Phe Ser Val Lys Glu Thr Asn Phe His Thr Lys His Leu
       50                  55                  60
   Ile Ala Leu Lys Gln Asp Val Asp Ser Val Glu Ile Asp Thr Gly Ser
   65                  70                  75                  80
   Asn Thr Ala Gly Ile Ser Asn Pro Ser Asn Phe Thr Ile Pro Tyr Thr
                   85                  90                  95
   Ala Glu Phe Gln Asp Asn His Thr Asn Cys Asn Gly Ser Ile Gly Tyr
               100                 105                 110
   Ala Phe Ala Glu Gly Pro Arg Ile Glu Ile Glu Leu Ser Tyr Glu Lys
           115                 120                 125
   Phe Asp Val Lys Asn Pro Thr Gly Tyr Thr Thr Val Lys Asp Ala Tyr
       130                 135                 140
   Arg Tyr Phe Ala Leu Ala Arg Glu Ile Asn Ile Ser Leu Phe Gln Pro
   145                 150                 155                 160
   Lys Gln Lys Glu Gly Ser Gly Ile Tyr His Val Val Met Lys Asn Asp
                   165                 170                 175
   Gly Leu Ser Ile Leu Ser Asn Ile Val Asn Ile Cys Tyr Asp Phe Ser
               180                 185                 190
   Leu Asn Asn Leu Pro Ile Ser Pro Tyr Leu Cys Gly Gly Met Gly Ile
           195                 200                 205
   Asn Ala Ile Glu Phe Phe Asp Ala Leu His Val Lys Phe Ala Tyr Gln
       210                 215                 220
   Ser Lys Ala Gly Ile Ser Tyr Gln Leu Leu Arg Lys Ile Asn Leu Phe
   225                 230                 235                 240
   Ile Asp Val Tyr Tyr Tyr Glu Val Ile Ser Asn Lys Phe Lys Asn Leu
                   245                 250                 255
   Lys Val Gln His Val His Glu Leu Lys Asp Asn Pro Lys Val Thr Ser
               260                 265                 270
   Ala Val Ala Thr Leu Asp Ile Ala Tyr Phe Gly Ser Glu Ala Gly Ile
           275                 280                 285
   Arg Ile Ile Phe
       290
<210> 59
   <211> 488
   <212> DNA
   <213> Ehrlichia canis
<400> 59
   aagcttctta tgaagaattt gacgttaaaa atcctgaagg atctactaca gactcctata      60
   gatatttcgc gttagcacgt ggcatggatg gtaataatat tcctacaagt caaaaattta     120
   ctgtaatgag aaacgacggg ttattaatct catctgttat gataaatggc tgttacaatg     180
   tcatactaaa tgatatacaa gcagaacctt acatatgtgc aggactagga ggagatttta     240
   tagaattctt caatggcttt catgttaagc tagcttatca aggtaaagta ggcattagtt     300
   atcaaatatt ccctgaagta agattattta ttgatggata ctaccataaa gtaaaaggca     360
   acaagtttaa aaatttacac gttcaacatg taggtgcact tgcagcactc cctaaagtta     420
   catctgcagt tgcaacactt aatattggat actttggttg tgaagctgga gtaagattca     480
   tattttaa                                                              488
<210> 60
   <211> 161
   <212> PRT
   <213> Ehrlichia canis
<400> 60
   Ala Ser Tyr Glu Glu Phe Asp Val Lys Asn Pro Glu Gly Ser Thr Thr
   1 5 10 15
   Asp Ser Tyr Arg Tyr Phe Ala Leu Ala Arg Gly Met Asp Gly Asn Asn
   20 25 30
   Ile Pro Thr Ser Gln Lys Phe Thr Val Met Arg Asn Asp Gly Leu Leu
   35 40 45
   Ile Ser Ser Val Met Ile Asn Gly Cys Tyr Asn Val Ile Leu Asn Asp
   50 55 60
   Ile Gln Ala Glu Pro Tyr Ile Cys Ala Gly Leu Gly Gly Asp Phe Ile
   65 70 75 80
   Glu Phe Phe Asn Gly Phe His Val Lys Leu Ala Tyr Gln Gly Lys Val
   85 90 95
   Gly Ile Ser Tyr Gln Ile Phe Pro Glu Val Arg Leu Phe Ile Asp Gly
   100 105 110
   Tyr Tyr His Lys Val Lys Gly Asn Lys Phe Lys Asn Leu His Val Gln
   115 120 125
   His Val Gly Ala Leu Ala Ala Leu Pro Lys Val Thr Ser Ala Val Ala
   130 135 140
   Thr Leu Asn Ile Gly Tyr Phe Gly Cys Glu Ala Gly Val Arg Phe Ile
   145 150 155 160
   Phe
<210> 61
   <211> 18
   <212> PRT
   <213> Ehrlichia chaffeensis
<220>
   <223> N-terminal amino acid sequence of P28
<400> 61
   Asp Pro Ala Gly Ser Gly Ile Asn Gly Asn Phe Tyr Ser Gly Lys Tyr
   1               5                   10                  15
   Met Pro
<210> 62
   <211> 81
   <212> PRT
   <213> Ehrlichia chaffeensis
<400> 62
   Glu Phe Phe Asp Ala Leu His Val Lys Phe Ala Tyr Gln Gly Lys Leu
   1               5                   10                  15
   Gly Ile Ser Tyr Gln Leu Phe Thr Lys Val Asn Leu Phe Leu Asp Gly
               20                  25                  30
   Tyr Tyr His Gln Val Ile Gly Asn Gln Phe Lys Asn Leu Asn Val Asn
           35                  40                  45
   His Val Tyr Thr Leu Lys Glu Ser Pro Lys Val Thr Ser Ala Val Ala
       50                  55                  60
   Thr Leu Asp Ile Ala Tyr Phe Gly Gly Glu Val Gly Ile Arg Phe Thr
   65                  70                  75                  80
   Phe
<210> 63
   <211> 256
   <212> PRT
   <213> Ehrlichia chaffeensis
<400> 63
   Asp Pro Ala Gly Ser Gly Ile Asn Gly Asn Phe Tyr Ile Ser Gly Lys
   1 5 10 15
   Tyr Met Pro Ser Ala Ser His Phe Gly Val Phe Ser Ala Lys Glu Glu
   20 25 30
   Arg Asn Thr Thr Val Gly Val Phe Gly Leu Lys Gln Asn Trp Asp Gly
   35 40 45
   Ser Ala Ile Ser Asn Ser Ser Pro Asn Asp Val Phe Thr Val Ser Asn
   50 55 60
   Tyr Ser Phe Lys Tyr Glu Asn Asn Pro Phe Leu Gly Phe Ala Gly Ala
   65 70 75 80
   Ile Gly Tyr Ser Met Asp Gly Pro Arg Ile Glu Leu Glu Val Ser Tyr
   85 90 95
   Glu Thr Phe Asp Val Lys Asn Gln Gly Asn Asn Tyr Lys Asn Glu Ala
   100 105 110
   His Arg Tyr Cys Ala Leu Ser His Asn Ser Ala Ala Asp Met Ser Ser
   115 120 125
   Ala Ser Asn Asn Phe Val Phe Leu Lys Asn Glu Gly Leu Leu Asp Ile
   130 135 140
   Ser Phe Met Leu Asn Ala Cys Tyr Asp Val Val Gly Glu Gly Ile Pro
   145 150 155 160
   Phe Ser Pro Tyr Ile Cys Ala Gly Ile Gly Thr Asp Leu Val Ser Met
   165 170 175
   Phe Glu Ala Thr Asn Pro Lys Ile Ser Tyr Gln Gly Lys Leu Gly Leu
   180 185 190
   Ser Tyr Ser Ile Ser Pro Glu Ala Ser Val Phe Ile Gly Gly His Phe
   195 200 205
   His Lys Val Ile Gly Asn Glu Phe Arg Asp Ile Pro Thr Ile Ile Pro
   210 215 220
   Thr Gly Ser Thr Leu Ala Gly Lys Gly Asn Tyr Pro Ala Ile Val Ile
   225 230 235 240
   Leu Asp Val Cys His Phe Gly Ile Glu Leu Gly Gly Arg Phe Val Phe
   245 250 255
<210> 64
   <211> 284
   <212> PRT
   <213> Cowdria ruminantium
<400> 64
   Met Asn Cys Lys Lys Ile Phe Ile Thr Ser Thr Leu Ile Ser Leu Val
   1 5 10 15
   Ser Phe Leu Pro Gly Val Ser Phe Ser Asp Val Ile Gln Glu Glu Asn
   20 25 30
   Asn Pro Val Gly Ser Val Tyr Ile Ser Ala Lys Tyr Met Pro Thr Ala
   35 40 45
   Ser His Phe Gly Lys Met Ser Ile Lys Glu Asp Ser Arg Asp Thr Lys
   50 55 60
   Ala Val Phe Gly Leu Lys Lys Asp Trp Asp Gly Val Lys Thr Pro Ser
   65 70 75 80
   Gly Asn Thr Asn Ser Ile Phe Thr Glu Lys Asp Tyr Ser Phe Lys Tyr
   85 90 95
   Glu Asn Asn Pro Phe Leu Gly Phe Ala Gly Ala Val Gly Tyr Ser Met
   100 105 110
   Asn Gly Pro Arg Ile Glu Phe Glu Val Ser Tyr Glu Thr Phe Asp Val
   115 120 125
   Arg Asn Pro Gly Gly Asn Tyr Lys Asn Asp Ala His Met Tyr Cys Ala
   130 135 140
   Leu Asp Thr Ala Ser Ser Ser Thr Ala Gly Ala Thr Thr Ser Val Asn
   145 150 155 160
   Val Lys Asn Glu Asn Leu Thr Asp Ile Ser Leu Met Leu Asn Ala Cys
                   165                 170                 175
   Tyr Asp Ile Asn Leu Asp Gly Met Pro Val Ser Pro Tyr Val Cys Ala
               180                 185                 190
   Gly Ile Gly Thr Asp Leu Val Ser Val Ile Asn Ala Thr Asn Pro Lys
           195                 200                 205
   Leu Ser Tyr Gln Gly Lys Leu Gly Ile Ser Tyr Ser Ile Asn Pro Glu
       210                 215                 220
   Ala Ser Ile Phe Ile Gly Gly His Phe His Arg Val Ile Gly Asn Glu
   225                 230                 235                     240
   Phe Lys Asp Ile Ala Thr Ser Lys Val Phe Thr Ser Ser Gly Asn Ala
                   245                 250                 255
   Ser Ser Ala Val Ser Pro Gly Phe Ala Ser Ala Ile Leu Asp Val Cys
               260                 265                 270
   His Phe Gly Ile Glu Ile Gly Gly Arg Phe Val Phe
           275                 280